# EUROPEAN PATENT SPECIFICATION

(11) **EP 2 544 688 B1**
(45) Date of publication and mention of the grant of the patent: **07.09.2016**
(21) Application number: 11751202.0
(22) Date of filing: 01.03.2011
(51) Int. Cl.: A61K 31/47, A61K 31/44, A61K 48/00, A61K 31/7105, A61K 39/395, A61K 38/17, A61K 38/16, A61P 25/00, A61K 31/4436, A61K 31/4525, A61K 31/4725, A61K 31/54, A61K 31/635

(54) **METHODS AND COMPOSITIONS FOR TREATMENT OF ANGELMAN SYNDROME**
VERFAHREN UND ZUSAMMENSETZUNGEN ZUR BEHANDLUNG DES ANGELMAN-SYNDROMS
PROCÉDÉS ET COMPOSITIONS POUR LE TRAITEMENT DU SYNDROME D'ANGELMAN

(30) Priority: 02.03.2010 US 309557 P
(43) Date of publication of application: 16.01.2013
(73) Proprietor: President and Fellows of Harvard College, Cambridge, MA 02138 (US); Children's Medical Center Corporation, Boston, MA 02115 (US)
(72) Inventor: GREENBERG, Michael, E., Brookline MA 02445 (US); GREER, Paul, L., Brookline MA 02115 (US)
(74) Representative: Crease, Devanand John
(86) International application number: PCT/US2011/026687
(87) International publication number: WO 2011/109398

(56) References cited:
- WO-A2-01/14390
- US-A1- 2004 067 978
- US-A1- 2004 067 978
- US-A1- 2009 192 199
- RENZO GUERRINI ET AL: "Cortical myoclonus in angelman syndrome", ANNALS OF NEUROLOGY, vol. 40, no. 1, 1 July 1996 (1996-07-01), pages 39-48, XP055057177, ISSN: 0364-5134, DOI: 10.1002/ana.410400109
- PAUL L. GREER ET AL: "The Angelman Syndrome Protein Ube3A Regulates Synapse Development by Ubiquitinating Arc", CELL, vol. 140, no. 5, 1 March 2010 (2010-03-01) , pages 704-716, XP055057176, ISSN: 0092-8674, DOI: 10.1016/j.cell.2010.01.026
- BERNARD DAN: "Angelman syndrome: Current understanding and research prospects", EPILEPSIA, vol. 50, no. 11, 1 November 2009 (2009-11-01), pages 2331-2339, XP055057183, ISSN: 0013-9580, DOI: 10.1111/j.1528-1167.2009.02311.x
- MACKOWIAK, M. ET AL.: 'An AMPA receptor potentiator modulates hippocampal expression of BDNF: an in vivo study' NEUROPHARMACOLOGY vol. 43, 2002, pages 1 - 10, XP008130553
- CHRISTENSEN KENNETH V ET AL: 'Levetiracetam attenuates hippocampal expression of synaptic plasticity-related immediate early and late response genes in amygdala-kindled rats' BMC NEUROSCIENCE, BIOMED CENTRAL, LONDON, GB vol. 11, no. 1, 9, 27 January 2010, pages 1 - 15, XP021066457 ISSN: 1471-2202
- Ronald L. Thibert ET AL: "Epilepsy in Angelman syndrome: A questionnaire-based assessment of the natural history and current treatment options", Epilepsia, vol. 50, no. 11, 1 November 2009 (2009-11-01), pages 2369-2376, XP055129398, ISSN: 0013-9580, DOI: 10.1111/j.1528-1167.2009.02108.x

## Description

### FIELD OF INVENTION

The present invention relates to molecular biology and neurological development. In particular, the present invention provides for compositions and methods for decreasing Arc expression and/or increasing AMPA receptor activity to ameliorate the affects (such as cognitive dysfunction) of Ube3A disruption in Angelman Syndrome and autism spectrum disorders.

### BACKGROUND

Angelman syndrome (AS) is a neuro-genetic disorder characterized by intellectual and developmental delay, sleep disturbance, seizures, jerky movements, and frequent laughter or smiling. Although the prevalence of Angelman syndrome is not precisely known, it is estimated at 1/10,000 to 1/20,000 children. This debilitating neurological disorder is caused by mutation of the E3 ubiquitin ligase *Ube3A,* a gene whose mutation has also recently been associated with autism spectrum disorders (ASDs). Ube3A is a member of the E3 ubiquitin ligase family of enzymes, a class of proteins that catalyzes the addition of ubiquitin moieties to target substrates, often leading to the degradation of the ubiquitinated protein. The function of Ube3A during nervous system development, and how *Ube3A* mutations give rise to cognitive impairment in individuals with Angleman Syndrome and autism spectrum disorders (ASDs), are not clear, and there is currently no effective therapy for these serious disorders. Guerrini et al. (Ann Neurol 1996; 40:39-48) studied the movement disorder associated with AS and suggested that treatment with piracetam may improve myoclonus in some patients. Bernard Dan (Epilepsia, 50(11):2331-2339, 2009) reviewed the clinical, neurophysiological and genetic features of AS and discussed the response to piracetam observed in some patients in the context of epileptic symptoms. Thibert et al. (Epilepsia, 50(11):2369-2376, 2009) studied epilepsy in AS patients and suggest that less commonly prescribed anti-epileptic drugs such as levetiracetam and lamotrigine may have similar efficacy in treating the symptoms of epilepsy as commonly prescribed drugs valproic acid and clonazepam.

### SUMMARY

Described herein are compositions and methods for decreasing Arc expression and/or increasing α-amino-3-hydroxy-5-methyl-4-isoxazolepropionic acid receptor (AMPAR) activity to ameliorate the affects (such as cognitive dysfunction) of Ube3A disruption in Angelman Syndrome (AS) and autism spectrum disorders (ASDs). For example, described is a composition for ameliorating the affects of Ube3A disruption comprising an agent that promotes AMPAR expression at neural synapses. Suitable agents may be an antagonist of metabotropic glutamate receptor subtype 5 (mGluR5), such as 2-methyl-6-(phenylethynyl)-pyridine (MPEP), or 3-[(2-methyl-1,3-thiazol-4-yl)ethynyl]pyridine (MTEP). Alternatively, the agent may be an agent that inhibits the activity of, or expression of, the synaptic protein activity-regulated cytoskeleton-associated protein (Arc). The agent may be a positive modulator of AMPAR, i.e. an agent that increases AMPAR activity, increases expression of AMPAR subunits, or reduces desensitization and/or deactivation of AMPAR.

This approach is based on the discovery that experience-driven neuronal activity induces *Ube3A* transcription, and that Ube3A then regulates excitatory synapse development by controlling the degradation of Arc, a synaptic protein that promotes the internalization of the AMPA sub-type of glutamate receptors. Disruption of Ube3A function in neurons leads to an increase in Arc expression and a concomitant decrease in the number of AMPA receptors at excitatory synapses. In the absence of Ube3A, elevated levels of Arc accumulate in neurons resulting in the excessive internalization of AMPA receptors (AMPARs) at synapses and impaired synaptic function. This deregulation of AMPA receptor expression and/or activity at synapses (i.e., impaired AMPAR trafficking) may contribute to the cognitive dysfunction that occurs in Angelman Syndrome and possible other autism spectrum disorders (ASDs). These findings provide therapeutic targets for treating AS, a disorder for which there is currently no effective therapy.

Accordingly, provided herein are agents for use in the treatment of Angelman Syndrome in subjects that are in need of treatment (e.g. human subjects). The treatments comprise administrating to the subject an agent that increases the expression, or increases activity of, α-amino-3-hydroxy-5-methyl-4-isoxazolepropionic acid receptor (AMPAR) at neuronal synapses, wherein the agent comprises 2-methyl-6-(phenylethynyl)-pyridine (MPEP) or 3-[(2-methyl-1,3-thiazol-4-yl)ethynyl]pyridine (MTEP).

Other agents that increases the expression of, or activity of, the α-amino-3-hydroxy-5-methyl-4-isoxazolepropionic acid receptor (AMPAR) at neuronal synapses is an antagonist of metabotropic glutamate receptor subtype 5 (mGluR5) are also envisaged. Non-limiting exemplary antagonists include LY293558 (Eli Lilly); 2-methyl 6-[(1E)-2-phenylethynyl]-pyridine; 6-methyl-2(phenylazo)-3-pyridinol; (RS)-a-methyl-4carboxyphenylglycine (MCPG); 3S,4aR,6S,8aRS-6-((((1Htetrazole-5-yl) methyl)oxy)methyl)-1,2,3,4,4a,5,6,7,8,8 adecahydroisoquinoline-3-carboxylic acid; 3S,4aR,6S,8aR-6((((1H-tetrazole-5-yl)methyl)oxy)methyl)-1,2,3,4,4a,5,6,7, 8,8a-decahydroisoquinoline-3-carboxylic acid; 3SR,4aRS, 6SR,8aRS-6-(((4-carboxy)phenyl)methyl)-1,2,3,4,4a,5,6,7, 8,8a-decahydroisoquinoline-3-carboxylic acid; and 3 S,4aR, 6S,8aR-6-(((4-carboxy)-phenyl)methyl)-1,2,3,4,4a,5,6,7,8, 8a-decahydroisoquinoline-3-carboxylic acid.

Other agents that increase the expression of, or activity of, the α-amino-3-hydroxy-5-methyl-4-isoxazolepropionic acid receptor (AMPAR) at neuronal synapses include a positive modulator of AMPAR selected from the group consisting of: diazoxide; cyclothiazide; 1-(1,3-benzodioxol-5-ylcarbonyl)-piperidine (1-BCP); S18986 [(S)-2,3-Dihydro-[3,4]Cyclopentano-1,2,4-benzothiadiazine-1,1-dioxide); 7-chloro-3-methyl-3,4-dihydro-2H-1,2,4-benzothiadiazine-S,S-dioxide (IDRA21); 7-chloro-3-methyl-3-4-dihydro-2H-1,2,4 benzothiadiazine S,S, dioxide and an ampikine.

In another embodiment, the agent that increases the expression of, or activity of, the α-amino-3-hydroxy-5-methyl-4-isoxazolepropionic acid receptor (AMPAR) at neuronal synapses is an RNA interfering agent (RNAi) directed against the synaptic protein activity-regulated cytoskeleton-associated protein (Arc) and that inhibits the expression of, or inhibits the activity of Arc, such as SEQ ID NO: 9 or SEQ ID NO: 10.

The agents useful in the therapeutic uses of the invention can be administered by any route, e.g. topical administration, enteral administration, and parenteral administration. The agent can be administered in a dose ranging from about 0.1 mg/kg to about 1000 mg/kg.

### DESCRIPTION OF THE DRAWINGS

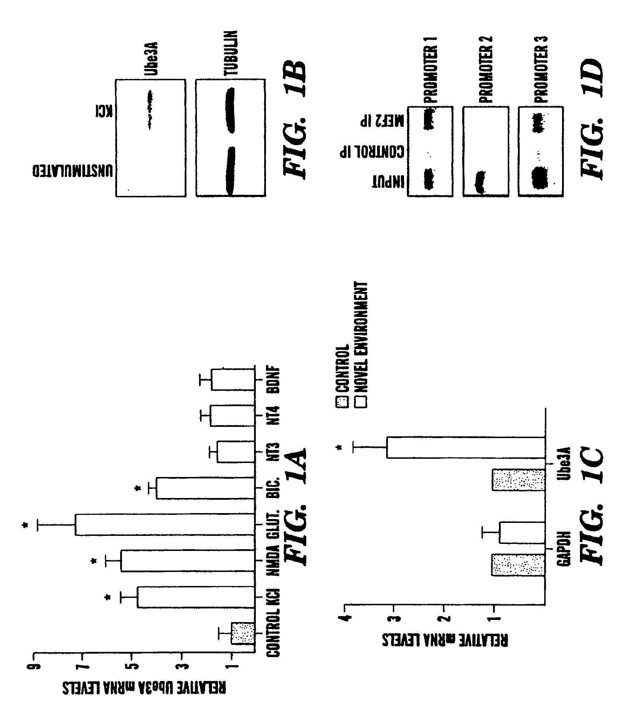

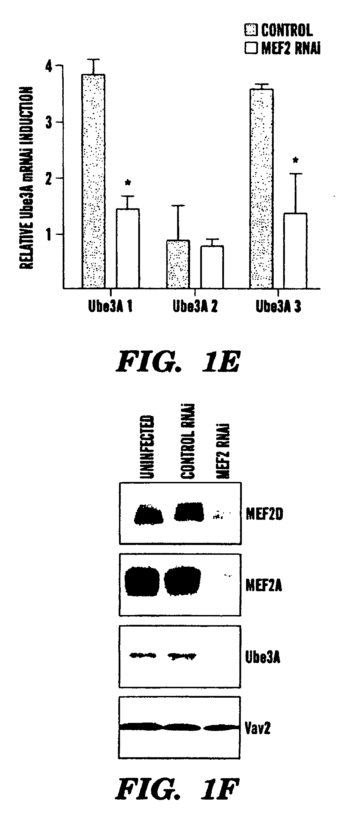

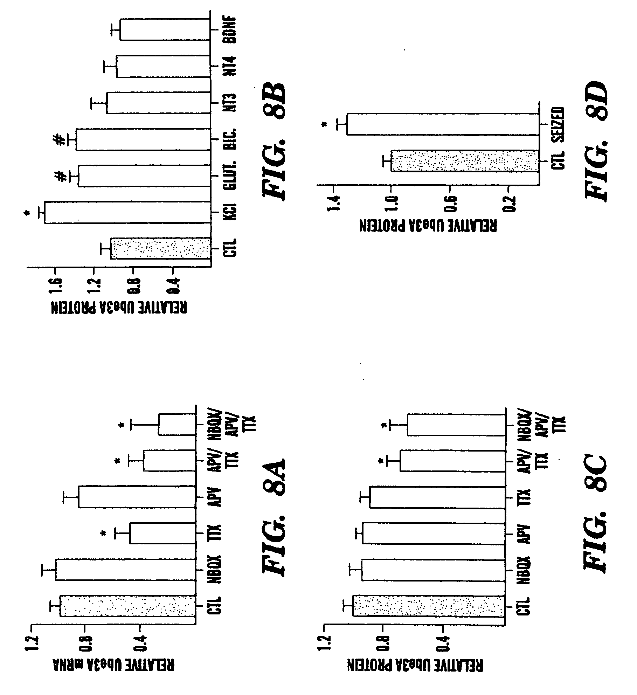

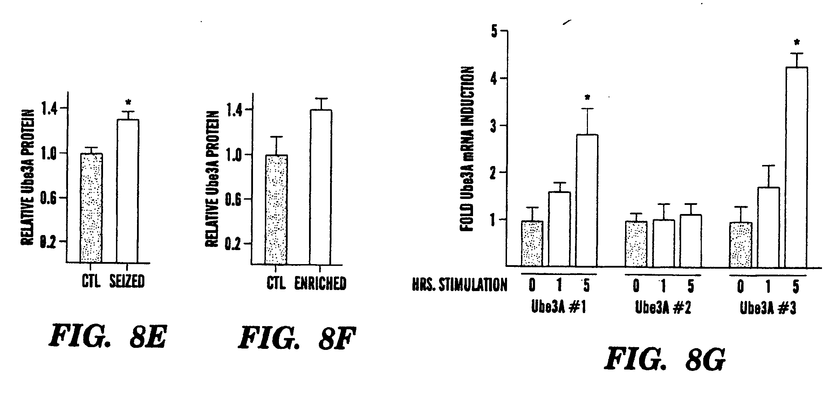

Figures 1A-1F show the regulation of Ube3A by neuronal activity. (Fig. 1A) qRT-PCR analysis of Ube3A mRNA extracted from hippocampal neurons at E18 + 10 days *in vitro* (DIV) stimulated for five hours with the indicated agent (Glut. = glutamate; Bic. = bicuculline). Data are means +/- SEM from three independent experiments. * indicates statistical significance in pairwise comparison to control: P < 0.01 T-test. (Fig. 1B) Western blot analyses of Ube3A and beta-tubulin. Protein lysates were collected from E18 + 10 DIV hippocampal neurons following stimulation with 55 mM KCl for seven hours. Three independent experiments were performed and a representative Western blot is shown. (Fig. 1C) qRT-PCR examining Ube3A and GAPDH mRNA levels in hippocampi of mice placed in standard laboratory cages (control) or in cages with novel objects (novel environment). The expression of Ube3A and GAPDH is normalized to the expression of beta-tubulin which serves as an internal standard. Data are presented as mean +/- SEM from three independent experiments. * indicates statistical significance in pairwise comparison: P < 0.05 T-test. (Fig. 1D) Chromatin immunoprecipitation with control or anti-MEF2 antibodies. PCR amplification is performed on genomic regions corresponding to the promoter regions of the three Ube3A transcripts. (Fig. 1E) qRT-PCR analysis of the three Ube3A transcripts in hippocampal neurons transduced with lentivirus expressing either control shRNA or shRNAs targeting MEF2A and MEF2D. Neurons were stimulated with 55 mM KCl for six hours before mRNA was harvested. Data are plotted as fold induction of stimulated cells over unstimulated cells. Data are presented as mean +/- SEM from three independent experiments. * indicates statistical significance in pairwise comparison: P < 0.01 T-test. (Fig. 1F) Western blot analyses of MEF2D, MEF2A, Ube3A, and the loading control Vav2. Protein lysates were collected from hippocampal neurons at E18 + 10 DIV. Neurons were uninfected or transduced with lentivirus encoding a control shRNA or shRNA targeting MEF2A and MEF2D at E18 + 3 DIV. This experiment was performed three times independently and a representative Western blot is shown here. *See also* Figure 8.

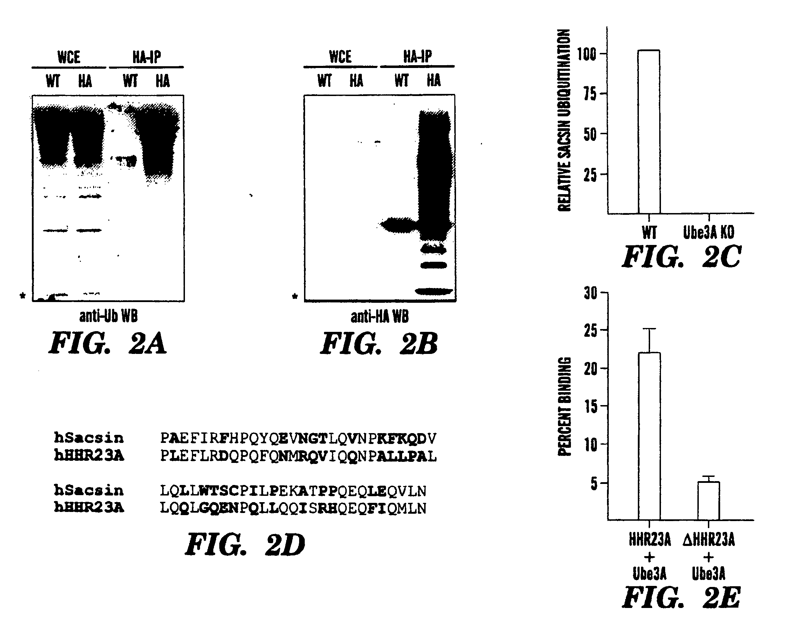

Figures 2A-2E identify a Ubc3A binding domain. (Fig. 2A) Analysis of ubiquitinated proteins in wild type and HA-ubiquitin mice. Western blots using an anti-Ubiquitin antibody were performed on cell lysates (WCE) or anti-HA immunoprecipitates from hippocampal mouse brain lysates prepared from wild type (WT) or HA-ubiquitin transgenice (HA) mice. * indicates the presence of free ubiquitin. (Fig. 2B) Analysis of ubiquitinated proteins in wild type and HA-ubiquitin mice. Western blots using an anti-HA antibody were performed on cell lysates (WCE) or anti-HA immunoprecipitations from hippocampal mouse brain lysates from wild type (WT) or HA-ubiquitin transgenic (HA) mice. * indicates the presence of free ubiquitin. (Fig. 2C) Quantification of the relative abundance of ubiquitinated Sacsin in the brain of wild type and Ube3A knockout mice. No peptides were detected corresponding to ubiquitinated Sacsin in Ube3A knockout mice. (Fig. 2D) Sequence alignment of human Sacsin (SEQ ID NO:1)and human HHR23A (SEQ ID NO:2). Identical residues are shown and similar residues are in bold. (Fig. 2E) Quantitative analysis of *in vitro* binding experiments using recombinant HHR23A, a version of HHR23A lacking the Ube3A binding domain (ΔHHR23A), and Ube3A. Western blotting was performed using an anti-HHR23A antibody. Data are presented as mean +/- SEM from three independent experiments.

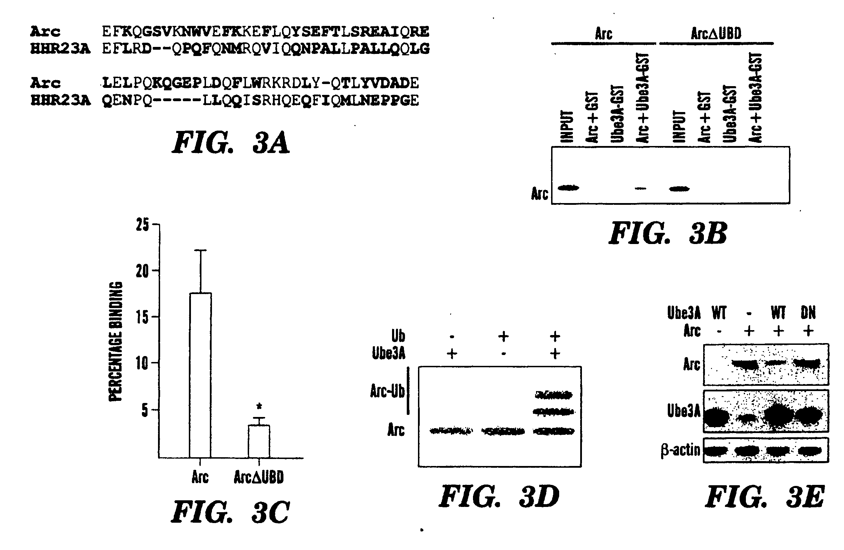

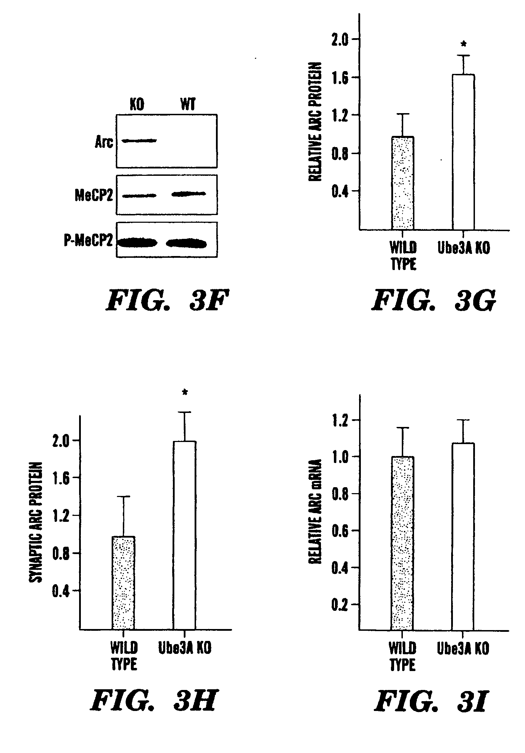

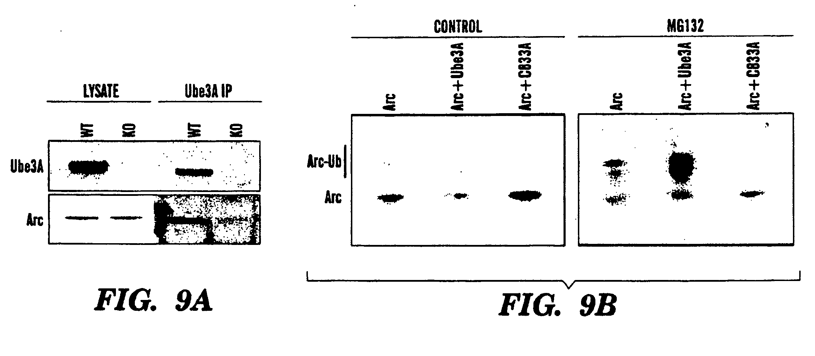

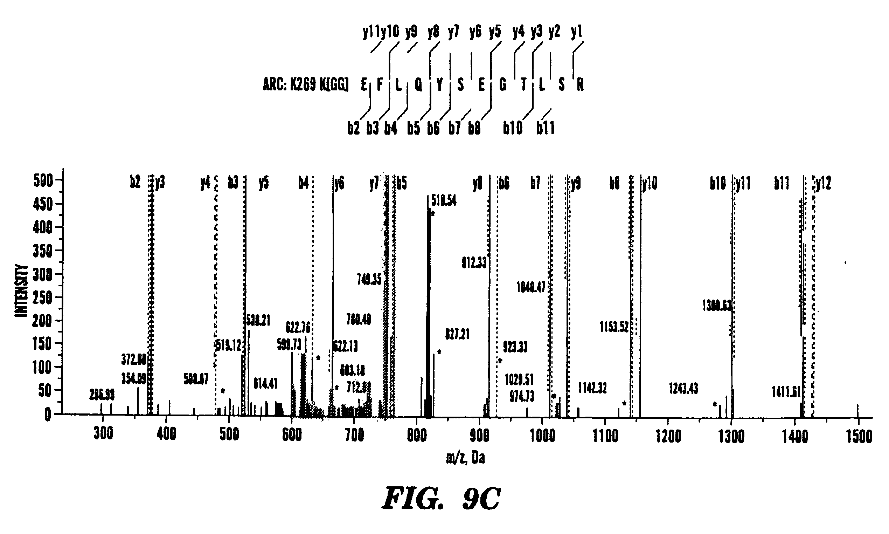

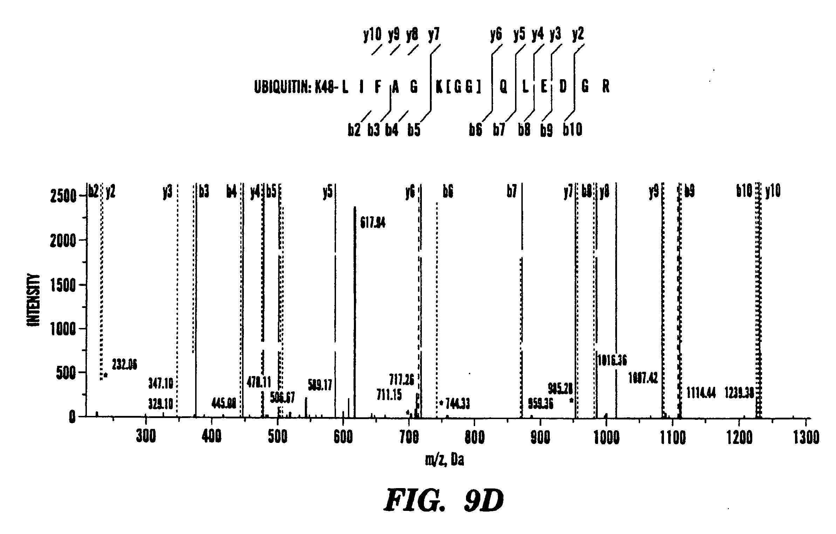

Figures 3A-3I demonstrate that Arc is a Ube3A substrate. (Fig. 3A) Sequence alignment of Arc (amino acids 255-318) (SEQ ID NO: 3) and HHR23A (amino acids 233-290) (SEQ ID NO: 4). Identical residues are shown and similar residues are in bold. Note that as the UBD may represent a sequence that encodes a particular protein folding structure, a strict one-to-one map of specific residues is not observed. (Fig. 3B) *In vitro* binding experiments using recombinant Arc, ArcΔUBD, and GST-tagged Ube3A. (Fig. 3C) Quantitative analysis of in vitro binding experiments using recombinant Arc, or ArcΔUBD, and Ube3A. Western blotting was performed using an anti-Arc antibody. Percentage binding refers to the percent of Arc bound to Ube3A relative to the input. Data are presented as mean +/- SEM from three independent experiments. (Fig. 3D) *In vitro* ubiquitination assay of Arc in the presence of Ubiquitin (Ub), and/or Ube3A. (Fig. 3E) Western blot analysis using anti-Arc, anti-Ube3A, or anti-actin antibodies on lysates from HEK293T cells transfected with the indicated constructs. (Fig. 3F) Western blot analysis of protein lysates prepared from the hippocampi of wild type and Ube3A knockout mice which had been injected with kainic acid. Western blots performed with anti-MeCP2, anti-phospho-MeCP2, and anti-Arc antibodies as indicated. Three individual experiments representing at least five animals per genotype were performed and a representative example is shown. (Fig. 3G) Quantification of Arc protein by Western blot analysis of protein lysates prepared from hippocampi of wild type and Ube3A knockout mice which had been exposed to an enriched environment. Data represent mean +/- SEM from four animals of each genotype. * denotes significance in pairwise comparison to control: P < 0.01 T-test. (Fig. 3H) Quantification of Arc protein by Western blot analysis of protein lysates prepared from synaptosomes isolated from hippocampi of wild type and Ube3A knockout mice which had been injected with kainic acid. Data represent mean +/- SEM from three animals of each genotype. * denotes significance in pairwise comparison to control: P < 0.05 T-test. (Fig. 3I) Real-time quantitative PCR analysis of Arc mRNA extracted from wild type and Ube3A knockout mice seized with kainic acid used in part (Fig. 3F). Data are presented as mean +/- SEM from three independent experiments. *See also* Figure 9.

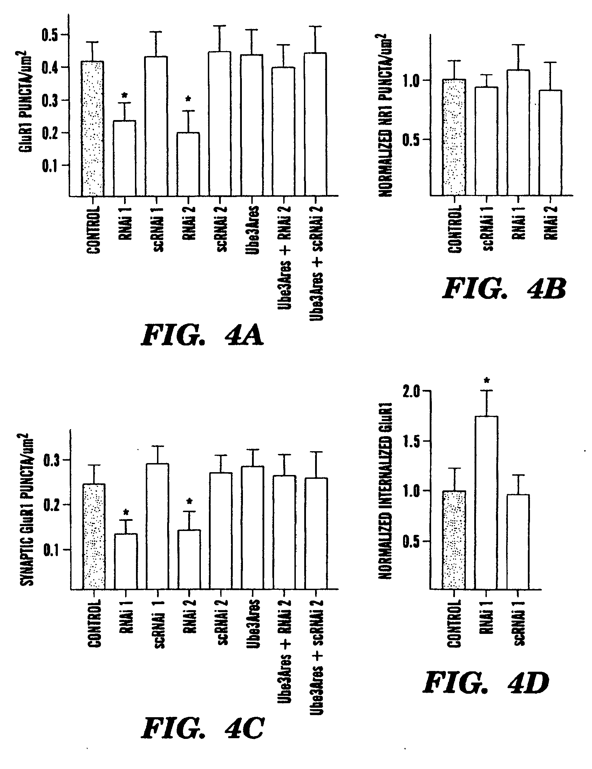

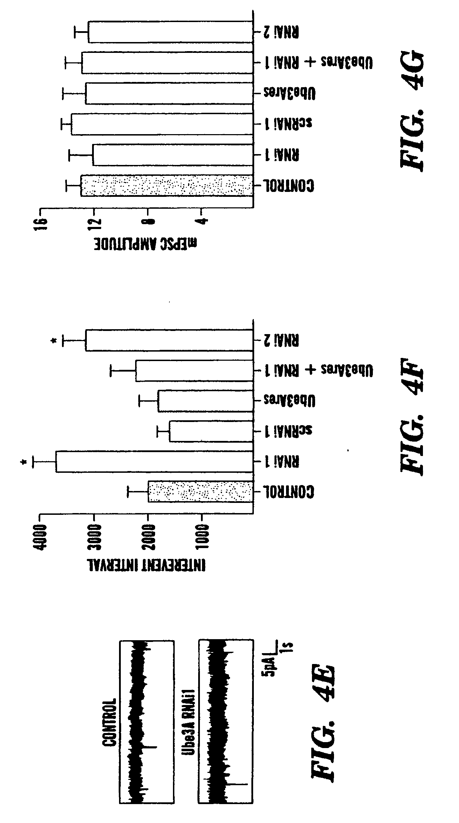

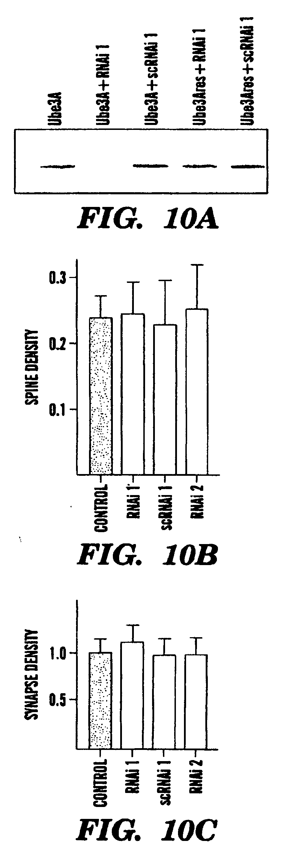

Figures 4A-4G show that Ube3A regulates AMPAR function. (Fig. 4A) Quantification of plasma membrane expression of AMPARs on E18 + 14 DIV hippocampal neurons transfected at 10 DIV with GFP and vector control, either of two shRNAs targeting Ube3A (RNAi 1 or 2), scrambled control shRNA (scRNAi 1 or 2), a form of Ube3A that is resistant to Ube3A shRNA (Ube3Ares) or Ube3A shRNA and Ube3A that is RNAi resistant (Ube3Ares + RNAi 2). At least 35 neurons were imaged for each condition. Data are presented as mean +/- SEM from three independent experiments. * indicates statistical significance P < 0.05, ANOVA using a Bonferroni correction for multiple comparisons. (Fig. 4B) Quantification of plasma membrane expression of NMDA receptors on E18 + 14 DIV hippocampal neurons transfected at 10 DIV with GFP and vector control, either of two shRNAs targeting Ube3A (RNAi 1 or 2), or a scrambled control shRNA (scRNAi 1). At least 20 neurons were imaged for each condition, and data are presented as mean +/- SEM from three independent experiments. (Fig. 4C) Same as in (4A) except only GluR1 puncta that co-localize with PSD95 are counted. At least 29 neurons were imaged for each condition, and data are presented as mean +/- SEM from three independent experiments. * indicates statistical significance P < 0.05, ANOVA using a Bonferroni correction for multiple comparisons. (Fig. 4D) Quantification of internalized GluR1 receptors from E18 + 14 DIV hippocampal neurons transfected at 10 DIV with GFP plus vector, Ube3a shRNA, or control scrambled shRNA. Data are presented as mean +/- SEM from three independent experiments. * indicates statistical significance P < 0.05, ANOVA using a Bonferroni correction for multiple comparisons. (Fig. 4E) Representative mEPSC traces of control transfected (top) or Ube3A RNAi transfected neurons (bottom) used for analysis in (Fig. 4F) and (Fig. 4G). (Fig. 4F) Quantification of mEPSC interevent interval (the time between mEPSC events and thus inversely proportional to mEPSC frequency) from E18 + 14 DIV hippocampal neurons transfected as in part (4A). Data are presented as mean +/- SEM from three independent experiments. * indicates statistical significance P < 0.01, t-test. (Fig. 4G) Quantification of mEPSC amplitude from E18 + 14 DIV hippocampal neurons transfected as in part (Fig. 4A). Data are presented as mean +/- SEM from three independent experiments. *See also* Figure 10.

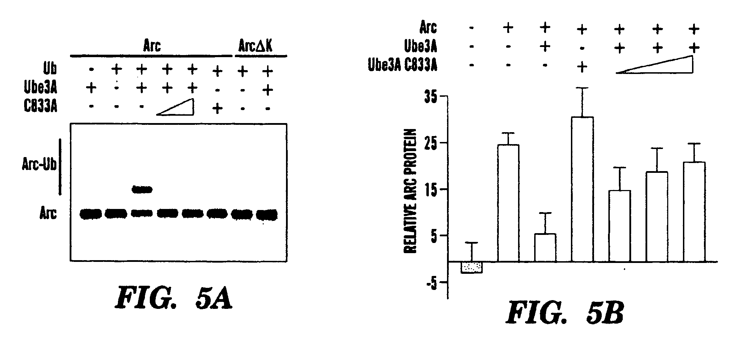

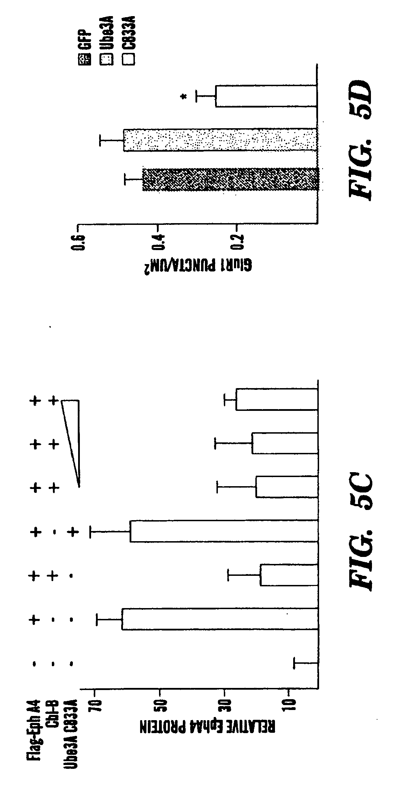

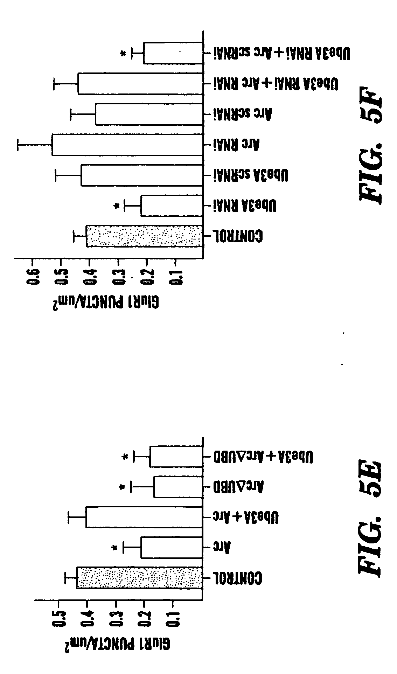

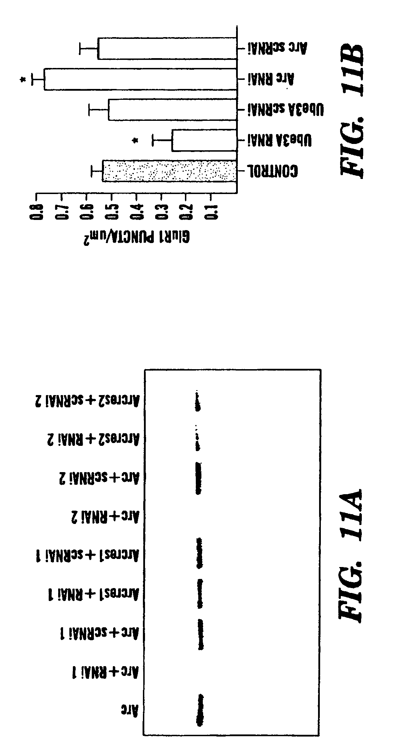

Figures 5A-5F show Ube3A-mediated degradation of Arc affects AMPAR cell surface expression. (Fig. 5A) *In vitro* ubiquitination assay of Arc or a version of Arc in which all lysine residues are mutated to arginine (ArcΔK) in the presence of Ubiquitin (Ub), Ube3A or Ube3A C833A (C833A). Western blotting analysis was performed with an anti-Arc antibody. (Fig. 5B) Quantitative Western blot analysis of protein lysates from HEK293T cells transfected with the indicated constructs. Western blots were performed using an anti-Arc antibody, and the signals were normalized to an actin loading control. (Fig. 5C) Quantitative Western blot analysis of protein lysates from HEK293T cells transfected with the indicated constructs. Western blots were performed using an anti-Flag antibody to detect EphA4, and the resultant values were normalized to an actin loading control. As previously reported Cbl-B promotes the degradation of EphA4 (Sharfe et al., 2003). Cbl-B-mediated degradation of EphA4 is not inhibited by Ube3A C833A, even though Ube3A and Cbl-B can employ the same E2 conjugating enzyme when ubiquitinating substrates. (Fig. 5D) Quantification of surface AMPAR expression for E18 + 17 DIV hippocampal neurons transfected with GFP and vector control, Ube3A, or Ube3A C833A plasmids. At least 30 neurons were imaged for each condition and data are presented as mean +/- SEM from three independent experiments. * indicates statistical significance P < 0.05, ANOVA, with Bonferroni correction for multiple comparison. (Fig. 5E) Quantification of surface AMPA receptor expression on E18 + 14 DIV hippocampal neurons transfected at 10 DIV with vector control, Arc, Ube3A + Arc, ArcΔUBD, or ArcΔUBD + Ube3A. Data are presented as mean +/- SEM from three independent experiments. * denotes statistical significance P < 0.05, ANOVA, with Bonferroni correction for multiple comparison. (Fig. 5F) Quantification of surface AMPAR expression on hippocampal neurons transfected with vector control, Ube3A RNAi, Arc RNAi, Ube3A RNAi and scrambled control Arc RNAi, or Ube3A RNAi and Arc RNAi. Data are presented as mean +/- SEM from three independent experiments. * denotes statistical significance P < 0.05, ANOVA, with Bonferroni correction for multiple comparison. *See also* Figure 11.

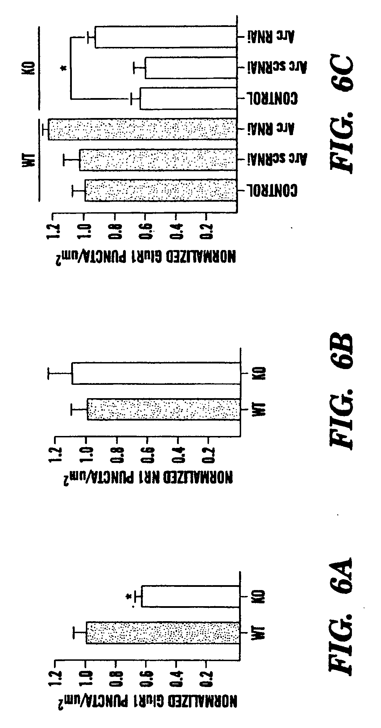

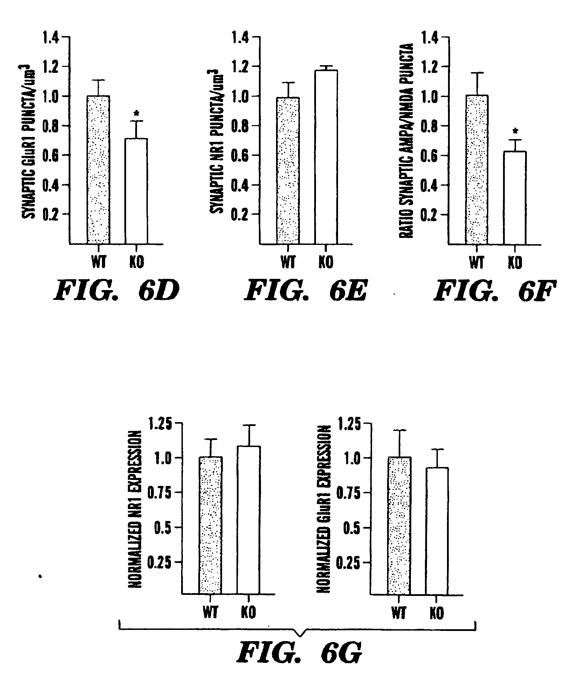

Figures 6A-6G demonstrate that Ube3A knockout mice have fewer synaptically expressed AMPARs. (Fig. 6A) Quantification of plasma membrane expression of AMPARs on P2 + 12 DIV hippocampal neurons isolated from wild type (WT) and Ube3A knockout (KO) animals transfected at 8 DIV with GFP. At least 40 neurons were imaged for each condition, and data are normalized to wild type and presented as mean +/- SEM from three independent experiments. * indicates statistical significance P < 0.01, T-test. (Fig. 6B) Quantification of plasma membrane expression of NMDA receptors on P2 + 12 DIV hippocampal neurons isolated from wild type (WT) and Ube3A knockout (KO) animals transfected at 8 DIV with GFP. At least 24 neurons were imaged for each condition, and data are normalized to wild type and presented as mean +/- SEM from three independent experiments. (Fig. 6C) Quantification of plasma membrane expression of AMPA receptors on P2 + 12 DIV hippocampal neurons isolated from wild type (WT) and Ube3A knockout (KO) animals transfected at 8 DIV with GFP and either vector control, scrambled control shRNAs, or shRNAs targeting Arc. At least 28 neurons were imaged for each condition, and data are normalized to wild type transfected with control and presented as mean +/- SEM from three independent experiments. * indicates statistical significance P < 0.01, ANOVA, with Bonferroni correction for multiple comparisons. (Fig. 6D) Quantification of the number of co-localized GluR1 and SV2 puncta in wild type and Ube3A knockout hippocampi. Data are presented as mean +/- SEM from three independent animals for each genotype. * indicates statistical significance P < 0.01 T-test. (Fig. 6E) Quantification of the number of co-localized NR1 and SV2 puncta in wild type and Ube3A knockout hippocampi. Data are presented as mean +/- SEM from three independent animals for each genotype. P > 0.05, T-test. (Fig. 6F) Analysis of the ratio of the density of GluR1 puncta that co-localize with SV2 to the density of NR1 puncta that co-localize with SV2 obtained from (Fig. 6D) and (Fig. 6E). * indicates statistical significance P < 0.01 T-test. (Fig. 6G) Quantitative Western blot analysis of protein lysates prepared from the hippocampi of P21 wild type and Ube3A knockout mice using anti-NR1 (left panel) and anti-GluR1 (right panel) antibodies. Band intensity was normalized to the intensity of actin to control for differences in protein concentration. Data are presented as mean +/- SEM from three independent experiments..

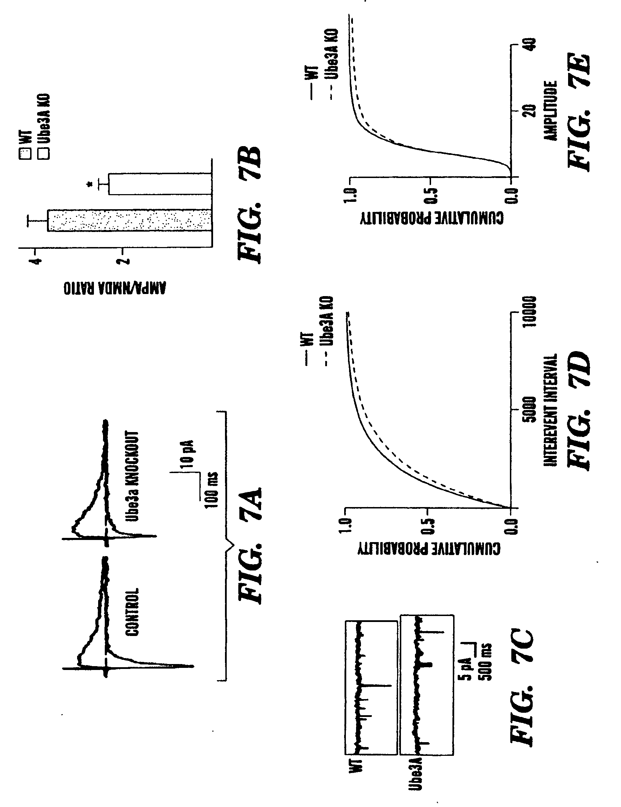

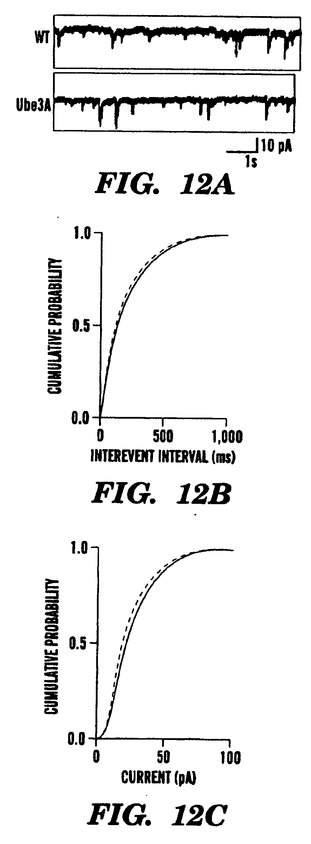

Figures 7A-7E illustrate analysis of synaptic function in the hippocampi of Ube3A knockout mice. (Fig. 7A) Representative traces of currents evoked while holding the neuron at-70 or +40 mV to measure AMPAR or NMDAR-mediated currents, respectively. Examples are shown from a control (left) and Ube3A knockout (right) neuron. Currents are scaled by the current amplitude measured between 50 and 70 ms after the peak of the evoked current at +40 mV to highlight the relative changes in AMPAR-mediated current. (Fig. 7B) A summary histogram of AMPA/NMDA receptor-mediated current ratios presented as the geometric mean +/- SEM. At least 15 cells were analyzed per condition. * p < 0.05 by students t-test of the geometric means for each neuron. (Fig. 7C) Representative mEPSC traces of hippocampal neurons from wild type (top) and Ube3A knockout neurons (bottom). (Fig. 7D) Quantification of mEPSC frequency from wild type (black line) and Ube3A knockout (gray line) mice. Data are presented as cumulative probability plots of interevent intervals and represent recordings from at least 14 neurons from at least three independent animals of each genotype. A significant difference was observed between wild type and Ube3A knockout mice, P < 0.01 by KS test. (Fig. 7E) Quantification of mEPSC amplitude from wild type (black line) and Ube3A knockout (gray line) mice. Data are presented as cumulative probability plots and represent recordings from at least 14 neurons from at least three independent animals of each genotype. No statistically significant difference was observed between wildtype and Ube3A knockout mice by KS test. *See also* Figure 12.

Figures 8A-8G show regulation of Ube3A mRNA and protein by neuronal activity. (Fig. 8A) Real-time PCR analysis of Ube3A mRNA extracted from hippocampal neurons at E18 + 10 DIV treated for six hours with the indicated agent. Data are means +/- SEM from three independent experiments. * indicates statistical significance in pairwise comparison to control: P < 0.01 T-test. (Fig. 8B) Quantitative Western blot analysis of Ube3A protein. Protein lysates were collected from hippocampal neurons at E18 + 8 DIV following treatment with the indicated agent for seven hours. This experiment was performed three times independently and the data were normalized to the control and are presented as means +/- SEM. * indicates P < 0.01, # indicates P < 0.05 in analysis of statistical significance in pairwise comparison to control by T-test. (Fig. 8C) Quantitative Western blot analysis of Ube3A protein. Protein lysates were collected from hippocampal neurons at E18 + 8 DIV following stimulation with the indicated agent for seven hours. This experiment was performed three times independently and the data were normalized to the control and are presented as mean +/- SEM. * indicates P < 0.05 in analysis of statistical significance in pairwise comparison to control by T-test. (Fig. 8D) Real-time PCR examining Ube3A and GAPDH mRNA levels in extracts from hippocampi of control mice injected with saline (ctl) or mice injected with kainic acid (kainate) to induce seizures. The expression of Ube3A and GAPDH is normalized to the expression of beta-tubulin which serves as an internal standard. Data are presented as mean +/- SEM from three independent experiments. * indicates statistical significance in pairwise comparison: P < 0.01 T-test. (Fig. 8E) Quantitative Western blot analysis of Ube3A protein from mice 2.5 hours after injection with saline (ctl) or kainic acid (seized) to induce seizures. Data are presented as mean +/- SEM from three independent experiments. * indicates statistical significance in pairwise comparison P < 0.05 T-test. (Fig. 8F) Quantitative Western blot analysis of Ube3A protein from mice housed in standard laboratory cages (control) or placed in cages with novel objects (enriched) for 2.5 hours. Data are presented as mean +/- SEM from three independent experiments. * indicates statistical significance in pairwise comparison P < 0.05 T-test. (Fig. 8G) Real-time PCR analysis of the three Ube3A transcripts from mRNA extracted from hippocampal neurons at E18 + 10 DIV stimulated for 0, 1, or 5 hours with 55 mM KCl. Data are presented as mean +/- SEM from three independent experiments. * indicates statistical significance in pairwise comparison: P< 0.01 T-test.

Figures 9A-9D demonstrate Ube3A mediates the polyubiquitination and degradation of Arc. (Fig. 9A) Western blot analysis of protein lysates made from brains of wild type and Ube3A knockout mice two hours following kainate acid injection. Immunoprecipitations were performed with an anti-Ube3A antibody and blotted with an anti-Arc antibody to reveal co-immunoprecipitated Arc. Images presented are representative of experiments performed on four independent sets of wildtype and Ube3A knockout mice. (Fig. 9B) Protein lysates were prepared from HEK293T cells transfected with Myc-Arc and HA-tagged ubiquitin and the indicated constructs and then treated with either vehicle control or the proteasome inhibitor MG132 (10 µM, 8 hours). Arc was then immunoprecipitated using the anti-Myc antibody 9E10, and Western blot analysis was performed using an anti-Arc antibody to reveal both non-ubiquitinated and ubiquitinated forms of Arc. (Fig. 9C) Mass spectrometric peaks reveal that Ube3A catalyzes the ubiquitination of Arc on lysine 269. Top panel reveals the peptide (SEQ ID NO: 5) assigned to the spectra on the bottom. SEQ ID NO: 5 is KGGEFLQYSEGTLSR (SEQ ID NO: 5) shown. Note the presence of two glycine residues covalently linked to the first lysine of this peptide which is indicative of ubiquitin being attached to that specific residue. The spectra depicted in the bottom panel shows the intensity of peaks on the Y-axis and the mass:charge ratio on the X-axis. Additional data not pictured here reveal the presence of ubiquitinated lysine 268 as well. (Fig. 9D) Similar to (Fig. 9C) but this spectra reveals the presence of ubiquitin conjugates on ubiquitin isolated from Arc immunoprecipitates, suggesting that Arc is polyubiquitinated by Ube3A. SEQ ID NO: 6 is LIFAGKGGQLEDGR (SEQ ID NO: 6) shown in upper panel of Fig. 9D.

Figures 10A-10C demonstrate that Ube3A RNAi reduces Ube3A protein expression. (Fig. 10A) Western blot analysis of Ube3A from protein lysates prepared from HEK293T cells transfected with the indicated construct(s). (Fig. 10B) Quantification of dendritic spine density from E18 + 14 DIV hippocampal neurons transfected at 10 DIV with GFP and vector control, either of two shRNAs targeting Ube3A (Ube3A RNAi 1 or 2) or scrambled control shRNA (Ube3A scRNAi 1). Data are presented as mean +/- SEM from three independent experiments. (Fig. 10C) Quantification of the overlap of PSD95 and synapsin1 puncta on E18 + 14 DIV hippocampal neurons transfected at 10 DIV with GFP and vector control, either of two shRNAs, targeting Ube3A (Ube3A RNAi 1 or 2) or scrambled control shRNA (Ube3A (scRNAi 1). Data are normalized to control and presented as mean +/- SEM from three independent experiments.

Figures 11A-11B show surface GluR1 expression. (Fig. 11A) Western blot analysis of extracts from HEK293T cells transfected with Arc alone, or in combination with either of two Arc shRNA constructs (RNAi 1 or 2), either of two control shRNAs (scRNAi 1 or 2), or either of two forms of Arc that are subtly mutated and thus resistant to the shRNAs (Arcres 1 or 2). Western blots were then performed on lysates from the transfected cells using an anti-Arc antibody. (Fig. 11B) Quantification of surface expression of GluR1 receptors from E18 + 19 DIV hippocampal neurons transfected with GFP and vector control, Ube3A RNAi, Ube3A scRNAi, Arc RNAi, or Arc scRNAi from Data are presented as mean +/- SEM from three independent experiments. * indicates statistical significance P < 0.05, ANOVA, with Bonferroni correction for multiple comparison.

Figures 12A-12C show that mIPSCs are unaltered in Ube3A knockout mice. (Fig. 12A) Representative mIPSC traces of hippocampal neurons from wild type (top) and Ube3A knockout neurons (bottom). (Fig.12B) Quantification of mIPSC frequency from wild type (solid line) and Ube3A knockout (dashed line) mice. Data are presented as cumulative probability plots of interevent intervals and represent recordings from at least 15 neurons from at least three independent animals of each genotype. (Fig. 12 C) Quantification of mIPSC amplitude from wild type (solid line) and Ube3A knockout (dashed line) mice. Data are presented as cumulative probability plots and represent recordings from at least 15 neurons from at least three independent animals of each genotype.

### DETAILED DESCRIPTION

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

As used herein and in the claims, the singular forms include the plural reference and vice versa unless the context clearly indicates otherwise. Other than in the operating examples, or where otherwise indicated, all numbers expressing quantities of ingredients or reaction conditions used herein should be understood as modified in all instances by the term "about."

All patents and other publications identified are cited herein for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

Angelman Syndrome (AS) is a neurodevelopmental disorder characterized by motor dysfunction, severe mental retardation, speech impairment, seizures, and a high prevalence of autism (Williams et al., 140, Am. J. Med. Genet. A. 413-18 (2006)). Genetic studies revealed that AS is associated with maternal deletions of chromosome 15q11-q13, paternal chromosome 15 uniparental disomy, or rare imprinting defects that affect the transcription of genes within 15q11-q13 (Clayton-Smith & Laan, 40 J. Med. Genet. 87-95 (2003)). Recent studies indicate that failure to inherit a normal maternal copy of the *UBE3A* gene (which resides within 15q11-q13) accounts for 85% to 90% of AS cases and specific loss-of function mutations in human *UBE3A* have been identified in a subset of affected individuals (Kishino et al., 15 Nat. Genet. 70-73 (1997); Matsuura et al., 15 Nat. Genet. 74-77 (1997)).

The role of *Ube3A* mutations in AS is supported by targeted inactivation of Ube3a in mice (Jiang et al., 21 Neuron 799-811 (1998); Miura et al., 9 Neurobiol. Dis. 149-59 (2002)). Upon inheritance of the mutation through the maternal germline, the mutant mice display features of AS. The finding that imprinting of Ube3A occurs in specific brain regions, reinforces the idea that loss of Ube3A function in the nervous system underlies AS (Jiang et al., 1998; Albrecht et al., 17 Nat. Genet. 75-78 (1997)).

The study of *Ube3A* mutations also provides insight into the causes of autism. Autism spectrum disorders (ASDs) are complex disorders characterized by an impairment in social interactions and the occurrence of repetitive behaviors. Despite the high prevalence of ASDs, little is known about the etiology of these disorders. Nonetheless there is a significant genetic component to ASDs, and thus considerable effort has gone into identifying genetic mutations that cause ASDs. These studies suggest that *Ube3A* is a candidate ASD gene. Abnormalities within chromosomal region 15q11-q13 are among the most prevalent mutations identified in ASDs, accounting for 1% to 2% of all ASD cases (Sutcliffe et al., 42 J. Am. Acad. Child Adoles. Psychiatry 253-56 (2003); Cook et al., 60 Am. J. Hum. Genet. 928-34 (1997)). Recent reports indicate that copy number variance within the *Ube3A* locus is associated with autism (Glessner et al., Nature 2009).

Despite the critical role that Ube3A plays in human cognitive function, little is known about Ube3A's contribution to nervous system development or how the mutation of *Ube3A* leads to cognitive impairment. Electrophysiological experiments have demonstrated impaired long term potentiation (LTP) in *Ube3A* knockout mice (Jiang et al., 1998). Additionally, a recent study implicates Ube3A in experience-dependent plasticity (Yashiro et al., Nature Neurosci. 2009)). Although these experiments demonstrate a crucial role for Ube3A in synaptic transmission, the mechanisms by which Ube3A regulates synaptic function are poorly understood. Possible insight into how Ube3A functions may come from the finding that Ube3A is a member of the E3 ubiquitin ligase family of enzymes, a class of proteins that catalyzes the addition of ubiquitin moieties to target substrates, often leading to the degradation of the ubiquitinated protein. Genetic studies indicate that the ubiquitin ligase activity of Ube3A is necessary for normal human cognitive function inasmuch as disruption of this activity leads to AS (Cooper et al., 279 J. Biol. Chem. 41208-17 (2004)). Nevertheless, the neuronal substrates of Ube3A that mediate its effects on synaptic function remain unknown.

The present invention is based upon the systematic determination of how disruption of Ube3A results in synaptic dysfunction. We have discovered that Ube3A is a neuronal activity-regulated protein that controls synaptic function by ubiquitinating and degrading the synaptic protein Arc. In the absence of Ube3A, elevated levels of Arc accumulate in neurons resulting in the excessive internalization of AMPA receptors (AMPARs) at synapses and impaired synaptic function. Not to be bound by theory, this impaired AMPAR trafficking may be a cause of the cognitive dysfunction that occurs in AS. These findings provide therapeutic targets for treating AS, a disorder for which there is currently no effective therapy.

More specifically, regulation of Ube3A is activity dependent. One clue as to how Ube3A might function in nervous system development comes from the observation that the symptoms of AS and ASDs become apparent within the first years of a child's life (Williams et al., 2006) during which sensory experiences play a key role in shaping neuronal connectivity. The effect of environmental cues on cognitive development is mediated in part by the release of glutamate at excitatory synapses. This triggers a program of gene expression that plays a critical role in synapse development (Greer & Greenberg, 59 Neuron 846-60 (2008)). This raises the possibility that AS may arise from a deficit in activity dependent regulation of Ube3A.

The expression of *Ube3A* mRNA in cultured neurons was significantly increased by either membrane depolarization or glutamate receptor activation (Figure 1A). Conversely, blocking neuronal activity with inhibitors of NMDARs, AMPARs and sodium channels results in a decrease in *Ube3A* mRNA expression (Figure 8A). Ube3A protein levels mirrored the change in mRNA level under these conditions. (Figure 1B, 8B, and 8C).

Whether Ube3A expression is induced by neuronal activity was studied in the intact mouse brain. During kainate-induced seizures, *Ube3A* mRNA and protein levels are increased compared to control (Figure 8D and 8E). Ube3A is also induced in response to environmental stimuli that trigger experience-dependent synaptic development (Figure 1C and 8F). Mice in a cage containing novel objects to induce exploratory behavior exhibited increased *Ube3A* mRNA and protein expression compared to mice in a standard laboratory cage (Figure 1C and 8F). These results demonstrate that *Ube3A* mRNA and Ube3A protein levels are regulated by synaptic activity both in culture and in the intact brain. These findings raise the possibility that synaptic glutamate release during early life experiences activates Ube3A expression, and that the absence of experience-dependent Ube3A induction may contribute to the neurological impairment in AS.

The mechanism by which neuronal activity triggers *Ube3A* induction was also investigated. Analysis of *Ube3A* transcripts present in EST databases revealed three distinct mRNA transcripts that are likely transcribed from unique promoters. Of the *Ube3A* transcripts, those initiating from promoters 1 and 3 were induced by neuronal activity (Figure 8G), and their promoters contain binding sites for the activity regulated transcription factor MEF2. These sites are conserved across phylogeny, and lie within 2kB of the putative transcriptional start sites of the two activity-regulated *Ube3A* transcripts as shown herein. The presence of potential MEF2-binding sites within *Ube3A* promoters was of interest because MEF2 is an activity-regulated transcription factor that controls synapse development and regulates genes implicated in ASDs (Flavell et al., 331 Science 1008-12 (2006); Flavell et al., 60 Neuron 1022-38 (2008); Morrow et al., 321 Science 218-23 (2008)).

Chromatin immunoprecipitation experiments revealed that DNA fragments corresponding to *Ube3A* promoters 1 and 3 are enriched in anti-MEF2 immunoprecipitates (Figure 1D). By contrast, there was no enrichment for DNA sequences surrounding *Ube3A* promoter 2 (Figure 1D). These data suggest that MEF2 may directly control the activity dependent transcription of *Ube3A* from promoters 1 and 3.

The neuronal activity-dependent induction of Ube3A promoter 1- and 3-driven mRNA transcripts and Ube3A protein are significantly reduced in neurons infected with lentiviruses encoding shRNAs targeting the MEF2 family members MEF2A and MEF2D (Figure 1E, 1F, and 8G). By contrast, the expression of Ube3A promoter 2-dependent mRNA transcripts as well as GAPDH, and beta3-tubulin are unaffected by the presence of MEF2 shRNA (Figure 1E). These experiments indicate that in response to neuronal activity, Ube3A promoter 1- and 3-driven mRNA transcripts and Ube3A protein expression are induced by a MEF2-dependent mechanism.

Ube3A substrates were also identified. Regulation of *Ube3A* mRNA expression by neuronal activity along with the association of Ube3A with AS, led us to investigate the role of Ube3A in nervous system development. Point mutations within the *Ube3A* coding region have been associated with AS, nearly all of which abrogate its E3 ubiquitin ligase activity (Cooper et al., 2004), suggesting that the catalytic activity of Ube3A is important for nervous system development.

Although several Ube3A substrates have been identified in non-neuronal cells, the identification of substrates of E3 ubiquitin ligases has been challenging. Ube3A substrates were identified using a transgenic mouse in which a Hemagglutin epitope tagged-version of ubiquitin (HA-ubiquitin) is knocked into the HPRT locus (Ryu et al., 26 EMBO J. 2693-706 (2007)). These mice express similar levels of free ubiquitin in their brains to that detected in the brains of wild type mice (Figure 2A). In addition, in the HA-ubiquitin mice HA-ubiquitin appears to be efficiently incorporated into substrates (Figure 2A and B). HA-ubiquitin transgenic mice were crossed with wild type or *Ube3A* knockout mice and immunoprecipitated HA-ubiquitinated proteins from brain lysates of these mice. Ubiquitinated proteins in wild type and *Ube3A* knockout mice were compared using quantitative mass spectrometry. If a given protein were a substrate of Ube3A, then in the absence of Ube3A it would be less ubiquitinated and thus less efficiently precipitated with anti-HA antibodies. Thus, HA- ubiquitinated proteins were identified whose abundance was decreased in *Ube3A* knockout mice.

The protein Sacsin was identified as a candidate Ube3A substrate. Peptides corresponding to ubiquitinated Sacsin were present in brain lysates of wild type but not Ube3A knockout mice, suggesting that Sacsin might not be efficiently ubiquitinated in the absence of Ube3A (Figure 2C). Sacsin is of interest as it is mutated in Charelvoix-Saguenay spastic ataxia, a neurological disorder with similarities to AS (Engert et al., 24 Nat. Genet 120-25 (2000)). Little is known about Sacsin's role in nervous system development, however, and the large size of the Sacsin protein suggested it would be difficult to study. Nevertheless, Sacsin has a 60 amino acid stretch that has similarity to a previously identified Ube3A substrate, HHR23A (Figure 2D). This region of homology corresponds to a well-characterized region of HHR23A consisting of five amphipathic helices suggesting that the corresponding region in Sacsin may have a similar structure (Kamionka & Feigon, 13 Protein Sci. 2370-77 (2004)). As the specificity of ubiquitin ligases is most strongly determined by substrate binding, we hypothesized that this region of similarity between Sacsin and HHR23A might serve as a Ube3A binding domain (UBD) that might be present in other Ube3A targets.

A mutant form of HHR23A was generated *(ΔHHR23A)* that lacks the UBD and assessed its ability to interact with, and be ubiquitinated by Ube3A. Although wild type HHR23A efficiently interacts with Ube3A, mutation of the *UBD* in *HHR23A* blocks this interaction (Figure 2E). Likewise, this domain is required for Ube3A to ubiquitinate HHR23A. These results suggest the existence of a motif on Ube3A substrates that mediates binding to Ube3A.

A search of mammalian genomes for proteins that contain the UBD identified proteins including the synaptic protein Arc and the RhoGEF ephexin 5 as potential Ube3A substrates (Figure 3A and Margolis et al., submitted). Arc was of interest because Arc regulates the trafficking of alpha-amino-3-hydroxy-5-methyl-4-isoxazole-propionate (AMPA) type of glutamate receptors at synapses. If Arc is a substrate of Ube3A such a finding could potentially begin to explain Ube3A's role in synaptic function (Chowdhury et al., 52 Neuron 445-59 (2006); Rial Verde et al., 52 Neuron 461-74 (2006); Shepherd et al., 52 Neuron 475-84 (2006)). Furthermore, like *Ube3A, Arc* transcription is regulated by neuronal activity through the action of MEF2 family transcription factors (Flavell et al., 2006) suggesting that these two proteins might function together in response to synaptic activation.

Purified Arc binds Ube3A in a manner that is dependent upon the UBD within Arc (Figure 3B and 3C). Co-immunoprecipitation experiments using mouse brain extracts confirmed that Arc and Ube3A also interact in the intact brain (Figure 9A). *In vitro* ubiquitination assays using purified recombinant proteins showed that Ube3A ubiquitinated Arc *in vitro* but did not ubiquitinate the control proteins p53 or MeCP2 (Scheffner et al., 75 Cell 495-505 (1993)) (Figure 3D). A catalytically inactive form of Ube3A, (Ube3A C833A), was incapable of catalyzing the ubiquitination of Arc (Kumar et al., 274 J. Biol. Chem. 18785-92 (1999)) (Figure 5A).

Whether Ube3A promotes the ubiquitination of Arc within cells was tested by transfecting HEK 293T cells with Arc and either Ube3A C833A or wild type Ube3A. Co-expression of wild type Ube3A, but not Ube3A C833A, led to a decrease in the level of Arc (Figure 3E). Incubation of transfected HEK293T cells with the proteasome inhibitor, MG132, blocked Ube3A-mediated degradation of Arc, suggesting that Ube3A degrades Arc via the ubiquitin proteasome (Figure 9B). The ubiquitination of Arc by Ube3A was confirmed by mass spectrometry (Figures 9C and 9D).

Arc expression in the brains of wild type mice was compared with that in *Ube3A* knockout mice. As the expression of both Ube3A and Arc is enhanced by neuronal activity, the mice were exposed to kainic acid or an enriched environment to boost the levels of Ube3A and Arc protein. Under these conditions, higher levels of Arc protein were detected in Ube3A knockout mice than in wild type controls (Figures 3F-3H). These findings suggest that Ube3A ubiquitination of Arc in the wild type brain contributes to Arc degradation. In contrast to Arc, the activity dependent phosphorylation of the transcriptional regulator MeCP2, and the induction of the activity regulated transcription factor NPAS4 are similar in wild type and *Ube3A* knockout brains suggesting that the increase in Arc in *Ube3A* knockout mouse brain is not the result of an overall increase in the activity dependent gene response (Figure 3G) (Zhou et al., 52 Neuron 255-69 (2009); Lin et al., 455 Nature 1198-204 (2008)). Furthermore, Arc mRNA levels are similar in the brains of wild type and *Ube3A* knockout mice indicating that the increase in the level of Arc protein detected in Ube3A knockout neurons is likely due to a defect in Ube3A-mediated degradation of Arc (Figure 3I). That Arc is ubiquitinated by Ube3A *in vitro* and in intact cells, and that the level of Arc protein is significantly higher in *Ube3A* knockout mice shows that Arc is a Ube3A substrate and that the decreased ubiquitination of Arc in *Ube3A* knockout mice results in increased levels of Arc in the brains of these animals.

Arc regulates the surface expression of AMPA receptors (AMPARs), mediators of fast excitatory neurotransmission in the CNS. Reducing Arc expression leads to an increase in the surface expression of AMPARs, whereas increasing Arc levels decreases the plasma membrane expression of AMPARs (Chowdhury et al., 2006; Rial Verde et al, 2006; Shepherd et al., 2006). As Arc levels are elevated in the absence of Ube3A, it is possible that there is a concomitant decrease in the expression of AMPARs on the plasma membrane. Such a finding would suggest a mechanism for the cognitive dysfunction observed in individuals with AS.

Reducing Ube3A expression might decrease the plasma membrane expression of AMPARs. Thus, Ube3A expression was decreased by transfecting neurons with shRNAs that target Ube3A expression and then assessed the surface expression of AMPARs as determined by Western Blot anlalysis (Figure 10A) and confocal imiages of hippocampal neurons transfected with Ube3A shRNA and GFP (data not shown). The focus was on the GluR1 subunit of the AMPA receptor because GluR1 insertion into the plasma membrane is regulated by neuronal activity and by Arc (Newpher & Ehlers, 58 Neuron 472-97 (2008); Kessels & Malinow, 61 Neuron 340-50 (2009); Rial Verde et al, 2006; Shepherd et al., 2006). To examine GluR1 expression at the plasma membrane of neurons, hippocampal neurons were stained with anti-GluR1 antibodies under non-permeabilizing conditions and quantified the number of GluR1 puncta expressed on the cell surface. Expression of either of two shRNAs targeting Ube3A resulted in a reduction in the levels of GluRl expressed at the plasma membrane that is rescued by co-expression of an RNAi-resistant form of Ube3A (Figure 4A). This decrease in surface GluR1 was not due to a change in the expression of AMPARs as wild type and Ube3A-deficient cells expressed similar levels of GluR1 and GluR2 subunits (data not shown). Furthermore, the plasma membrane expression of NR1 subunits of the NMDA receptor was unaltered in Ube3A-deficient cells (Figure 4B).

Because AMPA receptors are trafficked in and out of synapses, the effect of Ube3A knockdown on surface postsynaptic AMPA levels was examined, quantifying the number of GluR1 cell surface puncta that co-localize with the postsynaptic scaffolding protein PSD95. shRNAs targeting Ube3A caused a reduction in the number of GluRl puncta colocalizing with PSD95, indicating that Ube3A regulates recruitment of AMPA receptors to the post-synaptic region (Figure 4C).

Whether AMPAR endocytosis is enhanced in the absence of Ube3A was examined using GIuR1-specific antibodies to label surface AMPARs on neurons transfected with shRNAs targeted to Ube3A. Following membrane depolarization to induce the endocytosis of synaptic AMPARs, anti-GluR1 antibodies bound to the remaining surface GluR1 subunits were removed by acid stripping (Man et al., 104 P.N.A.S. 3579-84 (2007)). Subsequent permeabilization of the cells and staining with fluorescent secondary antibodies to detect the internalized component of GluR1, revealed increased levels of endocytosed GluR1 in Ube3A shRNA-expressing cells compared to control shRNA-transfected neurons (Figure 4D). Thus, the decreased expression of AMPARs in the plasma membrane of synapses of Ube3A-deficient cells is due, at least in part, to an increase in AMPAR endocytosis.

Whether increased AMPAR endocytosis affects AMPAR function at synapses, were investigated by recording miniature excitatory post synaptic currents (mEPSCs) in neurons expressing Ube3A-directed shRNAs. Compared to control shRNAs, the transfection of Ube3A shRNAs results in a significant decrease in mEPSC frequency with no change in mEPSC amplitude (Figure 4E, 4F, and 4G). This decrease in mEPSC frequency could be rescued by co-expression of an RNAi-resistant form of Ube3A. As mEPSC frequency is a measure of AMPAR-mediated synaptic transmission, this observation suggests that AMPAR function is altered at synapses of Ube3A deficient neurons.

Without being bound by theory, the observation that when Ube3A expression is knocked down there is a reduction in mEPSC frequency with no change in mEPSC amplitude could be explained by any of several possibilities: (a) a reduction in the number of synapses formed on the Ube3A deficient neuron, (b) reduced presynaptic probability of neurotransmitter release from neurons that synapse onto Ube3A deficient neurons, or (c) a subset of synapses that form on Ube3A deficient neurons could lack AMPA receptors and thus would be "silent synapses", not readily detected by mEPSC recordings. To distinguish between these possibilities, whether there are fewer synapses formed when Ube3A is knocked down was examined. At the time point of analysis where reduced mEPSC frequency was detected, there was no significant change in dendritic spine density or the number of synapses that form on Ube3A shRNA expressing neurons (Figure 10B and 10C). These findings, and the absence of any detectable change in the formation of inhibitory synapses, neuronal morphology, or cell survival associated when Ube3A expression is knocked down, suggest that the decrease in mEPSC frequency does not reflect a decrease in the number of synaptic connections formed on Ube3A-deficient neurons.

Although it is possible that a decrease in Ube3A expression in the post synaptic neuron reduces the presynaptic probability of release, the hypothesis that the loss of Ube3A leads to the elimination of AMPAR expression from a subset of synapses is supported by a number of reasons including: (a) loss of Ube3A function results in an increase in the levels of Arc, a protein whose expression has been shown to promote the endocytosis of AMPAR, (b) in the absence of Ube3A there were fewer GluR1 puncta that colocalize with PSD95, suggesting that when the level of Ube3A protein is reduced there are synapses that may not express AMPARs, (c) there is a reduction in the ratio of AMPA/NMDA receptor-mediated transmission in Ube3A knockout neurons consistent with the idea that some synapses that form on Ube3A-deficient neurons lack AMPARs.

Arc mediates the effect of Ube3A on AMPAR trafficking, and Ube3A enhances AMPAR endocytosis by ubiquitinating and degrading Arc. If the enhanced AMPAR endocytosis observed following Ube3A knockdown is mediated by the dysregulation of the ubiquitination of Arc, then (a) Ube3A's ubiquitin ligase activity would be required for its effect on AMPAR endocytosis; (b) over-expression of Arc would phenocopy the loss of Ube3A and reduce AMPAR plasma membrane expression; and (c) in Ube3A-deficient cells, restoring Arc expression to the level seen in wild type neurons should rescue the decrease in GluR1 surface expression observed in the absence of Ube3A.

Thus, whether the ubiquitin ligase activity of Ube3A is required for Ube3A to promote AMPAR expression at synapses was investigated by generating a Ube3A mutant in which the cysteine residue within the active site of the Ube3A ligase is mutated to alanine (Ube3A C833A). When overexpressed, this mutant should act in a dominant interfering manner to block the ability of endogenous Ube3A to ubiquitinate its substrates. Indeed, over-expression of Ube3A C833A blocked the ability of wildtype Ube3A to ubiquitinate its substrates (Figures 5A, 5B, and 5C). To determine if Ube3A's ubiquitin ligase activity is required for Ube3A to enhance AMPAR expression at synapses, neurons were transfected with wild type Ube3A or Ube3A C833A. Overexpression of Ube3A C833A, but not wild type Ube3A, caused a significant reduction in the number of AMPARs present on the cell surface, suggesting that Ube3A ubiquitin ligase activity is critical to the ability of Ube3A to promote expression of AMPARs at synapses (Figure 5D and Figure 11).

Whether the overexpression of Arc phenocopies the loss of Ube3A and reduces AMPAR expression was also examined. As previously reported, the over-expression of Arc results in a decrease in the plasma membrane expression of GluR1 (Chowdhury et al., 2006; Rial Verde et al, 2006; Shepherd et al., 2006) (Figure 5E). Co-expression of Ube3A with wild type Arc attenuates the ability of Arc to promote the endocytosis of GluR1. When a version of Arc lacking the UBD (Arc□UBD) was over-expressed in neurons, this form of Arc still promoted the endocytosis of GluR1 but the co-expression of Ube3A did not reverse this effect (Figure 5E). This suggests that Ube3A's ability to reduce the endocytosis of AMPARs is due to Ube3A-mediated degradation of Arc.

To further investigate if the ability of Ube3A to promote the expression of AMPARs at synapses is due to Ube3A dependent Arc ubiquitination and degradation, neurons were transfected with shRNAs targeting Ube3A to reduce Ube3A expression and/or shRNA directed against Arc to decrease Arc expression and the effect on AMPAR cell surface expression assessed. As described above, the expression of shRNAs targeting Ube3A in neurons led to a reduction in the number of AMPARs at the neuronal cell surface (Figure 5F). Introduction of shRNAs directed against Arc, but not control shRNAs, significantly reduced Arc expression in HEK293T cells (Figure 11A) and when transfected into neurons caused a small but statistically insignificant increase in surface AMPAR expression (Figure 5F). The failure of Arc shRNAs when transfected alone to affect AMPAR surface expression likely reflects the fact that given the low level of neuronal activity in these cultures Arc levels are also quite low and only minimally affect AMPAR surface expression.

Consistent with this possibility, in older cultures the expression of Arc shRNAs resulted in an increase in AMPAR plasma membrane expression (Figure 11B). The lack of significant Arc expression in younger neuronal cultures may also explain why over-expression of Ube3A does not significantly affect the plasma membrane expression of AMPARs in younger neuronal cultures. Expressing shRNAs against Ube3A, together with an shRNA directed against Arc, blocked the ability of Ube3A shRNA to suppress AMPAR expression at synapses (see Figures 5F, confirmed in representative images of surface GluR1 expression from E18 + 16 DIV hippocampal neurons transfected at 10 DIV with Ube3A shRNA, Arc shRNA, Ube3A shRNA + Arc shRNA or Ube3A shRNA + Arc scRNA (data not shown). These findings suggest that Ube3A promotes the expression of AMPARs at the plasma membrane of synapses by ubiquitinating and degrading Arc and that in the absence of Ube3A there is an excess of Arc protein, resulting in increased endocytosis of AMPARs.

Analysis of AMPAR function was explored in *Ube3A* knockout mice. These findings suggest that in AS the absence of Ube3A activity may lead to an increase in Arc expression, thereby resulting in a reduction in the expression of AMPARs at synapses. AMPAR expression and function at the synapses of *Ube3A* knockout mice which display features of AS (Jiang et al., 1998) were examined. Neurons from *Ube3A* knockout or wild type mice were cultured and assessed the expression of AMPARs. Ube3A knockout neurons had reduced GluRl expression at the plasma membrane of synapses when compared to wild type neurons (Figure 6A). This effect appears to be specific to AMPARs as there was no change in the surface expression of NMDARs (Figure 6B). Expression of shRNAs targeting *Arc* in *Ube3A* knockout neurons restores the expression of GluR1 surface expression in *Ube3A* knockout neurons (Figure 6C). These experiments suggest that the excessive internalization of AMPARs in Ube3A knockout neurons is likely a result of a failure to ubiquitinate and degrade Arc.

GluR1 expression at synapses is dysregulated in Ube3A knockout neurons in the context of an intact neuronal circuit was explored using array tomography, a technique in which ultra-thin sections of brain tissue are stained, imaged, and synapses visualized as a 3-D reconstruction (Micheva & Smith, 55 Neuron 25-36 (2007)). Array tomography using anti-GluR1 antibodies allowed visualization of AMPARs and anti-SV2 antibodies to mark presynaptic sites. The density of GluR1 puncta closely apposed to an SV2 puncta is decreased in Ube3a knockout mice (Figures 6D). Tomography images obtained from hippocampal sections of P21 Ube3A knockout stained with anti-GluR1 and anti-Sv2 antibodies or anti-NR1 and anti-SV2 antibodies (Data not shown). From the images it can be seen that some GluR1 puncta are in close apposition to SV2 puncta and other GluR1 puncta are not proximal to SV2 puncta. The percentage of GluR1 puncta associated with SV2 is significantly higher in wild type hippocampi compared to Ube3A knockout hippocampi. Note that SV2 is a synaptic vesicle associated protein and as synaptic vesicles are often fairly distant from post-synaptic components, there are a number of SV2 puncta that are not associated with any post-synaptic markers (data not shown). The density of SV2 puncta remained constant between the two genotypes, suggesting that the decrease in GluR1 synaptic localization in Ube3A knockout sections is not a result of fewer available presynaptic sites and instead reflects a decrease in GluR1 expression at synapses. In contrast, the number of NR1 puncta associated with SV2 puncta was similar at the synapses in the hippocampi of wild type and Ube3A knockout mice, suggesting that the expression of AMPARs is selectively decreased in the brains of Ube3A knockout mice (Figures 6E, 6F, image data not shown). This reduction in AMPAR expression at the synapses of Ube3A knockout mice is not a result of decreased overall expression of GluR1 as wild type and Ube3A knockout mice express similar levels of GluR1 and NR1 in their hippocampi (Figure 6G).

To determine if the decreased expression of AMPARs at the synapses of *Ube3A* knockout mice results in a functional decrease in synaptic transmission, whole-cell recordings were made from CA1 hippocampal pyramidal neurons. There was a significant decrease in the ratio of AMPA to NMDA receptor-mediated currents in *Ube3A* knockouts compared to wild type mice (Figure 7A and 7B). Although this decrease in AMPA/NMDA receptor ratio could reflect either a decrease in AMPAR or an increase in NMDAR currents, the findings that in *Ube3A* knockout mice there is a decrease in AMPAR expression at synapses but no change in NMDAR expression suggests that the decrease in AMPA/NMDA current ratio is most likely due to a decrease in AMPAR-mediated currents in Ube3A knockout mice.

As an independent means of assessing the effect of disrupting Ube3A on AMPAR function, mEPSCs from wildtype and *Ube3A* knockout hippocampal pyramidal neurons were recorded in acute slice preparations. There was a reduction in the frequency of mEPSCs, with no corresponding change in mIPSC frequency or amplitude in *Ube3A* knockout neurons, compared with wild type neurons (Figures 7C to 7E and 12). This observation supports the conclusion that AMPAR expression and function at synapses are significantly decreased in Ube3A knockout neurons.

Although it has been appreciated for more than a decade that mutation of *Ube3A* results in AS, remarkably little is understood about the role of Ube3A in nervous system development and function or why mutation of *Ube3A* results in the cognitive impairment underlying AS. This lack of insight has hampered the development of therapeutic strategies for treating AS and as a result there are currently no effective treatments for this disorder. The present invention demonstrates that in the absence of synaptic activation, Ube3A and Arc are expressed at low levels. In response to glutamate release at excitatory synapses, however, Arc is induced with relatively rapid kinetics (Flavell et al., 2006) and endoctyoses AMPAR from the plasma membrane. This induction of Arc is likely important for limiting the level of neuronal excitation since Arc-mediated endocytosis of AMPARs dampens neuronal excitability. The level of Arc expression must be effectively regulated, however, for synapses to function appropriately. Ube3A transcription is induced post-synaptically upon glutamate release at synapses with delayed kinetics relative to Arc, and Ube3A then functions to control the level of Arc protein expression by ubiquitinating and degrading Arc. In this way Ube3A tempers the Arc-mediated internalization of AMPARs. The absence of Ube3A activity in *Ube3A* knockout mice results in increased levels of Arc, and excessive internalization of AMPARs, leading to fewer synapses that express AMPARs at the plasma membrane and to defects in synaptic transmission.

Consistent with these observations, a recent study has demonstrated that Ube3A plays a role in experience-dependent synaptic plasticity (Yashiro et al., Nat. Neurosci. (2009)). Although Ube3A is not required for the initial sensory-independent stages of synapse development, Ube3A is necessary for sensory experience-driven maturation of excitatory circuits as *Ube3A* knockout mice have deficits in LTP, LTD, and decreased mEPSCs in visual cortex. The observation that Ube3A plays a role in experience-driven synaptic plasticity may be explained by the finding that both *Arc* and *Ube3A* transcription are induced by sensory experience, and that in response to neuronal activity in the absence of Ube3A there is excessive accumulation of Arc and increased internalization of AMPARs. As AMPARs play a central role in neurotransmission and information processing, this defect in AMPAR expression and function in the absence of Ube3A is likely to explain, at least in part, the deficits in synaptic plasticity observed in the absence of Ube3A.

The present work suggests that AS may be caused by the disruption of a crucial step in experience-dependent synaptic development, and provide evidence that the neuronal activity-regulated gene program plays a key role in human cognitive development. Further support for this hypothesis comes from the observation that mutation of another activity-regulated MEF2 target gene, Slc9A6, results in phenotypes that mimic AS (Gilfillan et al., 2008). Recent studies have shown that additional components of the activity-regulated gene program including *L-VSCC, RSK2, MeCP2, CBP, PDCH10,* and *DIA1* are mutated in human disorders, particularly epilepsy and ASDs *(see* Greer & Greenberg, 2008). These findings suggest that further investigation into the regulation and function of Ube3A, and the activity-dependent gene program in general, provide new insights into the mechanisms controlling human cognitive development, and how mutations that disrupt this process lead to developmental disabilities, including ASDs.

The finding that disruption of Ube3A activity leads to a decrease in AMPAR expression at synapses indicates that drugs that promote AMPAR expression at synapses should reverse symptoms associated with AS. Studies of another human disorder Fragile X syndrome (FXS) where a decrease in AMPAR expression at synapses has been observed suggest that this type of therapeutic strategy has potential. In FXS, the decrease in AMPAR expression at synapses is due to excessive mGluR5 signaling resulting in increased Arc translation and excessive AMPAR internalization (Dolen & Bear, 586 J. Physiol. 1503-08 (2008)). In a mouse model of FXS injection of the mGluR5 antagonist MPEP restored surface expression of AMPARs and prevented the symptoms associated with FXS (Dolen et al., 56 Neuron 955-62 (2007); Nakamoto et al., 104 P.N.A.S. 15537-42 (2007); Yan et al., 49 Neuropharmacology 1053-66 (2005)). These results have led to the development of more specific mGluR5 antagonists that are now entering clinical trials for the treatment of FXS.Tus, GLuR5 antagonists are compositions that can be used in methods of the invention for treating AS.

A recent study demonstrated that the mutation of an inhibitory phosphorylation site of alphaCaMKII rescues many behavioral deficits exhibited by Ube3A-deficient mice suggesting that subtle genetic manipulations can reverse Ube3A loss-of function phenotypes (van Woerden et al., 10 Nat. Neurosci. 280-82 (2007)). An intriguing aspect of this finding is that increasing CamKII activity results in increased AMPAR expression at synapses (Rose et al., 61 Neuron 351-58 (2009)), and this may explain why increased CaMKII activity rescues phenotypes associated with the loss of Ube3A.

Not to be bound by theory, it is likely that the defect in AMPAR expression at synapses is not the only thing that has gone awry in AS. For example, it is likely that Ube3A substrates in addition to Arc play roles in nervous system development. In addition, individuals with AS have sleep disturbances, hyperactivity, inappropriate laughter, and movement disorders. Given the broad phenotypic consequences of AS, it is likely that the disruption of the degradation of a number of Ube3A substrates contributes to AS. In the present works defines a Ube3A binding domain which has aided in the identification of new Ube3A substrates. One of these substrates is the RhoGEF ephexin5, which plays an important role in restricting the number of synapses formed by a neuron (Margolis et al., submitted). Sacsin is another Ube3A substrate which is mutated in Charlevoix-Saguenay spastic ataxia, and it is intriguing to speculate that in AS, the absence of Ube3A-mediated ubiquitination of Sacsin may contribute to the movement disorders associated with AS. In addition to ephexin5 and Sacsin, there are a number of other proteins which contain the UBD.

### AMPAR receptor

The α-amino-3-hydroxy-5-methyl-4-isoxazolepropionic acid receptor (AMPAR) is also known as AMPA receptor, or quisqualate receptor. AMPAR is a non-NMDA-type ionotropic transmembrane receptor for glutamate that mediates fast synaptic transmission in the central nervous system (CNS). AMPA receptors (AMPAR) are both glutamate receptors and cation channels that are integral to plasticity and synaptic transmission at many postsynaptic membranes (Honore T, Lauridsen J, Krogsgaard-Larsen P (1982) "The binding of [3H]AMPA, a structural analogue of glutamic acid, to rat brain membranes" Journal of Neurochemistry 38 (1): 173-178). AMPARs are found in many parts of the brain and are the most commonly found receptor in the nervous system. Native AMPA receptors (AMPAR) exist as heterotetramers consisting of combinations of four different protein subunits (GluR1-4) (for review see B. Bettler, C. Muller. AMPA and kainate receptors, Neuropharmacology 34 (1995) 123-139.). One gene (GRIA1-4) is encoded for each subunit (GluR1-4). Receptor subunit diversity is increased further as each subunit can undergo alternative splicing of a 38 amino acid sequence in the extracellular region just before the fourth membrane spanning domain M4. Such editing results in flip/flop receptor isoforms which differ in kinetic and pharmacological properties (Sommer B, Keinanen K, Verdoon T A, Wisden W, Burnashev N, Herb A, Kohler M, Takagi T, Sakmann B, Seeburg P H (1990) Science 249: 1580-1585). The term "AMPAR" as uses herein encompasses receptor isoforms.

As discussed above, AMPARs are composed of four types of subunits, designated as GluR1 (GRIA1), GluR2 (GRIA2), GluR3 (GRIA3), and GluR4, alternatively called GluRA-D2 (GRIA4), combine to form tetramers. Most AMPARs are heterotetrameric, consisting of symmetric 'dimer of dimers' of GluR2 and either GluR1, GluR3 or GluR4. AMPAR depolarization removes voltage dependent Mg²⁺ block of NMDA receptors which in turn leads to NMDA receptor activation, an integral stage in the induction of Long Term Potentiation (Bliss T V P, Collingridge G L (1993) Nature 361: 31-9). LTP is a physiological measure of increased synaptic strength following a repetitive stimulus or activity, such as occurs during learning.

Direct activation of glutamate receptors by agonists, in conditions where glutamate receptor function is reduced, increases the risk of excitotoxicity and additional neuronal damage. AMPAR positive allosteric modulators, alone, do not activate the receptor directly. However, when the ligand (L-glutamate or AMPA) is present AMPAR modulators increase receptor activity. Thus, AMPA receptor allosteric modulators enhance synaptic function when glutamate is released and is able to bind at post-synaptic receptor sites.

The glutamate receptor, ionotropic, AMPA 1 of Homo sapiens (NCBI Gene ID 2890), is a subunit of AMPAR and referred to herein as "GluR1" is also known as GLUH1, GLURA, GluA1, HBGR1, MGC133252, and GRIA1. Two isoforms of the protein exist. The mRNA for isoform 1 precursor is GI:167001418 (SEQ ID NO: 31) while the isoform 2 precursor is GI:167001483 (SEQ ID NO: 32).

The glutamate receptor, ionotropic, AMPA 2 of Homo sapiens (NCBI Gene ID 2891), is a subunit of AMPAR and referred to herein as "GluR2" is also known as GLURB, GluA2, HBGR2, GluR-K2 and GRIA2. Three isoforms of the protein exist. The mRNA for isoform 1 precursor is GI:134304849 (SEQ ID NO: 33), the isoform 2 precursor is GI:134304847 (SEQ ID NO: 34) and the isoform 3 precursor is GI: 134304850 (SEQ ID NO:35). The subunit encoded by this gene is subject to RNA editing (CAG->CGG; Q->R) within the second transmembrane domain, which is thought to render the channel impermeable to Ca(2+).

The glutamate receptor, ionotropic, AMPA 3 of Homo sapiens (NCBI Gene ID 2892), is a subunit of AMPAR and referred to herein as "GluR3" is also known as GLURC, GluA3, MRX94, GLUR-C, GluR-K3 and GRIA3. Two isoforms of the protein exist. The mRNA for isoform 1 precursor is GI:163659855 (SEQ ID NO:36) while the isoform 2 precursor is GI:163659857 (SEQ ID NO:37). The subunit encoded by this gene is subject to RNA editing (AGA->GGA; R->G).

The glutamate receptor, ionotropic, AMPA 4 of Homo sapiens (NCBI Gene ID 2893), is a subunit of AMPAR and referred to herein as "GluR4" is also known as GLURD, GluA4, GLUR4C and GRIA4. Four isoforms of the protein exist. The mRNA for isoform 1 precursor is GI:164419733 (SEQ ID NO: 38), the isoform 2 precursor is GI:164419735 (SEQ ID NO:39), the isoform 3 precursor is GI:116284389 (SEQ ID NO: 40) and the isoform 4 precursor is GI:164419738 (SEQ ID NO:41). The subunit encoded by this gene is subject to RNA editing (AGA->GGA; R->G).

### Compounds that increase transcription ofAMPAR or its expression at the synapse

Compounds that increase AMPAR activity may increase expression of AMPAR subunits or enhance the prevalence of AMPAR at the synapse, reduce desensitization, or reduce deactivation. Notably, positive AMPA receptor modulators, that potentiate AMPA-class glutamate receptor mediated currents, have been demonstrated to increase BDNF expression (i.e., gene transcription and protein synthesis) by hippocampal and neocortical neurons indicating that these drugs may be useful therapeutics for enhancing neurotrophin expression and, secondary to this, supporting neuronal viability and function (Lauterborn et al., 2000, J Neurosci 20:8-21; Legutko et al.,2001, Neuropharmacology 40:1019-27; Mackowiak et al.,2002, Neuropharmacology 43:1-10; Lauterborn etal., 2003, J Pharmacol Exp Ther 307, 297-305). The mechanism by which this occurs involves activation of L-type voltage sensitive calcium channels leading to increases in intracellular calcium. Increases in calcium, in turn, activate subcellular signaling to eventually increase BDNF gene transcription (Ghosh et al., 1994, Science 263:1618-23; Tao et al, 1998, Neuron 20:709-26; Lauterborn et al, 2000, JNeurosci 20:821).

Positive AMPAR modulators include, but are not limited to, diazoxide and cyclothiazide (CTZ), two benzothiadiazides used clinically as antihypertensives or diuretics (Yamada and Rotham, 1992, J Physiol (LOnd) 458:409-423; Yamada and Tang, 1993, J Neurosci 13:3904-3915), 1-(1,3-benzodioxol-5-ylcarbonyl)-piperidine (1-BCP) (Yamada Neuroscience Letters 1998 249:119-122), S18986 [(S)-2,3-Dihydro-[3,4]Cyclopentano-1,2,4-benzothiadiazine-1,1-dioxide)(Bourasset et al., Drug Metabolism and Disposition 2005 33:1137-43), 7-chloro-3-methyl-3,4-dihydro-2H-1,2,4-benzothiadiazine-S,S-dioxide (IDRA21)(Yamada et al., Neurobiology of Disease 1998 5:196-205) and 7-chloro-3-methyl-3-4-dihydro-2H-1,2,4 benzothiadiazine S,S, dioxide, as described in Zivkovic et al, 1995, J. Pharmacol. Exp. Therap, 272:300-309; Thompson et al, 1995, Proc. Nat. Acad. Sci. USA, 92:7667-7671.

Positive modulators of AMPAR activity include the class of drugs known as ampakines. AMPAKINES® slow AMPA-type glutamate receptor deactivation (channel closing, transmitter dissociation) and desensitization rates and thereby enhance fast excitatory synaptic currents *in vitro* and *in vivo* and AMPA receptor currents in excised patches (Arai et al., 1994, Brain Res 638:343-346; Staublietal, 1994, Proc Natl Acad Sci USA 91:777-781; Arai et al., 1996, J Pharmacol Exp Ther 278:627-638; Arai et al., 2000, Mol Pharmacol 58(4):802-813). The drugs do not have agonistics or antagonistic properties but rather modulate the receptor rate constants for transmitter binding, channel opening and desensitization (Arai et al., 1996, J Pharmacol Exp Ther 278:627-638). Additionally, this class of molecules is able to cross the blood-brain barrier (Staubli et al., 1994, Proc Natl Acad Sci USA 91:11158-11162), are orally active, (Lynch et al., 1997, Exp Neurol 145:89-92; Goff et al., 2001, J Clin Psychopharmacol 21:484-487) and repeated administration of AMPAKINES® produced lasting improvements in learned behaviors without causing evident side effects (Hampson et al, 1998, JNeurosci 18:2748-2763).

Without limitation, exemplary ampakines may include, CX516 which has been used by Cortex Pharmaceuticals in Phase II trials for the treatment of Fragile X and autism (US Government Clinical Trial ID:NCT00054730); cyclothiazide, CX614 (2H,3H,6aH-pyrrolidino[2",1"-3',2']1,3oxazino[6',5'-5,4]benzo[e]1,4-dioxan-10-one; LiD37 or BDP-37) (Arai et al, 1997, Soc Neurosci Abstr 23:313; Hennegrif et al, 1997, J Neurchem 68:2424-2434; Kessler et al, 1998, Brain Res 783:121-126), ORG26576, farampator, CX546 (GR87 or BDP-17) (Rogers et al, 1988, Neurobiol Aging 9:339-349; Hoist et al, 1998, Proc Natl Acad Sci USA 95:2597-2602), CX691, CX717, CX929, CX1739, LY451395, LY450108, DP75 (U.S. Pat. No. 6,030,968), aniracetam (7-chloro-3-methyl-3-4-dihydro-2H-1,2,4 benzothiadiazine S,S, dioxide)(Zivkovic et al., J Pharmacol Exp. Therap 1995 272:300-9; Thompson etal., Proc Nat Acad Sci 1995 92:7667-71), compounds taught in Ward et al British Jouranal of Pharmacology 2010 160:181-190 and the AMPAKINES described in WO 94/02475 (PCT/US93/06916); U.S. Pat. Nos. 5,650,409, 6,329,368, 6,030,968 5,747,492, 5,773,434, 5,852,008, 5,891,876, 6,030,968, 6,083,947, 6,166,008, 6,274,600, 6,329,368, US 2009/0192199 A1, and WO 98/12185. Also, stereoisomers thereof, or pharmaceutically acceptable salts or hydrates of the foregoing may be used. The compounds disclosed in the literature and patents cited above can be prepared by conventional methods known to those skilled in the art of synthetic organic chemistry.

In other examples, a positive AMPAR modulator may be chosen from compounds having pharmacophore structures including benzoxazines, benzoyl piperidines, benzoyl pyrrolidines, benzofurazans, benzothiadiazines and biarylpropylsulfonamides find use in the present methods. Such compounds and their synthesis are described for example, in U.S. Pat. Nos. 6,620,808; 6,329,368; 6,274, 600; 6,083,947; 6,030,968; 5,985,871; 5,962,447; 5,891,876; 5,852,008; 5,747,492; 5,736,543; 5,650,409 and U.S. Patent Publication No. 2002/0055508. Exemplified positive AMPAR modulators are taught, for example, in U.S. Pat. Nos. 5,736,543; 5,962,447; 5,985,871; and 6,313,115, and PCT publication WO 03/045315. Additional positive AMPAR modulator that find use in the present methods include, for example, N-2-(4-(4-cyanophenol)phenol)propyl-2-propanesulfonamide (LY404187) and (R)-4'-[1-fluoro-1-methyl-2-(propane-2-sulfonylamino)-ethyl]-biphenyl-4-carboxylic acid methylamide (LY503430) (Ryder, et al., J Pharmacol Exp Therapeut (2006) 319:293; LY392098 (Li, et al. Cell Mol Neurobiol (2003) 23:419); LY451646 (Bai, et al, Neuropharmacol (2003) 44:1013); LY395153 (Linden, et al, Neuropharmacol (2001) 40:1010). AMPA receptor potentiators include sulphonamide derivatives described, for example, in U.S. Pat. Nos. 7,135,487; 6,911,476; 6,900,353; 6,803,484; 6,713,516 and 6,703,425. Positive AMPAR modulators include monofluoroalkyl derivatives described, for example, in U.S. Pat. No. 7,034,045. Positive AMPAR modulators further include other excitatory amino acid receptor modulators described, for example, in U.S. Pat. Nos. 7,125, 871 and 7,081,481.

In other examples, AMPAR activity is positively modulated by increasing the level of AMPAR found at the synapse. Increasing the levels of AMPAR at the synapse can be accomplished by a number of methods which include, but are not limited to, increasing CaMK11 activity (Hayashi et al., Science 2000 287:2262-2268), proteasome inhibition, inhibition of the ubiquitination of PSD-95 (Colledge et al., Neuron 2003 40:595-607), and exogenous expression of AMPA subunits by means of a viral vector (Lissin et al., PNAS 1998 95:7097-7102; Sudo et al., Molecular Brain Research 1997 50:91-99; Okada et al., European Journal of Neuroscience 2001 13:1635-1643).

Compounds which act as AMPAR positive allosteric modulators (i.e. compounds that increase the activity of AMPAR) have been shown to increase ligand affinity for the receptor (Arai A, Guidotti A, Costa E, Lynch G (1996) Neuroreport. 7: 2211-5.); reduce receptor desensitization and reduce receptor deactivation (Arai A C, Kessler M, Rogers G, Lynch G (2000) 58: 802-813) and facilitate the induction of LTP both *in vitro* (Arai A, Guidotti A, Costa E, Lynch G (1996) 7: 2211-5.) and *in vivo* (Staubli U, Perez Y, Xu F, Rogers G, Ingvar M, Stone-Elander S, Lynch G (1994) Proc Natl Acad Sci 91: 11158-11162). Such compounds also enhance the learning and performance of various cognitive tasks in rodent (Zivkovic I, Thompson D M, Bertolino M, Uzunov D, DiBella M, Costa E, Guidotti A (1995) JPET 272: 300-309, Lebrun C, Pilliere E, Lestage P (2000) Eu J Pharmacol 401: 205-212), sub-human primate (Thompson D M, Guidotti A, DiBella M, Costa E (1995) Proc Natl Acad Sci 92: 7667-7671) and man (Ingvar M, Ambros-Ingerson J, Davis M, Granger R, Kessler M, Rogers G A, Schehr R S, Lynch G (1997) Exp Neurol 146: 553-559). Compounds that can potentiate AMPAR can also be found in U.S. Patent No. 7,741,351.

### Exemplary AMPAR activation assays

Positive modulators of AMPAR suitable for use in the methods described herein can be identified using routine, well-known methods which are described in the scientific and patent literature. They include *in vitro* and *in vivo* assays for the identification of additional positive AMPA receptor modulators by monitoring the effect of test agents as described in U.S. Publication No. 2009/0192199 A1 and U.S. Pat. Nos. 5,747,492, 5,773,434, 5,852,008, 5,891,876, 6,030,968, 6,083,947, 6,166,008, and 6,274,600.

An exemplary *in vitro* assay for potential positive AMPAR modulators is as follows. Cultured hippocampal slices are prepared from rat pups (9 d postnatal) essentially as described by Lauterborn et al. (Lauterborn et al., 2000, J Neurosci 20(1):8-21). Slices are explanted onto Millicel-CM biomembrane inserts (Millipore, Bedford, Mass.; 6 slices/membrane) in a 6-well culture cluster plate (Corning, Cambridge, Mass.) containing sterile media (1 ml/well) consisting of minimum essential media, 30 mM dextrose, 30 mM HEPES, 5 mM Na2HC03, 3 mM glutamine, 0.5 mM ascorbic acid, 2 mM CaCl2, 2.5 mM MgS04, 1 mg/l insulin and 20% horse serum (pH 7.2; all reagents from Sigma, St. Louis, Mo.) and maintained for 10-18 d in a humidified incubator at 37°C in 5% C02. Media is changed three times/week.

Cultured rat hippocampal slices are then treated with the prospective positive AMPAR receptor modulator and appropriate controls, e.g. in absence of test agent. Cultures are processed for the *in situ* hybridization localization of BDNF mRNA and examined by photomicroscopy for BDNF cRNA labeling. cRNA probes are transcribed in the presence of ³⁵S-labeled UTP (DuPont NEN, Boston, Mass.). The cRNA to BDNF exon V is generated from PvuII-digested recombinant plasmid pR1 112-8 (Isackson et al, 1991, Neuron 6:937-948), yielding a 540 base length probe with 384 bases complementary to BDNF exon V-containing mRNA (Timmusk et al, 1993, Neuron 10:475-489). *In situ* hybridization is performed essentially as described by Lauterborn et al. (Lauterborn et al, 2000, J Neurosci 20(1):8-21; Lauterborn et al, 1994, Mol Cell Neurosci 5:46-62). Briefly, for *in situ* hybridization analyses, treatments are terminated by slice fixation with 4% paraformaldehyde in 0.1 M phosphate buffer, pH 7.2 (PPB). Cultures are re-sectioned parallel to the broad explant surface, slide-mounted, and processed for the in situ hybridization localization of BDNF mRNA using the ³⁵S-labeled BDNF cRNA probe described above. Following hybridization, the tissue is processed for film (Kodak Biomax) autoradiography. For quantification of *in situ* hybridization, hybridization densities are measured from film autoradiograms, with labeling densities calibrated relative to film images of 14C-labeled standards (|xCi/g), using the AIS system (Imaging Research Inc.). Significance is determined using the two-way ANOVA followed by Student-Newman-Keuls (SNK) or Student's t tests for individual comparisons. BDNF protein levels are examined in such culture samples by homogenizing the tissue in RIPA (RadioImmunoprecipitation Assay) buffer containing 10mM Tris, pH7.2, 158mM NaCl, 1 mM EDTA, 0.1% SDS, 1% sodium deoxycholate, 1% triton-X, Complete Protease Inhibitor Cocktail (Roche Diagnostics; Indianapolis, Ind.), and Phosphatase Inhibitor Cocktails 1 and 2 (P2850 and 5726, Sigma). Samples are normalized for protein content using the Bio-Rad protein assay and analyzed by Western blot analysis. Following addition of reducing SDS-polyacrylamide gel electrophoresis sample buffer, protein samples are separated on 4-20% gradient gels, transferred to polyvinylidene difluoride membranes, and incubated with antibodies specific for BDNF (1:2000, Santa Cruz Biotechnology). Binding of anti-BDNF antibodies to BDNF can be detected by enhanced chemiluminescence and quantified using ImageQuant software (Molecular Dynamics, Sunnyvale, Calif). An increase in BDNF mRNA/protein as compared to a control in the absence of the test agent indicate a positive AMPAR modulator.

An *in vivo* assay for positive AMPAR modulators is as follows. Adult male rats are injected intraperitoneally twice per day, 6 h apart, for 4 days with the potential modulator and controls. Immediately after injections, animals, are placed, as groups, in an enriched environment consisting of a wedge-shaped box with partitions and platforms for exploration and social interaction. Eighteen hours after the last injection, animals are killed and hippocampal samples are collected and processed for BDNF ELISA. The BDNF immunoassay is performed essentially as described by Lauterborn et al. (Lauterborn et al, 2000, J Neurosci 20(1):8-21). Samples are collected into 100 (xl of cold lysis buffer (137 mM NaCl, 20 mM Tris, 10% glycerol, 1 mM PMSF, 10 (xg/ml aprotinin, 1 (xg/ml leupeptin, 0.5 mM Na vanadate, and 1% NP-40). Tissue is manually homogenized in lysis buffer, acidified to pH 2.5 with IN HCl, and incubated for 15 min on ice. The pH is neutralized to pH 8.0 with IN NaOH, and samples are frozen (-70° C.) until assayed. Total BDNF protein content for each sample is measured using the BDNF Emax Immunassay System (Promega, Madison, Wis.) according to kit instructions, with the absorbance at 450 nm determined using a plate reader.

A primary assay for testing the activity of a potential modulator is measurement of enlargement of the excitatory postsynaptic potential (EPSP) in *in vitro* brain slices, such as rat hippocampal brain slices. In this assay, slices of hippocampus from a mammal such as rat are prepared and maintained in an interface chamber using conventional methods. Field EPSPs are recorded in the stratum radiatum of region CAlb and elicited by single stimulation pulses delivered once per 20 seconds to a bipolar electrode positioned in the Schaffer-commissural projections (see Granger et al., 1993, Synapse 15:326-329; Staubli et al., 1994a, Proc. Natl. Acad. Sci. USA 91:777-781; Staubli et al, 1994b, Proc. Natl. Acad. Sci. USA 91:11158-11162; Arai et al, 1994, Brain Res 638:343346; Arai et al, 1996a, Neuroscience 75:573-585, and Arai et al, 1996, J Pharm Exp Ther 278:627-638). An increase in EPSP as compared to a control in the absence of a test agent is indicative of a positive modulator of AMPAR.

### Metabotropic glutamate receptor subtype 5 (mGluR5) antagonists

Suitably, the agent that increases expression/activity of AMPAR (e.g. increases the number of AMPARs at the synapses of neurons) for treatment of Angelman syndrome is an antagonist of Group 1 metabotropic glutamate receptors (e.g. metabotropic glutamate receptor subtype 5 (mGluR5) or metabotropic glutamate receptor 1 (mGLuR1).

The glutamate receptor, metabotropic 5 of Homo sapiens (NCBI Gene ID:2915), referred to herein as "mGluR5", is also known as mGlu5, GPRC1E, and GRM5. It belongs to Group I of the glutamate receptor family. Group I also includes Grm1 and both of these proteins activate phospholipase C. Multiple isoforms of mGluR5 exist; including those coded for by transcript variant a (GI:225903435; SEQ ID:42) and transcript variant b (GI:225903434; SEQ ID NO:43).

The antagonist may be, for example, a chemical antagonist, a pharmacokinetic antagonist, an antagonist by receptor block, a non-competitive antagonist, or a physiological antagonist. Antagonists may act the level of the ligand-receptor interactions, such as by competitively or non-competitively (e.g., allosterically) inhibiting ligand binding. For example, the antagonist may act downstream of the receptor, such as by inhibiting receptor interaction with a G protein or downstream events associated with G protein activation such as stimulation of phospholipase C, elevation in intracellular calcium, the production of or levels of cAMP or adenylcyclase, stimulation and/or modulation of ion channels (e.g., K⁺, Ca⁺⁺). The antagonists can alter, diminish, halt, inhibit or prevent the above-referenced cellular signaling events.

A "pharmacokinetic antagonist" effectively reduces the concentration of the active drug at its site of action, e.g., by increasing the rate of metabolic degradation of the active ligand. Antagonism by receptor-block involves two important mechanisms: 1) reversible competitive antagonism and 2) irreversible, or non-equilibrium, competitive antagonism. Reversible competitive antagonism occurs when the rate of dissociation of the antagonist molecule from the receptor is sufficiently high that, on addition of the ligand, the antagonist molecules binding the receptors are effectively replaced by the ligand. Irreversible or non-equilibrium competitive antagonism occurs when the antagonist dissociates very slowly or not at all from the receptor, with the result that no change in the antagonist occupancy takes place when the ligand is applied. Thus, the antagonism is insurmountable. As used herein, a "competitive antagonist" is a molecule which binds directly to the receptor or ligand in a manner that sterically interferes with the interaction of the ligand with the receptor.

Non-competitive antagonism describes a situation where the antagonist does not compete directly with ligand binding at the receptor, but instead blocks a point in the signal transduction pathway subsequent to receptor activation by the ligand. Physiological antagonism loosely describes the interaction of two substances whose opposing actions in the body tend to cancel each other out. An antagonist can also be a substance that diminishes or abolishes expression of functional mGluR. Thus, an antagonist can be, for example, a substance that diminishes or abolishes: 1) the expression of the gene encoding mGluR5, 2) the translation of mGluR5 RNA, 3) the post-translational modification of mGluR5 protein, or 4) the insertion of GluR5 into the cell membrane.

In one embodiment the mGluR antagonist is a mGluR5 antagonist. Antagonists of mGluR5 are known to those skilled in the art and in one embodiment is 2-methyl-6-(phenylethynyl)-pyridine (MPEP), or 3-[(2-methyl-1,3-thiazol-4-yl)ethynyl]pyridine (MTEP). Other the mGluR5 antagonist may be, but are not limited to, a 2-arylalkenyl-, 2-heteroarylalkenyl-, 2-arylalkynyl-, 2-heteroaryl-alkynyl-, 2-arylazo- and 2-heteroarylazo-pyridine as described in European Patent 1117403, 6-methyl-2-phenylazo-pyridin-3-ol (SIB-1757), 2-methyl-6-((E)-styryl)-pyridine (SIB-1893), M-MPEP, [3H]-M-MPEP, a MTEP derivative with a methyl or methyoxymethyl at the 5-pyridyl position, methyoxy-PEPy, derivatives of MTEP which are meta subsituted bipyridyl analogs, or para substituted bipyridyl analogs, diaryl acetylene derivativatives of MPEP or M-MPEP, aminopyridine derivatives of MPEP, imidazole acetylene derivatives including 2-(4-[2-(2-chloropyridin-4-yl)-ethynyl]-2-methyl-imidazol-1-yl)-6-trifluoromethyl-pyridine and 2-cyclopropyl-6-[2-methyl-4-(2-methyl-pyridin-4-yl-ethynyl)-imidazol-1-yl]-pyridine, [4-(1,3-benzoxazol-2-yl)2-chlorophenyl]-acetonitrile, 4-(1,3-benzoxazol-2-yl)2-methoxyphenyl]-acetonitrile,2-pyridyl derivatives of [4-(1,3-benzoxazol-2-yl)2-chlorophenyl]-acetonitrile, imidazo[1,2-a]pyridine, dipyridin-3-ylisoxazolo[4,5-c]pyridin-4(5H)-one, and other antagonists disclosed in Slassi et al., Current Topics in Med Chem 2005 5:897-911. Other mGluR5 antagonists include dipryridyl amides as disclosed in Bonnefous et al., Bioorg Med Chem Lett 2005 15:1197-1200 and heteroarylazoles as described in Roppe et al., J. Med. Chem. 2004, 47:4645-8. Also envisioned are pharmaceutically acceptable salts, analogues and derivatives of the foregoing.

Additional mGluR5 inhibitors may include, without limitation, LY293558, 2-methyl 6-[(1E)-2-phenylethynyl]-pyridine, 6-methyl-2(phenylazo)-3-pyridinol, (RS)-a-methyl-4carboxyphenylglycine (MCPG), 3S,4aR,6S,8aRS-6-((((1Htetrazole-5-yl) methyl)oxy)methyl)-1,2,3,4,4a,5,6,7,8,8adecahydroisoquinoline-3-carboxylic acid, 3S,4aR,6S,8aR-6((((1H-tetrazole-5-yl)methyl)oxy)methyl)-1,2,3,4,4a,5,6,7, 8,8a-decahydroisoquinoline-3-carboxylic acid, 3SR,4aRS, 6SR,8aRS-6-(((4-carboxy)phenyl)methyl)-1,2,3,4,4a,5,6,7, 8,8a-decahydroisoquinoline-3-carboxylic acid and 3 S,4aR, 6S,8aR-6-(((4-carboxy)-phenyl)methyl)-1,2,3,4,4a,5,6,7,8, 8a-decahydroisoquinoline-3-carboxylic acid, and their pharmaceutically acceptable salts, analogues and derivatives thereof (U.S. Patent No. 7,648,993).

Antagonists of mGluR5 are also described in WO 03/093236, WO 01/12627, WO 01/66113, WO 01/32632, WO 01/14390, WO 01/08705, WO 01/05963, WO 01/02367, WO 01/02342, WO 01/02340, WO 00/20001, WO 00/73283, WO 00/69816, WO 00/63166, WO 00/26199, WO 00/26198, EP-A-0807621, WO 99/54280, WO 99/44639, WO 99/26927, WO 99/08678, WO 99/02497, WO 98/45270, WO 98/34907, WO 97/48399, WO 97/48400, WO 97/48409, WO 98/53812, WO 96/15100, WO 95/25110, WO 98/06724, WO 96/15099 WO 97/05109, WO 97/05137, U.S. Pat. Nos. 6,218,385, 5,672,592, 5,795,877, 5,863,536, 5,880,112, 5,902,817, allowed U.S. application Ser. Nos. 08/825,997, 08/833,628, 08/842,360, and 08/899,319. For example, different classes of mGluR5 antagonists are described in WO 01/08705 (pp. 3-7), WO 99/44639 (pp. 3-11), and WO 98/34907 (pp. 3-20).

Another class of mGluRl antagonists, antisense oligonucleotides, is described in WO 01/05963. Antisense oligonucleotides to mGluR5 can be prepared by analogy and used to selectively antagonize mGluR5, as desired. Gene silencing of mGluR5 can be accomplished by a number of means further described herein, including but not limited to, RNAi, shRNA, miRNA, and siRNA.

Clinical trials utilizing mGluR5 to treat Fragile X Syndrome are underway and in this case a mGluR5 antagonist may be, but is not limited to, any of the following drugs. Neuropharm is using fenobam (NPL-2009)(Porter et al., J Pharmacol Exp Ther 2005 315:711-21) in trials and has completed Phase II trials (US Government Clinical Trial ID: NCT00637221). Novartis has completed one Phase II trial (US Government Clinical Trial ID:NCT00718341) of AFQ056 and is conducting another (US Government Clinical Trial ID: NCT01253629). Seaside Therapeutics, Inc. is using arbaclofen (STX209) in Phase II trials (US Government Clinical Trial ID:NCT01013480 and NCT00788073) and has Phase III trials scheduled (US Government Clinical Trial ID:NCT01282268). Additionally, they are conducting Phase I trials with STX107 (US Government Clinical Trial ID:NCT00965432). Hoffman-LaRoche is using RO4917523 in Phase II trials (US Government Clinical Trial ID:NCT01015430).

In one embodiment, the antagonist inhibits expression by at least 10%, at least 20%, at least 30%, at least 40%, at least 50%, at least 60%, at least 70%, at least 80%, at least 90%, at least 95%, or even at least 99%.

In one embodiment, antagonists are those that provide a reduction of activation by the ligand of at least 10%, at least 20%, at least 30%, at least 40%, at least 50%, at least 60%, at least 70%, at least 80%, at least 90%, at least 95%, or even at least 99% at a concentration of the antagonist, for example, of 1 µg/ml, 10 µg/ml, 100 µg/ml, 500 µg/ml, 1 mg/ml, 10 mg/ml, or 100 mg/ml.

The percentage antagonism represents the percentage decrease in activity of mGluR, e.g., mGluR5, in a comparison of assays in the presence and absence of the antagonist. Any combination of the above mentioned degrees of percentage antagonism and concentration of antagonist may be used to define an antagonist of the invention, with greater antagonism at lower concentrations being preferred.

An antagonist for use as described herein may be a relatively non-specific antagonist that is an antagonist of mGluRs in general. Preferably, however, an antagonist selectively antagonizes group I mGluRs. Even more preferably, an antagonist used in the invention is a selective antagonist of mGluR5. A selective antagonist of mGluR5 is one that antagonizes mGluR5, but antagonizes other mGluRs only weakly or substantially not at all, or at least antagonizes other mGluRs with an EC50 at least 10 or even 100 or 1000 times greater than the EC50 at which it antagonizes mGluR5. Most preferred antagonists are those which can selectively antagonize mGluR5 at low concentrations, for example, those that cause a level of antagonism of 50% or greater at a concentration of 100 µg/ml or less.

### Exemplary mGluR5 Antagonist Assays

Methods for identifying mGluR antagonists suitable for use in the treatment of Angelman Syndrome and ASD are well known to those of skill in the art. Such methods essentially comprise determining whether a test agent is an mGluR5 antagonist and determining whether an antagonist so identified can be used in the treatment.

One example of an assay for determining the activity of a test compound as an antagonist of mGluR5 comprises expressing mGluR5 in CHO cells which have been transformed with cDNAs encoding the mGluR5 receptor protein (Daggett et al., 1995, Neuropharmacology, 34, 871). The mGluR5 is then activated by the addition of quisqualate and/or glutamate and can be assessed by, for example the measurement of: (1) phosphoinositol hydrolysis (Litschig et al., 1999, Mol. Pharmacol. 55, 453); (ii) accumulation of [³H] cytidinephosphate-diacylglycerol (Cavanni et al., 1999, Neuropharmacology 38, A10); or fluorescent detection of calcium influx into cells Kawabata et al., 1996, Nature 383, 89-1; Nakahara et al., 1997, J. Neurochemistry 69, 1467). The assay may be carried out both in the presence and absence of a test product in order to determine whether the test compound can antagonize the activity of the test product. This assay is amenable to high throughput screening.

GluR5 receptor antagonists may also be identified by radio labelled ligand binding studies at the cloned and expressed human GluR5 receptor (Korczak et al., 1994, Recept. Channels 3; 41-49), by whole cell voltage clamp electro-physiological recordings of functional activity at the human GluR5 receptor (Korczak et al., 1994, Recept. Channels 3; 41-49) and by whole cell voltage clamp electro-physiological recordings of currents in acutely isolated rat dorsal root ganglion neurons (Bleakman et al., 1996, Mol. Pharmacol. 49; 581-585).

Suitable control experiments can be carried out. For example, a putative antagonist of mGluR5 could be tested with mGluR1 in order to determine the specificity of the putative antagonist, or other receptors unrelated to mGluRs to discount the possibility that it is a general antagonist of cell membrane receptors.

Suitable test products for identifying an mGluR5 antagonist include combinatorial libraries, defined chemical identities, peptides and peptide mimetics, oligonucleotides and natural product libraries. The test products may be used in an initial screen of, for example, ten products per reaction, and the products of batches that show antagonism tested individually. Furthermore, antibody products (for example, monoclonal and polyclonal antibodies, single chain antibodies, chimeric bodies and CDR-grafted antibodies) may be used, as well as nucleic acid agents, such as RNAi.

### Arc

The Activity-Regulated Cytoskeleton Associated Protein (Arc, also known as Arg 3.1) is an immediate-early gene which promotes endocytosis of the AMPA sub-type of glutamate receptors, causing a downregulation in AMPAR activity. Arc is known to interact with F-actin, dynamin, endophilin and the results show herein indicates that it binds to and is targeting by, Ube3A.

Arc is quickly induced in the striatum by dopamine receptor agonists in a manner similar to c-fos, junB, DfosB, and NGFI-A. Unlike these transcription factors, Arc is a cytoskeletal protein with some homology to a-spectrin and is found in both the nucleus and the dendrites of neurons. Expression of Arc is induced by synaptic activity, behavioral learning, morphine, and cocaine. If stimulation is maintained at a high frequency, Arc will localize selectively to activated dendrites. Arc mRNA and protein induction during behavioral learning is so robust and reproducible that cellular imaging of Arc induction provides a powerful methodology to detect neural networks that underlie information processing and memory. Knockdowns of Arc show deficits in long-term synaptic potentiation, long-term memory consolidation, and spatial learning although short-term synaptic potentiation, task acquisition, and short-term memory is not perturbed.

At least three possible mRNAs have been identified for the Activity-regulated cytoskeleton-associated protein (ARC) (NCBI Gene ID 23237) gene. They are GI:6319151 (SEQ ID NO:44), GI:15147373 (SEQ ID NO:45), and GI:15744312 (SEQ ID NO:46)

### Antagonists of ARC

The expression of ARC is influenced by a number of factors and without wishing to be bound by theory, proper modulation of any of these inputs is envisioned as a means of decreasing the expression and therefore the activity of ARC for the purposes taught herein. ARC expression and protein levels can be increased by insulin in a p21^{ras}, mitogen-activated protein kinase/extracellular regulated kinase in a src tyrosine kinase dependent manner (Kremerskothen et al., Neuroscience Letters 2001 321:153-6). Thus, inhibitors of p21 ^{ras}can serve as an atagonist of Arc. Activation of muscarinic acetylcholine receptors (mAChR) also induce the expression of ARC while this effect can be inhibited by the nonselective muscarinic receptor antagonist atropine and M1/M3 subtype-specific antagonists (Teber et al., Molecular Brain Research 2004 121: 131-6). Thus, in one example, the antagonist is a non-selective muscarinc receptor antagonist. Furthermore, it has been observed that ARC mRNA present in synaptosomes is associated with polysomes (Bagni et al., Journal of Neuroscience 2000 20:RC76, 1-6). ARC expression is decreased in response to a high fat diet by decreasing NMDAR activity. ARC expression is specifically decreased in response to 27-hydroxycholesterol (Mateos et al., Brain Pathology 2009 19:69-80).

Expression of ARC is sensitive to 5-HT and related molecules, with variable patterns of induction and repression (Pei et al., Neuropharmacology 2000 39:463-470). Since induction of ARC expression can be accomplished by administering H89, a PKA antagonist (Bloomer et al., J Biol Chem.2008 283:582-592) or inhibiting MEK (Waltereit et al., J Neurosci. 2001 21:5484-5493.), in one example, the agent that inhibits Arc expression is a PKA agonist, or a MEK agonist. Furthermore, the transcription factors SRF, CREB, MEF2, and zif268 are known to promote ARC transcription (Kawashima et al., PNAS. 2009 106:316-321; Li et al., Mol and Cell Biol. 2005 25:10286-10300), thus, in one embodiment, the agent inhibits SRF, CREB, MEF2, and zif268.

Inhibition of ARC expression can also be accomplished via gene silencing techniques known to those skilled in the art and has been demonstrated using antisense oligodexoynucleotides (Guzowski et al., Journal of Neuroscience 2000 20:3993-4001; Messaoudi et al., Journal of Neuroscience 2007 27:10445-10455). In one embodiment the agent that inhibits Arc expression is an RNAi agent. Means for identifying suitable RNAi agents are known in the art, and are dexcribed herein under the heading "test agents".

### Assays for Identifying ARC Inhibitors

While the mechanism of action for ARC is not currently known, it is well established that it promotes the removal of AMPAR from the synapse, so inhibition of ARC is easily assayed by measuring an increase in surface expression of AMPAR. Without wishing to limit ourselves, one method of measuring surface AMPAR is as follows: Low-density hippocampal neurons are prepared as described previously (Banker and Cowan Brian Res 1977 126:397-342) or high-density cortical cultures from embryonic day 18 (E18) rat pups were prepared. To label surface GluRl-containing AMPAR, 2.5 µg of GluR1-N JH1816 pAb was added to neuronal growth media and incubated at 10°C for 20 min. The unbound excess antibody was quickly washed with fresh warmed growth medium and then fixed and mounted according to the methods described above. Cells are fixed in 4% paraformaldehyde, 4% sucrose containing PBS solution for 20 min at 4°C and are subsequently permeabilized with 0.2% Triton X-100 in PBS for 10 min. Cells were are blocked for 1 hr in 10% normal donkey/goat serum (NGS). Alexa488, Alexa555, or Alexa647-conjugated secondary antibodies (1:500; Molecular Probes, Eugene, OR) to the appropriate species is diluted in 10% NDS and incubated at room temperature for 1 hr. Coverslips are mounted on precleaned slides with PermaFluor and DABCO (Sheperd et al., Neuron 2006 52:475-484).

### Test Agents

As used herein, the terms "compound" or "agent" are used interchangeably and refer to molecules and/or compositions that modulate expression of a gene (e.g. AMPAR gene (Gene ID No:'s GI:167001418, GI:134304849, GI:163659855, GI:164419735); Arc gene (Gene ID NO. GI:23237); mGluR5 (GI:225903435)), or modulate the activity of a protein encoded by a gene identified herein (e.g. Arc, AMPAR, or mGluR5). The compounds/agents include, but are not limited to, chemical compounds and mixtures of chemical compounds, e.g., small organic or inorganic molecules; saccharines; oligosaccharides; polysaccharides; biological macromolecules, e.g., peptides, proteins, and peptide analogs and derivatives; peptidomimetics; nucleic acids; nucleic acid analogs and derivatives; extracts made from biological materials such as bacteria, plants, fungi, or animal cells or tissues; naturally occurring or synthetic compositions; peptides; aptamers; and antibodies, or fragments thereof.

A compound/agent can be a nucleic acid RNA or DNA, and can be either single or double stranded. Example nucleic acid compounds include, but are not limited to, a nucleic acid encoding a protein activator or inhibitor (e.g. transcriptional activators or inhibitors), oligonucleotides, nucleic acid analogues (e.g. peptide-nucleic acid (PNA), pseudo-complementary PNA (pc-PNA), locked nucleic acid (LNA) etc.), antisense molecules, ribozymes, small inhibitory or activating nucleic acid sequences (e.g. RNAi, shRNAi, siRNA, micro RNAi (mRNAi), antisense oligonucleotides etc.) A protein and/or peptide agent can be any protein that modulates gene expression or protein activity. Non-limiting examples include mutated proteins; therapeutic proteins and truncated proteins, e.g. wherein the protein is normally absent or expressed at lower levels in the target cell. Proteins can also be selected from genetically engineered proteins, peptides, synthetic peptides, recombinant proteins, chimeric proteins, antibodies, midibodies, minibodies, triabodies, humanized proteins, humanized antibodies, chimeric antibodies, modified proteins and fragments thereof. A compound or agent that increases expression of a gene or increases the activity of a protein encoded by a gene is also known as an activator or activating compound. A compound or agent that decreases expression of a gene or decreases the activity of a protein encoded by a gene is also known as an inhibitor or inhibiting compound.

The terms "polypeptide", "peptide" and "protein" refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acids.

As used herein, the terms "test compound" or "test agent" refer to a compound or agent and/or compositions thereof that are to be screened for their ability to inhibit or activate a gene identified herein (e.g. increase expression/activity of AMPAR, or inhibit expression/activity of mGLuR5, or inhibit expression/activity of Arc).

Various biochemical and molecular biology techniques or assays well known in the art can be employed in a screen. For example, techniques are described in, e.g., Handbook of Drug Screening, Seethala et al. (eds.), Marcel Dekker (1st ed., 2001); High Throughput Screening: Methods and Protocols (Methods in Molecular Biology, 190), Janzen (ed.), Humana Press (1st ed., 2002); Current Protocols in Immunology, Coligan et al. (Ed.), John Wiley & Sons Inc (2002); Sambrook et al., Molecular Cloning: A Laboratory Manual, Cold Spring Harbor Press (3rd ed., 2001); and Brent et al., Current Protocols in Molecular Biology, John Wiley & Sons, Inc. (ringbound ed., 2003). Screens involve the test agent, which is a candidate molecule which is to be used in a screen and/or applied in an assay for a desired activity (e.g., inhibition or activation of gene expression, or inhibition or activation of protein activity, etc.)

Test agents are first screened for there ability to modulate gene expression or protein activity and those test agents with modulatory effect are identified. Positive modulatory agents are then tested for efficacy with respect to increasing the activity of AMPAR, increasing expression of AMPR, inhibiting expression of mGluR5, inhibiting activity of mGluR5, inhibiting expression of Arc, or inhibiting the activity of Arc) using any known assay. Some exemplary assays are identified herein.

Generally, compounds can be tested at any concentration that can modulate expression or protein activity relative to a control over an appropriate time period. In some embodiments, compounds are tested at concentration in the range of about 0.1nM to about 1000mM. In one embodiment, the compound is tested in the range of about 0.1µM to about 20µM, about 0.1µM to about 10µM, or about 0.1µM to about 5µM. In one assay, compounds are tested at 1 µM.

Depending upon the particular embodiment being practiced, the test compounds can be provided free in solution, or may be attached to a carrier, or a solid support, e.g., beads. A number of suitable solid supports may be employed for immobilization of the test compounds. Examples of suitable solid supports include agarose, cellulose, dextran (commercially available as, i.e., Sephadex, Sepharose) carboxymethyl cellulose, polystyrene, polyethylene glycol (PEG), filter paper, nitrocellulose, ion exchange resins, plastic films, polyaminemethylvinylether maleic acid copolymer, glass beads, amino acid copolymer, ethylene-maleic acid copolymer, nylon, silk, etc. Additionally, for the methods described herein, test compounds may be screened individually, or in groups. Group screening is particularly useful where hit rates for effective test compounds are expected to be low such that one would not expect more than one positive result for a given group.

To screen test agents, *an in vitro* assay system and/or a cell-based assay system can be used. For example, test agents can be screened for binding to a gene or protein encoded by a gene, screened for altering the expression level of a gene, or screened for modulating activity/function of a protein encoded by a gene.

In one assay, protein/peptide test agents can be assessed for their ability to bind an encoded protein *in vitro.* Example direct binding assays include, but are not limited to, labeled *in vitro* protein-protein binding assays, electrophoretic mobility shift assays, immunoassays for protein binding, ELISA assays, co- immunoprecipitation assays, competition assays (e.g. with a known binder), and the like. See, e.g., U.S. Patents 4,366,241; 4,376,110; 4,517,288; and 4,837,168; and also Bevan et al., Trends in Biotechnology 13:115-122, 1995; Ecker et al., Bio/Technology 13:351-360, 1995; and Hodgson, Bio/Technology 10:973-980, 1992. The test agent can also be identified by detecting a signal that indicates that the agent binds to a protein of interest e.g., fluorescence quenching or FRET. Test agent polypeptides can also be monitored for their ability to bind nucleic acid in vitro, e.g. ELISA-format assays can be a convenient alternative to gel mobility shift assays (EMSA) for analysis of protein binding to nucleic acid.

Binding of a test agent to an encoded protein provides an indication the agent can be a modulator of protein activity. Test agents can also be screened for their ability inhibit or increase the activity/function of the protein, e.g. as described herein.

In one example, the test agent is assayed for the ability either upregulate or downregulate the biological activity or function of a protein encoded by a gene (i.e. upregulate AMPAR activity, or downregulate mGluR activity, or down regulate Arc activity). The assay used will be dependent on the function of the protein and can be readily determined by a skilled artisan.

In one example the test agent is assayed for the ability to inhibit or increase transcription of a gene. Transcriptional assay are well known to those of skill in the art (see e.g. United States Patent 7,319,933, 6,913,880). For example, modulation of expression of a gene can be examined in a cell-based system by transient or stable transfection of a reporter expression vector into cultured cell lines. Test compounds can be assayed for ability to inhibit or increase expression of a reporter gene (e.g., luciferase gene) under the control of a transcription regulatory element (e.g., promoter sequence) of a gene. An assay vector bearing the transcription regulatory element that is operably linked to the reporter gene can be transfected into any mammalian cell line for assays of promoter activity. Reporter genes typically encode polypeptides with an easily assayed enzymatic activity that is naturally absent from the host cell. Typical reporter polypeptides for eukaryotic promoters include, e.g., chloramphenicol acetyltransferase (CAT), firefly or Renilla luciferase, beta-galactosidase, beta-glucuronidase, alkaline phosphatase, and green fluorescent protein (GFP). Vectors expressing a reporter gene under the control of a transcription regulatory element of a gene can be prepared using routinely practiced techniques and methods of molecular biology (see, e.g., e.g., Samrbook et al., supra; Brent et al., supra).

In addition to a reporter gene, the vector can also comprise elements necessary for propagation or maintenance in the host cell, and elements such as polyadenylation sequences and transcriptional terminators. Exemplary assay vectors include pGL3 series of vectors (Promega, Madison, WI; U.S. Patent No. 5,670,356), which include a polylinker sequence 5' of a luciferase gene. General methods of cell culture, transfection, and reporter gene assay have been described in the art, e.g., Samrbook et al., supra; and Transfection Guide, Promega Corporation, Madison, WI (1998). Any readily transfectable mammalian cell line may be used to assay expression of the reporter gene from the vector, e.g., HCT116, HEK 293, MCF-7, and HepG2 cells.

Alternatively, modulation of mRNA levels can be assessed using, e.g., biochemical techniques such as Northern hybridization or other hybridization assays, nuclease protection assay, reverse transcription (quantitative RT-PCR) techniques and the like. Such assays are well known to those in the art. In one embodiment, nuclear "run-on" (or "run-off") transcription assays are used (see e.g. Methods in Molecular Biology, Volume: 49, Sep-27-1995, Page Range: 229-238). Arrays can also be used; arrays, and methods of analyzing mRNA using such arrays have been described previously, e.g. in EP0834575, EP0834576, WO96/31622, U.S. Pat. No. 5,837,832 or WO98/30883. WO97/10365 provides methods for monitoring of expression levels of a multiplicity of genes using high density oligonucleotide arrays.

In one example the test agent is assayed for the ability to inhibit or increase translation of a gene. Gene translation can be measured by quantitiation of protein expressed from a gene, for example by Western blotting, by an immunological detection of the protein, ELISA (enzyme-linked immunosorbent assay), Western blotting, radioimmunoassay (RIA) or other immunoassays and fluorescence-activated cell analysis (FACS) to detect protein.

In one embodiment, the modulating compound is an RNA interfering inhibitory or activating agent, for example a siRNA or a miRNA gene silencer or activator that decreases or increases respectively, the mRNA level of a gene identified herein. The modulating compound results in a decrease or increase, respectively, in the mRNA level in a cell for a target gene by at least about 5%, about 10%, about 20%, about 30%, about 40%, about 50%, about 60%, about 70%, about 80%, about 90%, about 95%, about 99%, about 100% of the mRNA level found in the cell without the presence of the miRNA or RNA interference molecule. In one embodiment, the mRNA levels are decreased or increased respectively by at least about 70%, about 80%, about 90%, about 95%, about 99%, about 100%.

As used herein, the term "RNAi" refers to any type of interfering RNA, including but are not limited to, siRNAi, shRNAi, endogenous microRNA and artificial microRNA; inhibitory or activating of gene expression.

As used herein an "siRNA" refers to a nucleic acid that forms a double stranded RNA, which double stranded RNA has the ability to reduce or inhibit expression of a gene or target gene when the siRNA is present or expressed in the same cell as the target gene, the genes identified in Tables 1-17. The double stranded RNA siRNA can be formed by the complementary strands. In one embodiment, an siRNA refers to a nucleic acid that can form a double stranded siRNA. The sequence of the siRNA can correspond to the full length target gene, or a subsequence thereof. Typically, the siRNA is at least about 15-50 nucleotides in length (e.g., each complementary sequence of the double stranded siRNA is about 15-50 nucleotides in length, and the double stranded siRNA is about 15-50 base pairs in length, preferably about 19-30 base nucleotides, preferably about 20-25 nucleotides in length, e.g., 20, 21, 22, 23, 24, 25, 26, 27, 28, 29, or 30 nucleotides in length). In one embodiment, the double stranded siRNA can contain a 3' and/or 5' overhang on each strand having a length of about 1, 2, 3, 4, or 5 nucleotides. In one embodiment, the siRNA is capable of promoting inhibitory RNA interference through degradation or specific post-transcriptional gene silencing (PTGS) of

The term "complementary" or "complementarity" as used herein refers to two nucleotide sequences which comprise antiparallel nucleotide sequences capable of pairing with one another (by the base-pairing rules) upon formation of hydrogen bonds between the complementary base residues in the antiparallel nucleotide sequences. For example, the sequence 5'-AGT-3' is complementary to the sequence 5'-ACT-3'. Complementarity can be "partial" or "total." "Partial" complementarity is where one or more nucleic acid bases are not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. A "complement" of a nucleic acid sequence as used herein refers to a nucleotide sequence whose nucleic acids show total complementarity to the nucleic acids of the nucleic acid sequence.

As used herein "shRNA" or "small hairpin RNA" (also called stem loop) is a type of siRNA. In one embodiment, these shRNAs are composed of a short, e.g. about 19 to about 25 nucleotide, antisense strand, followed by a nucleotide loop of about 5 to about 9 nucleotides, and the analogous sense strand. Alternatively, the sense strand can precede the nucleotide loop structure and the antisense strand can follow.

The terms "microRNA" or "miRNA" are used interchangeably herein are endogenous RNAs, some of which are known to regulate the expression of protein-coding genes at the posttranscriptional level. Endogenous microRNA are small RNAs naturally present in the genome which are capable of modulating the productive utilization of mRNA. The term artificial microRNA includes any type of RNA sequence, other than endogenous microRNA, which is capable of modulating the productive utilization of mRNA. MicroRNA sequences have been described in publications such as Lim, et al., Genes & Development, 17, p. 991-1008 (2003), Lim et al Science 299, 1540 (2003), Lee and Ambros Science, 294, 862 (2001), Lau et al., Science 294, 858-861 (2001), Lagos-Quintana et al, Current Biology, 12, 735-739 (2002), Lagos Quintana et al, Science 294, 853-857 (2001), and Lagos-Quintana et al, RNA, 9, 175-179 (2003). Multiple microRNAs can also be incorporated into a precursor molecule. Furthermore, miRNA-like stem-loops can be expressed in cells as a vehicle to deliver artificial miRNAs and short interfering RNAs (siRNAs) for the purpose of modulating the expression of endogenous genes through the miRNA and or RNAi pathways.

As used herein, "double stranded RNA" or "dsRNA" refers to RNA molecules that are comprised of two strands. Double-stranded molecules include those comprised of a single RNA molecule that doubles back on itself to form a two-stranded structure. For example, the stem loop structure of the progenitor molecules from which the single-stranded miRNA is derived, called the pre-miRNA (Bartel et al. 2004. Cell 116:281-297), comprises a dsRNA molecule.

Means for selecting nucleotide sequences (e.g. RNAi, siRNA, shRNA) that can serve as inhibitors or activators of target gene expression are well known and practiced by those of skill in the art. Many computer programs are available to design RNAi agents against a particular nucleic acid sequence. The targeted region of RNAi (e.g. siRNA etc.) can be selected from a given target gene sequence (e.g., a sequence of a target gene identified in Tables 1-17), beginning from about 25 to 50 nucleotides, from about 50 to 75 nucleotides, or from about 75 to 100 nucleotides downstream of the start codon. Nucleotide sequences can contain 5' or 3' UTRs and regions nearby the start codon. One method of designing a siRNA molecule of the present invention involves identifying the 23 nucleotide sequence motif AA(N19)TT (where N can be any nucleotide), and selecting hits with at least 25%, 30%, 35%, 40%, 45%, 50%, 55%, 60%, 65%, 70% or 75% G/C content. The "TT" portion of the sequence is optional. Alternatively, if no such sequence is found, the search can be extended using the motif NA(N21), where N can be any nucleotide. In this situation, the 3' end of the sense siRNA can be converted to TT to allow for the generation of a symmetric duplex with respect to the sequence composition of the sense and antisense 3' overhangs. The antisense RNAi molecule can then be synthesized as the complement to nucleotide positions 1 to 21 of the 23 nucleotide sequence motif. The use of symmetric 3' TT overhangs can be advantageous to ensure e.g. that the small interfering ribonucleoprotein particles (siRNPs) are formed with approximately equal ratios of sense and antisense target RNA-cleaving siRNPs (Elbashir et al. (2001) supra and Elbashir et al. 2001 supra).

In one embodiment, the RNAi agent targets at least 5 contiguous nucleotides in the identified target gene sequence. In one embodiment, the RNAi agent targets at least 6, 7, 8, 9 or 10 contiguous nucleotides in the identified target sequence. In one embodiment, the RNAi agent targets at least 11, 12, 13, 14, 15, 16, 17, 18 or 19 contiguous nucleotides in the identified target sequence.

In some embodiments, in order to increase nuclease resistance in an RNAi agent as disclosed herein, one can incorporate non-phosphodiester backbone linkages, as for example methylphosphonate, phosphorothioate or phosphorodithioate linkages or mixtures thereof, into one or more non-RNASE H-activating regions of the RNAi agents. Such non-activating regions may additionally include 2'-substituents and can also include chirally selected backbone linkages in order to increase binding affinity and duplex stability. Other functional groups may also be joined to the oligonucleoside sequence to instill a variety of desirable properties, such as to enhance uptake of the oligonucleoside sequence through cellular membranes, to enhance stability or to enhance the formation of hybrids with the target nucleic acid, or to promote cross-linking with the target (as with a psoralen photo-cross- linking substituent). See, for example, PCT Publication No. WO 92/02532.

Agents in the form of a protein and/or peptide or fragment thereof can also be designed to modulate a gene listed herein, i.e. modulate gene expression or encoded protein activity. Such agents are intended to encompass proteins which are normally absent as well as proteins normally endogenously expressed within a cell, e.g. expressed at low levels. Examples of useful proteins are mutated proteins, genetically engineered proteins, peptides, synthetic peptides, recombinant proteins, chimeric proteins, antibodies, intrabodies, midibodies, minibodies, triabodies, humanized proteins, humanized antibodies, chimeric antibodies, modified proteins and fragments thereof. Agents also include antibodies (polyclonal or monoclonal), neutralizing antibodies, antibody fragments, peptides, proteins, peptide-mimetics, or hormones, or variants thereof that function to inactivate the nucleic acid and/or protein of the genes identified herein. Modulation of gene expression or protein activity can be direct or indirect. In one example, a protein/peptide agent directly binds to a protein encoded by a gene identified herein, or directly binds to a nucleic acid of a gene identified herein.

The agent may function directly in the form in which it is administered. Alternatively, the agent can be modified or utilized intracellularly to produce something which modulates the gene, e.g. introduction of a nucleic acid sequence into the cell and its transcription resulting in the production of an inhibitor or activator of gene expression or protein activity.

The agent may comprise a vector. Many vectors useful for transferring exogenous genes into target mammalian cells are available, e.g. the vectors may be episomal, e.g., plasmids, virus derived vectors such cytomegalovirus, adenovirus, etc., or may be integrated into the target cell genome, through homologous recombination or random integration, e.g., retrovirus derived vectors such MMLV, HIV-1, ALV, etc. Many viral vectors are known in the art and can be used as carriers of a nucleic acid modulatory compound into the cell. For example, constructs containing the modulatory compound may be integrated and packaged into non-replicating, defective viral genomes like Adenovirus, Adeno-associated virus (AAV), or Herpes simplex virus (HSV) or others, including reteroviral and lentiviral vectors, for infection or transduction into cells. Alternatively, the construct may be incorporated into vectors capable of episomal replication, e.g EPV and EBV vectors. The nucleic acid incorporated into the vector can be operatively linked to an expression control sequence when the expression control sequence controls and regulates the transcription and translation of that polynucleotide sequence.

The term "operatively linked" includes having an appropriate start signal (e.g., ATG) in front of the polynucleotide sequence to be expressed, and maintaining the correct reading frame to permit expression of the polynucleotide sequence under the control of the expression control sequence, and production of the desired polypeptide encoded by the polynucleotide sequence. In some examples, transcription of a nucleic acid modulatory compound is under the control of a promoter sequence (or other transcriptional regulatory sequence) which controls the expression of the nucleic acid in a cell- type in which expression is intended. It will also be understood that the modulatory nucleic acid can be under the control of transcriptional regulatory sequences which are the same or which are different from those sequences which control transcription of the naturally-occurring form of a protein. In some instances the promoter sequence is recognized by the synthetic machinery of the cell, or introduced synthetic machinery, required for initiating transcription of a specific gene. The promoter sequence may be a "tissue-specific promoter," which means a nucleic acid sequence that serves as a promoter, i.e., regulates expression of a selected nucleic acid sequence operably linked to the promoter, and which affects expression of the selected nucleic acid sequence in specific cells, e.g. pancreatic beta-cells, muscle, liver, or fat cells. The term also covers so-called "leaky" promoters, which regulate expression of a selected nucleic acid primarily in one tissue, but cause expression in other tissues as well.

In some embodiments, the modulatory compound used in methods of the invention is a small molecule. As used herein, the term "small molecule" can refer to compounds that are "natural product-like," however, the term "small molecule" is not limited to "natural product-like" compounds. Rather, a small molecule is typically characterized in that it contains several carbon-carbon bonds, and has a molecular weight of less than 5000 Daltons (5 kD), preferably less than 3 kD, still more preferably less than 2 kD, and most preferably less than 1 kD. In some cases it is preferred that a small molecule have a molecular weight equal to or less than 700 Daltons.

Test agents can be small molecule compounds, e.g. methods for developing small molecule, polymeric and genome based libraries are described, for example, in Ding, et al. J Am. Chem. Soc. 124: 1594-1596 (2002) and Lynn, et al., J. Am. Chem. Soc. 123: 8155-8156 (2001). Commercially available compound libraries can be obtained from, e.g., ArQule, Pharmacopia, graffinity, Panvera, Vitas-M Lab, Biomol International and Oxford. These libraries can be screened using the screening devices and methods described herein. Chemical compound libraries such as those from NIH Roadmap, Molecular Libraries Screening Centers Network (MLSCN) can also be used. A comprehensive list of compound libraries can be found at www.broad.harvard.edu/chembio/platform/screening/compound_libraries/index.htm. A chemical library or compound library is a collection of stored chemicals usually used ultimately in high-throughput screening or industrial manufacture. The chemical library can consist in simple terms of a series of stored chemicals. Each chemical has associated information stored in some kind of database with information such as the chemical structure, purity, quantity, and physiochemical characteristics of the compound.

In one example, the test agents include peptide libraries, e.g. combinatorial libraries of peptides or other compounds can be fully randomized, with no sequence preferences or constants at any position. Alternatively, the library can be biased, i.e., some positions within the sequence are either held constant, or are selected from a limited number of possibilities. For example, in some cases, the nucleotides or amino acid residues are randomized within a defined class, for example, of hydrophobic amino acids, hydrophilic residues, sterically biased (either small or large) residues, towards the creation of cysteines, for cross-linking, prolines for SH-3 domains, serines, threonines, tyrosines or histidines for phosphorylation sites, or to purines.

The test agents can be naturally occurring proteins or their fragments. Such test agents can be obtained from a natural source, e.g., a cell or tissue lysate. Libraries of polypeptide agents can also be prepared, e.g., from a cDNA library commercially available or generated with routine methods. The test agents can also be peptides, e.g., peptides of from about 5 to about 30 amino acids, with from about 5 to about 20 amino acids being preferred, and from about 7 to about 15 being particularly preferred. The peptides can be digests of naturally occurring proteins, random peptides, or "biased" random peptides. In some methods, the test agents are polypeptides or proteins. The test agents can also be nucleic acids. Nucleic acid test agents can be naturally occurring nucleic acids, random nucleic acids, or "biased" random nucleic acids. For example, digests of prokaryotic or eukaryotic genomes can be similarly used as described above for proteins.

Libraries of test agents to be screened with the methods can also be generated based on structural studies of the proteins, or their fragments, encoded by the genes identified herein. Such structural studies allow the identification of test agents that are more likely to bind to the proteins and modulate their activity. The three-dimensional structures of the proteins can be studied in a number of ways, e.g., crystal structure and molecular modeling. Methods of studying protein structures using x- ray crystallography are well known in the literature. See Physical Bio-chemistry, Van Holde, K. E. (Prentice-Hall, New Jersey 1971), pp. 221-239, and Physical Chemistry with Applications to the Life Sciences, D. Eisenberg & D. C. Crothers (Benjamin Cummings, Menlo Park 1979). Computer modeling of structures provides another means for designing test agents to screen for modulators. Methods of molecular modeling have been described in the literature, e.g., U.S. Patent No. 5,612,894 entitled "System and method for molecular modeling utilizing a sensitivity factor," and U.S. Patent No. 5,583,973 entitled "Molecular modeling method and system". In addition, protein structures can also be determined by neutron diffraction and nuclear magnetic resonance (NMR). See, e.g., Physical Chemistry, 4th Ed. Mooτe, W. J. (Prentice-Hall, New Jersey 1972), and NMR of Proteins and Nucleic Acids, K. Wuthrich (Wiley-Interscience, New York 1986).

Modulating agents of the present invention also include antibodies that specifically bind to a protein encoded by a gene identified herein. Such antibodies can be monoclonal or polyclonal. The antibodies can be generated using methods well known in the art. For example, the production of non-human monoclonal antibodies, e.g., murine or rat, can be accomplished by, for example, immunizing the animal with a protein that is encoded by a gene identified herein, or its fragment (See Harlow and Lane, Antibodies, A Laboratory Manual, Cold Spring Harbor Press, 3rd ed., 2000). The immunogen can be obtained from a natural source, by peptides Humanized forms of mouse antibodies can be generated by linking the CDR regions of non-human antibodies to human constant regions by recombinant DNA techniques. See Queen et al., Proc. Natl. Acad. Sci. USA 86, 10029-10033 (1989) and WO 90/07861. Human antibodies can be obtained using phage-display methods. See, e.g., Dower et al., WO 91/17271; McCafferty et al., WO 92/01047. In these methods, libraries of phage are produced in which members display different antibodies on their outer surfaces. Antibodies are usually displayed as Fv or Fab fragments. Phage displaying antibodies with a desired specificity are selected by affinity enrichment to a protein.

Human antibodies against a protein can also be produced from non-human transgenic mammals having transgenes encoding at least a segment of the human immunoglobulin locus and an inactivated endogenous immunoglobulin locus. See, e.g., Lonberg et al., WO 93/12227 (1993); Kucherlapati, WO 91/10741 (1991). Human antibodies can be selected by competitive binding experiments, or otherwise, to have the same epitope specificity as a particular mouse antibody. Such antibodies are particularly likely to share the useful functional properties of the mouse antibodies. Human polyclonal antibodies can also be provided in the form of serum from humans immunized with an immunogenic agent. Optionally, such polyclonal antibodies can be concentrated by affinity purification using an encoded protein or its fragment.

In some embodiments, the test compound that is screened and identified to modulate expression of a gene identified herein, or identified to modulate the activity of a protein encoded by a gene identified herein can increase expression of AMPAR at the synapses of neurons by at least 5%, 10%, 20%, 30%, 40%, 50%, 50%, 70%, 80%, 90%, 1-fold, 1.1-fold, 1.5-fold, 2-fold, 3-fold, 4-fold, 5-fold, 10-fold, 50-fold, 100-fold or more higher relative to an untreated control.

### Autism Spectrum Disorder

As described herein, methods are also envisaged for the treatment of ASD Spectrum Disorders (ASDs), also known as Pervasive Developmental Disorders (PDDs), cause severe and pervasive impairment in thinking, feeling, language, and the ability to relate to others. These disorders are usually first diagnosed in early childhood and range from a severe form, called autistic disorder, through pervasive development disorder not otherwise specified (PDD-NOS), to a much milder form, Asperger syndrome. They also include two rare disorders, Rett syndrome and childhood disintegrative disorder. Prevalence studies have been done in several states and also in the United Kingdom, Europe, and Asia. A recent study of a U.S. metropolitan area estimated that 3.4 of every 1,000 children 3-10 years old had ASD.

All children with ASD demonstrate deficits in 1) social interaction, 2) verbal and nonverbal communication, and 3) repetitive behaviors or interests. In addition, they will often have unusual responses to sensory experiences, such as certain sounds or the way objects look. Anxiety and hyperactivity may also be apparent. Each of these symptoms run the gamut from mild to severe. They will present in each individual child differently. For instance, a child may have little trouble learning to read but exhibit extremely poor social interaction. Each child will display communication, social, and behavioral patterns that are individual but fit into the overall.

In social interactions and relationships, symptoms can include: significant problems developing nonverbal communication skills, such as eye-to-eye gazing, facial expressions, and body posture; failure to establish friendships with children the same age; lack of interest in sharing enjoyment, interests, or achievements with other people; lack of empathy. People with ASD can have difficulty understanding another person's feelings, such as pain or sorrow. Additionally, there is often an aversion to physical contact or signs of affection. In verbal and nonverbal communication, symptoms can include: delay in, or lack of, learning to talk. As many as 50% of people with ASD never speak and it is common for them to have problems taking steps to start a conversation. Also, people with ASD have difficulties continuing a conversation once it has begun. A repetitive use of language is can be present and patients will often repeat over and over a phrase they have heard previously (echolalia). Autistic individuals have difficulty understanding their listener's perspective. For example, a person with ASD may not understand that someone is using humor. They may interpret the communication word for word and fail to catch the implied meaning. People with ASD may show limited interest in activities or play and display an unusual focus on pieces. Younger children with ASD often focus on parts of toys, such as the wheels on a car, rather than playing with the entire toy or are preoccupied with certain topics. For example, older children and adults may be fascinated by train schedules, weather patterns, or license plates. A need for sameness and routines is often exhibited such as a need to always eat bread before salad or an insistance on driving the same route every day to school. People with ASD may also display typical behaviors such as body rocking and hand flapping.

Children with ASD do not follow the typical patterns of child development. In some children, hints of future problems may be apparent from birth. In most cases, the problems in communication and social skills become more noticeable as the child lags further behind other children the same age. Some other children start off well enough. Often times between 12 and 36 months old, the differences in the way they react to people and other unusual behaviors become apparent. Some parents report the change as being sudden, and that their children start to reject people, act strangely, and lose language and social skills they had previously acquired. In other cases, there is a plateau, or leveling, of progress so that the difference between the child with ASD and other children the same age becomes more noticeable.

ASD is defined by a certain set of behaviors that can range from the very mild to the severe. ASD has been associated with mental retardation (MR). It is said that between 75% and 90% of all autistics are mentally retarded. However, having ASD does not necessarily mean that one will have MR. ASD occurs at all IQ levels, from genius levels to the severely learning-disabled. Furthermore, there is a distinction between ASD and MR. People with MR generally show even skill development, whereas individuals with ASD typically show uneven skill development. Individuals with ASD may be very good at certain skills, such as music or mathematical calculation, yet perform poorly in other areas, especially social communication and social interaction.

Currently, there is no single test for ASD. In evaluating a child, clinicians rely on behavioral characteristics to make a diagnosis. Some of the characteristic behaviors of ASD can be apparent in the first few months of a child's life, or they can appear at any time during the early years. For the diagnosis, problems in at least one of the areas of communication, socialization, or restricted behavior must be present before the age of 3. The diagnosis requires a two-stage process. The first stage involves developmental screening during "well child" checkups; the second stage entails a comprehensive evaluation by a multidisciplinary team.

In one example, diagnosis is by the ASD Diagnostic Interview-Revised (ADI-R) (Lord C, et al., 1993, Infant Mental Health, 14: 234-52). In another example, diagnosis is by symptoms fitting an Autism Genetic Resource Exchange (AGRE) classification of ASD. Symptoms may be broad spectrum (patterns of impairment along the spectrum of pervasive developmental disorders, including PDD-NOS and Asperger's syndrome).

Several clinical methods of assessing the severity of ASD in totality as well as the severity of individual symptoms exist. These methods include, but are not limited to, the Austism Diagnostic Observation Schedule (ADOS), Childhood Autism Rating Scale (CARS), the Social Responsiveness Scale (SRS) and the ADI-R. The ADOS has recently been standardized specifically to allow for a severity metric (Gotham et al., Journal of Autism and Developmental Disorders 2009 39:693-705). Additionally, magnetoencephalography has been reported as a quantitative means of diagnosing ASD (Roberts et al., RSNA 2008; Roberts et al., International Journal of Psychophysiology 2008 68:149-60). Hand grip strength has also been correlated with CARS scores (Kern et al., Research in Autism Spectrum Disorders published online 2010). Repetitive behaviors can also be quantified by various means, including the Yale-Brown Obssessive Compulsive Scale (YBOCS)(US 2006/0105939 A1). The Autism Treatment Evaluation Checklist (ATEC) can also be used to quantify severity of impairments in speech, language, communication, sensory cognitive awareness, health, physical, and behavior, and social skills and demonstrate improvement in these metrics (US 2007/0254314 A1). Furthermore, correlations between expression of certain genes or biomarkers (including but not limited to neurexin-1β, NBEA, FHR1, apolipoprotein B, transferrin, TNF-alpha converting enzyme, dedicator of cytokinesis protein 1 (DOCK 180), fibronectin 1, complement C1q, complement component 3 precursor protein, and complement component 4B proprotein) and ASD has been reported (US 2009/0197253 A1; US 2006/0194201 A1; US 7,604,948).

### Angleman Syndrome

An aspect of the invention, provides agents for use in the treatment of Angelman syndrome (AS). Angelman syndrome is a neuro-genetic disorder characterized by intellectual and developmental delay, sleep disturbance, seizures, jerky movements (especially hand-flapping), frequent laughter or smiling, and usually a happy demeanor. AS is caused by mutation of the E3 ubiquitin ligase *Ube3A.* AS can be caused by mutation on the maternally inherited chromosome 15 while the paternal copy, which may be of normal sequence, is imprinted and therefore silenced. It is estimated that 1/10,000 to 1/20,000 children present with AS.

Symptoms of Angelman syndrome can include; developmental delays such as a lack of crawling or babbling at 6 to 12 months, mental retardation, no speech or minimal speech, ataxia (inability to move, walk, or balance properly), a puppet-like gait with jerky movements, hyperactivity, trembling in the arms and legs, frequent smiling and laughter, bouts of inappropriate laughter, widely spaced teeth, a happy, excitable personality, epilepsy, an electroencephalographic abnormality with slowing and notched wave and spikes, seizures which usually begin at 2 to 3 years of age, stiff or jerky movements, seizures accompanied by myoclonus and atypical absence, partial seizures with eye deviation and vomiting, a small head which is noticeably flat in the back (microbrachyoephaly), crossed eyes (strabismus), thrusting of the tongue and suck/swallowing disorders, protruding tongue, excessive chewing/mouthing behaviors, hyperactive lower extremity deep tendon reflexes, wide-based gait with pronated or valgus-positioned ankles, increased sensitivity to heat, walking with the arms up in the air, fascination with water or crinkly items such as some papers or plastics, obesity in older children, constipation, a jutting lower jaw, light pigmentation of the hair, skin, and eyes (hypopigmentation), frequent drooling, prognathia, feeding problems and/or truncal hypotonia during infancy, and scoliosis. Symptoms are usually not evident at birth and are often first evident as developmental delays such as a failure to crawl or babble between the ages of 6 to 12 months as well as slowing head growth before the age of 12 months. Individuals with Angleman syndrome may also suffer from sleep disturbances including difficulty initiating and maintaining sleep, prolonged sleep latency, prolonged wakefulness after sleep onset, high number of night awakenings and reduced total sleep time, enuresis, bruxism, sleep terrors, somnambulism, nocturnal hyperkinesia, and snoring.

Management of symptoms is known to those skilled in the art (Guerrini et al., Pediatric Druge 2003 5;647-661) and severity of symptoms has been measured clinically (Williams et al., American Journal of Medical Genetics 2005 140A; 413-8) and quantification of the severity of different symptoms is refined enough to allow segregation of patients based upon the particular genetic mechanism of their disease (Lossie et al., Journal of Medical Genetics 2001 38;834-845; Ohtsuka et al., Brain and Development 2005 27; 95-100) and may include the extent of language ability, degree of independent mobility, frequency and severity of seizures, ability to comprehend language, acquisition of motor skills, growth parameters. Lossie et al. have developed a screening procedure for suspected Angelman syndrome patients that quantifies the severity of 22 distinct criteria. Other measurements of symptom severity include psychometric methods to distinguish the degree of developmental delay with respect to pyschomotoer developmental achievement, visual skills, social interactions based on non-verbal events, expressive language abilities, receptive language abilities, and speech impairment. The degree of gait and movement disturbances has been measured as well as attention ability and the extent of EEG abnormalities (Williams et al., American Journal of Medical Genetics 2005 140A; 413-8).

Since there isn't a way to repair chromosome defects, there's no cure for Angelman syndrome. Thus, treatment has focused on managing the medical and developmental problems that the chromosome defects cause. Depending on the signs and symptoms, treatment for Angelman syndrome may involve the following: Anti-seizure medication to control seizures caused by Angelman syndrome; physical therapy to learn to walk better and overcome other movement problems with the help of physical therapy; and communication therapy to increase verbal skills; and behavior therapy to overcome hyperactivity and a short attention span, which can aid in developmental progress. Although the level of development people with Angelman syndrome can achieve varies widely, many are outgoing and are able to build relationships with friends and family. The methods of treatment described herein treat an underlying cause of Angleman Syndrome, thus significant improvement in the symptoms of Angleman Syndrome are expected.

### Treatment of Angelman Syndrome and ASDs

Agents are provided for use in methods for the treatment of Angelman syndrome comprising administering to a subject an agent that increases the expression of, or increases the activity of AMPAR. Treatments for ASDs are also envisaged.

By "treatment", "prevention" or "amelioration" of a disease or disorder is meant delaying or preventing the onset of such a disease or disorder, reversing, alleviating, ameliorating, inhibiting, slowing down or stopping the progression, aggravation or deterioration the progression or severity of a condition associated with such a disease or disorder. In one embodiment, the symptoms of a disease or disorder (e.g. AS or ASD) are alleviated by at least 5%, at least 10%, at least 20%, at least 30%, at least 40%, at least 50%, at least 60%, at least 70%, at least 80%, at least 90%.

In one embodiment, at least one symptom is alleviated by at least 5%, at least 10%, at least 20%, at least 30%, at least 40%, at least 50%, at least 60%, at least 70%, at least 80%, at least 90%. In one embodiment, at least two symptoms are alleviated by at least 5%, at least 10%, at least 20%, at least 30%, at least 40%, at least 50%, at least 60%, at least 70%, at least 80%, at least 90%.

In one embodiment, at least three symptoms are alleviated by at least 5%, at least 10%, at least 20%, at least 30%, at least 40%, at least 50%, at least 60%, at least 70%, at least 80%, at least 90%. In one embodiment, at least four or more symptoms are alleviated by at least 5%, at least 10%, at least 20%, at least 30%, at least 40%, at least 50%, at least 60%, at least 70%, at least 80%, at least 90%.

Treatment of Angelman Syndrome and ASD are determined by standard medical methods. In some embodiments, a goal of Angelman syndrome treatment is to reduce the frequency and severity of seizures, to reduce sleep disturbance, to reduce jerky movements, and/or to improve speech e.g. by at least 5%, at least 10%, at least 20%, at least 30%, at least 40%, at least 50%, at least 60%, at least 70%, at least 80%, at least 90%. Severity of symptoms can be measured by means well known to clinicians in the art, See, for example, the heading "Angelman Syndrome" herein.

In some embodiments, a goal of treatment of ASDs is to reduce repetitive behaviors, increase social interaction, reduce anxiety, reduce hyperactivity, increase empathy, and/or to improve speech e.g. by at least 5%, at least 10%, at least 20%, at least 30%, at least 40%, at least 50%, at least 60%, at least 70%, at least 80%, at least 90%. Severity of symptoms can be measured by means well known to clinicians, See, for example, the heading "Autism Spectrum Disorder" herein.

Delaying the onset of Angelman Syndrome or ASD in a subject refers to delay of onset of at least one symptom of the syndrome or disorder, or combinations thereof, for at least 1 week, at least 2 weeks, at least 1 month, at least 2 months, at least 6 months, at least 1 year, at least 2 years, at least 5 years, at least 10 years, at least 20 years, at least 30 years, at least 40 years or more, and can include the entire lifespan of the subject.

As used herein, a "subject" means a human or animal. Usually the animal is a vertebrate such as a primate, rodent, domestic animal or game animal. In certain embodiments, the subject is a mammal, e.g., a primate, e.g., a human. The terms, "patient" and "subject" are used interchangeably herein. The terms, "patient" and "subject" are used interchangeably herein.

Preferably, the subject is a mammal. The mammal can be a human, non-human primate, mouse, rat, dog, cat, horse, or cow, but are not limited to these examples. Mammals other than humans can be advantageously used as subjects that represent animal models of Angelman Syndrome. In addition, the methods described herein can be used to treat domesticated animals and/or pets. A subject can be male or female. A subject can be one who has been previously diagnosed with or identified as suffering from or having Angelman Syndrome. A subject can also be one who is not suffering from Angelman Syndrome, but is at risk of developing Angelman Syndrome.

In some embodiments, the therapeutic uses of the invention further comprise selecting a subject identified as being in need of treatment. As used herein, the phrase "subject in need of treatment" refers to a subject who is diagnosed with or identified as suffering from, having or at risk for developing, Angelman Syndrome. A subject in need can be identified using any method used for diagnosis of Angelman Syndrome, including for example genetic analysis.

### Pharmaceutical Compositions

For administration to a subject, the agents can be provided in pharmaceutically acceptable compositions. These pharmaceutically acceptable compositions comprise a therapeutically-effective amount of one or more of inhibitors or activators, formulated together with one or more pharmaceutically acceptable carriers (additives) and/or diluents. As described in detail below, the pharmaceutical compositions of the present invention can be specially formulated for administration in solid or liquid form, including those adapted for the following: (1) oral administration, for example, drenches (aqueous or non-aqueous solutions or suspensions), lozenges, dragees, capsules, pills, tablets (e.g., those targeted for buccal, sublingual, and systemic absorption), boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) transmucosally; or (9) nasally. Additionally, compounds can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

As used here, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used here, the term "pharmaceutically-acceptable carrier" means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C₂-C₁₂ alcohols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

The phrase "therapeutically-effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment. For example, an amount of a compound administered to a subject that is sufficient to produce a statistically significant, measurable change in at least one symptom of Angelman Syndrome or ASD, such as Determination of a therapeutically effective amount is well within the capability of those skilled in the art. Generally, a therapeutically effective amount can vary with the subject's history, age, condition, sex, as well as the severity and type of the medical condition in the subject, and administration of other pharmaceutically active agents. In one embodiment a therapeutically effective amount reduces at least one symptom of Angelman Syndrome by at least 5%, at least 10%, at least 20%, at least 30%, at least 40%, at least 50%, at least 60%, at least 70%, at least 80%, at least 90%.

In one embodiment, a therapeutically effective amount is the amount of a compound, material, or composition comprising a compound of the present invention which is effective for increasing the expression of AMPAR receptors in neurons relative to the expression of AMPAR receptors in the absence of the compound. The therapeutically effective dose can be estimated initially from a suitable cell culture assays, then a dose may be formulated in animal models to achieve a circulating plasma concentration range that includes the EC50 as determined in cell culture. Methods for assessing the levels of AMPAR receptors at the surface of synapses in neurons are known in the art, and suitable methods are described herein. One exemplary method includes an acid-strip immunocytochemical staining protocol.

As used herein, the term "administer" refers to the placement of a composition into a subject by a method or route which results in at least partial localization of the composition at a desired site such that desired effect is produced. A compound or composition described herein can be administered by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration.

Exemplary modes of administration include, but are not limited to, injection, infusion, instillation, inhalation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion.

Methods of delivering RNAi interfering (RNAi) agents (e.g., an siRNA), other nucleic acid modulators, or vectors containing modulatory nucleic acids, to the target cells (e.g., neuronal cells) can include, for example directly contacting the cell with a composition comprising a modulatory nucleic acid, or local or systemic injection of a composition containing the modulatory nucleic acid. In one embodiment, nucleic acid agents (e.g. RNAi, siRNA, or other nucleic acid) are injected directly into any blood vessel, such as vein, artery, venule or arteriole, via, e.g., hydrodynamic injection or catheterization. In some embodiments modulatory nucleic acids can be delivered locally to specific organs or delivered by systemic administration, wherein the nucleic acid is complexed with, or alternatively contained within a carrier. Example carriers for modulatory nucleic acid compounds include, but are not limited to, peptide carriers, viral vectors, gene therapy reagents, and/or liposome carrier complexes and the like.

The compound/agents described herein for treatment of Angelman syndrome can be administered to a subject in combination with another pharmaceutically active agent. Exemplary pharmaceutically active compound include, but are not limited to, those found in Harrison's Principles of Internal Medicine, 13th Edition, Eds. T.R. Harrison et al. McGraw-Hill N.Y., NY; Physicians Desk Reference, 50th Edition, 1997, Oradell New Jersey, Medical Economics Co.; Pharmacological Basis of Therapeutics, 8th Edition, Goodman and Gilman, 1990; United States Pharmacopeia, The National Formulary, USP XII NF XVII, 1990; current edition of Goodman and Oilman's The Pharmacological Basis of Therapeutics*;* and current edition of *The Merck Index*. In some embodiments, pharmaceutically active agent include those agents known in the art for treatment of seizures, for example, Tegretol or Carbatrol (carbamazepine), Zarontin (ethosuximide), Felbatol, Gabitril, Keppra, Lamictal, Lyrica , Neurontin (Gabapentin), Dilantin (Phenytoin), Topamax, Trileptal, Depakene, Depakote (valproate, valproic acid), Zonegran, Valium and similar tranquilizers such as Klonopin or Tranxene, etc.

The compounds and the additional pharmaceutically active agent (e.g. anti-seizure medication) can be administrated to the subject in the same pharmaceutical composition or in different pharmaceutical compositions (at the same time or at different times). When administrated at different times, compound of the invention and the pharmaceutically active agent can be administered within 5 minutes, 10 minutes, 20 minutes, 60 minutes, 2 hours, 3 hours, 4, hours, 8 hours, 12 hours, 24 hours of administration of the other. When the modulatory compound, and the pharmaceutically active agent are administered in different pharmaceutical compositions, routes of administration can be different. For example, an inhibitor (e.g. of ARC or mGluR5) or activator (e.g. of AMPAR) is administered by any appropriate route known in the art including, but not limited to oral or parenteral routes, including intravenous, intramuscular, subcutaneous, transdermal, airway (aerosol), pulmonary, nasal, rectal, and topical (including buccal and sublingual) administration, and pharmaceutically active agent is administration by a different route, e.g. a route commonly used in the art for administration of said pharmaceutically active agent.

The amount of compound which can be combined with a carrier material to produce a single dosage form will generally be that amount of the compound which produces a therapeutic effect. Generally out of one hundred percent, this amount will range from about 0.1% to 99% of compound, preferably from about 5% to about 70%, most preferably from 10% to about 30%.

Toxicity and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compositions that exhibit large therapeutic indices, are preferred.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized.

The therapeutically effective dose can be estimated initially from cell culture assays. A dose may be formulated in animal models to achieve a circulating plasma concentration range that includes the 1C50 (i.e., the concentration of the therapeutic which achieves a half-maximal inhibition of symptoms) as determined in cell culture. Levels in plasma may be measured, for example, by high performance liquid chromatography. The effects of any particular dosage can be monitored by a suitable bioassay.

The dosage may be determined by a physician and adjusted, as necessary, to suit observed effects of the treatment. Generally, the compositions are administered so that a modulatory agent/compound is given at a dose from 1 µg/kg to 150 mg/kg, 1 µg/kg to 100 mg/kg, 1 µg/kg to 50 mg/kg, 1 µg/kg to 20 mg/kg, 1 µg/kg to 10 mg/kg, 1 µg/kg to 1mg/kg, 100 µg/kg to 100 mg/kg, 100 µg/kg to 50 mg/kg, 100 µg/kg to 20 mg/kg, 100 µg/kg to 10 mg/kg, 100µg/kg to 1mg/kg, 1 mg/kg to 100 mg/kg, 1 mg/kg to 50 mg/kg, 1 mg/kg to 20 mg/kg, 1 mg/kg to 10 mg/kg, 10 mg/kg to 100 mg/kg, 10 mg/kg to 50 mg/kg, or 10 mg/kg to 20 mg/kg. It is to be understood that ranges given here include all intermediate ranges, for example, the range 1 mg/kg to 10 mg/kg includes 1mg/kg to 2 mg/kg, 1mg/kg to 3 mg/kg, 1mg/kg to 4 mg/kg, 1mg/kg to 5 mg/kg, 1mg/kg to 6 mg/kg, 1mg/kg to 7 mg/kg, 1mg/kg to 8 mg/kg, 1mg/kg to 9 mg/kg, 2mg/kg to 10mg/kg, 3mg/kg to 10mg/kg, 4mg/kg to 10mg/kg, 5mg/kg to 10mg/kg, 6mg/kg to 10mg/kg, 7mg/kg to 10mg/kg,8mg/kg to 10mg/kg, 9mg/kg to 10mg/kg etc. It is to be further understood that the ranges intermediate to the given above are also within the scope of this invention, for example, in the range 1mg/kg to 10 mg/kg, dose ranges such as 2mg/kg to 8 mg/kg, 3mg/kg to 7 mg/kg, 4mg/kg to 6mg/kg etc.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary from once a week to daily depending on a number of clinical factors, such as the subject's sensitivity to the polypeptides. The desired dose can be administered at one time or divided into subdoses, e.g., 2-4 subdoses and administered over a period of time, e.g., at appropriate intervals through the day or other appropriate schedule. Such sub-doses can be administered as unit dosage forms. In some embodiments, administration is chronic, e.g., one or more doses daily over a period of weeks or months. Examples of dosing schedules are administration daily, twice daily, three times daily or four or more times daily over a period of 1 week, 2 weeks, 3 weeks, 4 weeks, 1 month, 2 months, 3 months, 4 months, 5 months, or 6 months or more. The pharmaceutical compositions can be administered during infancy (between 0 to about 1 year of life), childhood (the period of life between infancy and puberty) and during puberty (between about 8 years of life to 18 years of life). The pharmaceutical compositions can also be administered to treat adults (greater than about 18 years of life).

### Definitions

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

Other than in the operating examples, or where otherwise indicated, all numbers expressing quantities of ingredients or reaction conditions used herein should be understood as modified in all instances by the term "about." The term "about" when used in connection with percentages may mean ±1%.

The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

The terms "decrease" , "reduced", "reduction" , "decrease" or "inhibit" are all used herein generally to mean a decrease by a statistically significant amount. However, for avoidance of doubt, ""reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10-100% as compared to a reference level.

The terms "increased" ,"increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "statistically significant" or "significantly" refers to statistical significance and generally means a two standard deviation (2SD) below normal, or lower, concentration of the marker. The term refers to statistical evidence that there is a difference. It is defined as the probability of making a decision to reject the null hypothesis when the null hypothesis is actually true. The decision is often made using the p-value.

As used herein, the term "IC50" refers to the concentration of an inhibitor that produces 50% of the maximal inhibition of activity or expression measurable using the same assay in the absence of the inhibitor. The IC50 can be as measured *in vitro* or *in vivo.* The IC50 can be determined by measuring activity using a conventional *in vitro* assay (e.g. protein activity assay, or gene expression assay).

As used herein, the term "EC50", refers to the concentration of an activator that produces 50% of maximal activation of measurable activity or expression using the same assay in the absence of the activator. Stated differently, the "EC50" is the concentration of activator that gives 50% activation, when 100% activation is set at the amount of activity that does not increase with the addition of more activator. The EC50 can be as measured *in vitro* or *in vivo.*

The term "modulates expression" refers to downmodulation (inhibition) or upregulation (increasing) of gene expression (e.g. inhibition of Arc gene expression, inhibition of mGluR5 gene expression, or activation of AMPAR gene expression). Expression of a gene can be modulated by affecting transcription, translation, or post-translational processing. In one embodiment, a compound that modulates expression of a gene, modulates transcription from the gene. In one embodiment, a compound that modulates expression of a gene modulates mRNA translation of mRNA transcribed from the gene. In one embodiment, a compound that modulates expression of a gene modulates post-translational modification of the protein encoded by the gene. To downmodulate expression is to inhibit expression by at least 5%, at least 10%, at least 20%, at least 30%, at least 40%, at least 50%, at least 60%, at least 70%, at least 80%, at least 90%, at least 95%, at least 98%, or 100% (e.g. complete loss of expression) relative to an uninhibited control. To upregulate expression is to increase expression by at least 5%, at least 10%, at least 20%, at least 30%, at least 40%, at least 50%, at least 60%, at least 70%, at least 80%, at least 90%, at least 95%, at least 98%, or 100% (e.g. complete loss of expression) relative to a control not treated with an upregulating compound. Gene expression can be measured, for example, by measuring the level of mRNA transcript or by measuring the level of protein or post translational modification, e.g. by Western analysis quantitated by densitometry or by mass spectrometry. Gene expression analysis can also be performed using reporter assays, for example by utilizing a vector or cell line comprising gene regulatory elements (e.g. promoter) operably linked to a measurable reporter gene, e.g. fluorescent reporter.

The term "modulates the activity", with respect to protein, refers to downregulation (inhibits activity) or upregulation (activates or increases activity) of protein activity or function (e.g. inhibit activity of Arc, inhibit activity of mGluR5, or increase activity of AMPAR). In one embodiment, the modulation occurs by directly inhibiting or increasing the activity of a protein, i.e. via direct physical interaction with the protein. In one embodiment, the activity of the protein is modulated indirectly, for example, in signaling, by inhibiting an upstream effector of the protein activity. In some embodiments of this and other aspects of the invention, activity of the protein encoded by the gene is inhibited or lowered by at least 5%, at least 10%, at least 20%, at least 30%, at least 40%, at least 50%, at least 60%, at least 70%, at least 80%, at least 90%, at least 95%, at least 98%, or 100% (e.g. complete loss of activity) relative to an uninhibited control. In some embodiments of this and other aspects of the invention, the inhibitor has an IC50 of less than or equal to 500nM, less than or equal to 250nM, less than or equal to 100nM, less than or equal to 50nM, less than or equal to 10nM, less than or equal to 1nM, less than or equal to 0.1nM, less than or equal to 0.01nM, or less than or equal to 0.001nM. In some embodiments of this and other aspects of the invention, activity of the protein is increased by at least 5%, at least 10%, at least 20%, at least 30%, at least 40%, at least 50%, at least 60%, at least 70%, at least 80%, at least 90%, at least 1-fold, at least 1.1-fold, at least 1.5-fold, at least 2-fold, at least 3-fold, at least 4-fold, at least 5-fold, or more relative to an un-activated control, e.g. in absence of activating agent. In some embodiments of this and other aspects of the invention, the activator of protein activity has an EC50 of less than or equal to 500nM, less than or equal to 250nM, less than or equal to 100nM, less than or equal to 50nM, less than or equal to 10nM, less than or equal to 1nM, less than or equal to O.1nM, less than or equal to 0.01nM, or less than or equal to 0.001nM.

All patents and other publications identified are cited herein for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

To the extent not already indicated, it will be understood by those of ordinary skill in the art that any one of the various embodiments herein described and illustrated may be further modified to incorporate features shown in any of the other embodiments disclosed herein. The terminology used herein is for the purpose of describing particular embodiments only, and the scope of protection is defined solely by the claims.

Further features of the concepts described herebefore can be defined in any of the following numbered paragraphs:
Paragraph 1: A method for treatment of Angelman Syndrome comprising administrating to a subject an agent that increases the expression of, or increases activity of, α-amino-3-hydroxy-5-methyl-4-isoxazolepropionic acid receptor (AMPAR) at neuronal synapses.
Paragraph 2: The method of paragraph 1, wherein the agent that increases the expression of, or activity of, the α-amino-3-hydroxy-5-methyl-4-isoxazolepropionic acid receptor (AMPAR) at neuronal synapses is an antagonist of metabotropic glutamate receptor subtype 5 (mGluR5).
Paragraph 3: The method of paragraph 2, wherein the antagonist is selected from the group consisting of: LY293558; 2-methyl 6-[(1E)-2-phenylethynyl]-pyridine; 6-methyl-2(phenylazo)-3-pyridinol; (RS)-a-methyl-4carboxyphenylglycine (MCPG); 3S,4aR,6S,8aRS-6-((((1Htetrazole-5-yl) methyl)oxy)methyl)-1,2,3,4,4a,5,6,7,8,8adecahydroisoquinoline-3-carboxylic acid; 3S,4aR,6S,8aR-6((((1H-tetrazole-5-yl)methyl)oxy)methyl)-1,2,3,4,4a,5,6,7, 8,8a-decahydroisoquinoline-3-carboxylic acid; 3SR,4aRS, 6SR,8aRS-6-(((4-carboxy)phenyl)methyl)-1,2,3,4,4a,5,6,7, 8,8a-decahydroisoquinoline-3-carboxylic acid; and 3 S,4aR, 6S,8aR-6-(((4-carboxy)-phenyl)methyl)-1,2,3,4,4a,5,6,7,8, 8a-decahydroisoquinoline-3-carboxylic acid.
Paragraph 4: The method of paragraph 2, wherein the antagonist comprises 2-methyl-6-(phenylethynyl)-pyridine (MPEP) or 3-[(2-methyl-1,3-thiazol-4-yl)ethynyl]pyridine (MTEP).
Paragraph 5: The method of claim 1, wherein the agent that increases the expression of, or activity of, the α-amino-3-hydroxy-5-methyl-4-isoxazolepropionic acid receptor (AMPAR) at neuronal synapses is selected from the group consisting of: diazoxide; cyclothiazide; 1-(1,3-benzodioxol-5-ylcarbonyl)-piperidine (1-BCP); S18986 [(S)-2,3-Dihydro-[3,4]Cyclopentano-1,2,4-benzothiadiazine-1,1 -dioxide); 7-chloro-3-methyl-3,4-dihydro-2H-1,2,4-benzothiadiazine-S,S-dioxide (IDRA21); 7-chloro-3-methyl-3-4-dihydro-2H-1,2,4 benzothiadiazine S,S, dioxide; and an ampikine.
Paragraph 6: The method of paragraph 1, wherein the agent inhibits the expression of, or inhibits the activity of, the synaptic protein activity-regulated cytoskeleton-associated protein (Arc).
Paragraph 7: The method of paragraph 6, wherein the agent is an RNA interfering agent (RNAi).
Paragraph 8: The method of paragraph 7, wherein the RNAi comprises SEQ ID NO: 9 or SEQ ID NO: 10.
Paragraph 9: The method of any of paragraphs 1-8, wherein the agent is selected from the group consisting of a small molecule, a nucleic acid, a protein, a peptide, an antibody, and an immunogenic fragment.
Paragraph 10: The method of any of paragraphs 1-9, wherein the agent is administered by a route selected from the group consisting of topical administration, enteral administration, and parenteral administration.
Paragraph 11: The method of any of paragraphs 1-10, wherein the subject is a human subject.
Paragraph 12: The method of any of paragraphs 1-11, wherein the agent is administered in a dose ranging from about .1 mg/kg to about 1000 mg/kg.
Paragraph 13: A method for treatment of an autism spectrum disorder comprising administrating to a subject an agent that increases the expression, or increases activity of, α-amino-3-hydroxy-5-methyl-4-isoxazolepropionic acid receptor (AMPAR) at neuronal synapses.
Paragraph 14: The method of paragraph 13, wherein the agent that increases the expression of, or activity of, the α-amino-3-hydroxy-5-methyl-4-isoxazolepropionic acid receptor (AMPAR) at neuronal synapses is an antagonist of metabotropic glutamate receptor subtype 5 (mGluR5).
Paragraph 15: The method of claim 14, wherein the antagonist is selected from the group consisting of: LY293558; 2-methyl 6-[(1E)-2-phenylethynyl]-pyridine; 6-methyl-2(phenylazo)-3-pyridinol, (RS)-α-methyl-4carboxyphenylglycine (MCPG); 3S,4aR,6S,8aRS-6-((((lHtetrazole-5-yl) methyl)oxy)methyl)-1,2,3,4,4a,5,6,7,8,8adecahydroisoquinoline-3-carboxylic acid; 3S,4aR,6S,8aR-6((((1H-tetrazole-5-yl)methyl)oxy)methyl)-1,2,3,4,4a,5,6,7, 8,8a-decahydroisoquinoline-3-carboxylic acid; 3SR,4aRS, 6SR,8aRS-6-(((4-carboxy)phenyl)methyl)-1,2,3,4,4a,5,6,7, 8,8a-decahydroisoquinoline-3-carboxylic acid; and 3 S,4aR, 6S,8aR-6-(((4-carboxy)-phenyl)methyl)-1,2,3,4,4a,5,6,7,8, 8a-decahydroisoquinoline-3-carboxylic acid.
Paragraph 16: The method of paragraph 14, wherein the antagonist comprises 2-methyl-6-(phenylethynyl)-pyridine (MPEP) or 3-[(2-methyl-1,3-thiazol-4-yl)ethynyl]pyridine (MTEP).
Paragraph 17: The method of claim 13, wherein the agent that increases the expression of, or activity of, the α-amino-3-hydroxy-5-methyl-4-isoxazolepropionic acid receptor (AMPAR) at neuronal synapses is selected from the group consisting of: diazoxide; cyclothiazide; 1-(1,3-benzodioxol-5-ylcarbonyl)-piperidine (1-BCP); S18986 [(S)-2,3-Dihydro-[3,4]Cyclopentano-1,2,4-benzothiadiazine-1,1-dioxide); 7-chloro-3-methyl-3,4-dihydro-2H-l,2,4-benzothiadiazine-S,S-dioxide (IDRA21); 7-chloro-3-methyl-3-4-dihydro-2H-1,2,4 benzothiadiazine S,S, dioxide; and an ampikine.
Paragraph 18: The method of paragraph 13, wherein the agent inhibits the expression of, or inhibits the activity of, the synaptic protein activity-regulated cytoskeleton-associated protein (Arc).
Paragraph 19: The method of paragraph 18, wherein the agent is an RNA interfering agent (RNAi).
Paragraph 20: The method of paragraph 19, wherein the RNAi comprises SEQ ID NO: 9 or SEQ ID NO: 10.
Paragraph 21: The method of any of paragraphs 13-20, wherein the agent is selected from the group consisting of a small molecule, a nucleic acid, a protein, a peptide, an antibody, and an immunogenic fragment.
Paragraph 22: The method of any of paragraphs 13-21, wherein the agent is administered by a route selected from the group consisting of topical administration, enteral administration, and parenteral administration.
Paragraph 23: The method of any of paragraphs 13-22, wherein the subject is a human subject.
Paragraph 24: The method of any of claims 13-23, wherein the agent is administered in a dose ranging from about .1 mg/kg to about 1000 mg/kg.
Paragraph 25: Use of an agent that increases the expression, or increases activity of, α-amino-3-hydroxy-5-methyl-4-isoxazolepropionic acid receptor (AMPAR) at neuronal synapses, for treatment of Angelman Syndrome or an autism spectrum disorder in a subject.
Paragraph 26: The use of paragraph 25, wherein the agent is selected from the group consisting of: an agent that is an antagonist of metabotropic glutamate receptor subtype 5 (mGluR5), an agent that inhibits the expression of, or inhibits the activity of, the synaptic protein activity-regulated cytoskeleton-associated protein (Arc); and a positive modulator of AMPAR.
Paragraph 27: The use of any of paragraphs 25-26, wherein the agent is selected from the group consisting of a small molecule, a nucleic acid, a protein, a peptide, an antibody, and an immunogenic fragment.
Paragraph 28: The use of any of paragraphs 25-27, wherein the agent is formulated for administration by a route selected from the group consisting of topical administration, enteral administration, and parenteral administration.
Paragraph 29: Use of any of paragraphs 25-28, wherein the subject is a human subject.
Paragraph 30: The use of any of paragraphs 25-29, wherein the agent is formulated for administration in a dose ranging from about .1 mg/kg to about 1000 mg/kg.

The following examples illustrate some embodiments and aspects of the invention. It will be apparent to those skilled in the relevant art that various modifications, additions, substitutions, and the like can be performed without altering the scope of the invention, and such modifications and variations may be encompassed within the scope of the invention as defined in the claims which follow. The following examples do not in any way limit the invention.

### EXAMPLES

### Example 1. Experimental procedures

HEK293T cells and hippocampal neurons were cultured, transfected, and infected as previously described (Flavell et al., 2006). Organotypic slice cultures were prepared from P3-6 rat or mouse brains and 350 µm slices of hippocampus were prepared and transfected as described previously (Zhou et al., 2006). Acute slices were prepared from P15-18 mice as described previously (Lin et al., 2008).

Images were acquired on a Zeiss LSM5 Pascal confocal microscope. For spine and synapse analysis, 12-bit images were acquired with a 63X objective at 1024x1024 pixel resolution. Images were acquired using z-stacks of 0.48 µm thickness. Maximum intensity projections were created from the z-stacks and analyzed using MetaMorph image analysis software (Molecular Devices). For each experiment image acquisition and image analysis were performed blinded to genotype and/or condition. Quantification of dendritic spine densities, lengths and widths were obtained manually using MetaMorph software. For all spine measurements at least 200 µm of dendrite was used for each neuron.

For Western blotting, whole rat or mouse brains or cultured cells were collected and homogenized in RIPA buffer (50 mM Tris pH 7.5-8.0, 150 mM NaCl, 1% TritonX-100, 0.5% Sodium Deoxycholate, 0.1% SDS, 5 mM EDTA, 10 mM NaF supplemented with complete protease inhibitor cocktail tablet (Roche)). Samples were boiled for 3-5 minutes in SDS sample buffer, resolved by SDS PAGE, transferred to nitrocellulose, and immunoblotted. Antibodies specific for Ube3A (Sigma), MEF2D (BD Biosciences), MEF2A (Santa Cruz Biotechnology), Arc (Santa Cruz Biotechnology), HA (Roche), and beta-actin (Abcam) are all commercially available. Antibodies for MeCP2 and phospho MeCP2 (Zhou et al., 2006) as well as Vav2 (Cowan et al., 46 Neuron 205-17 (2005)) were previously described. Immunostaining of surface GluR1 receptors was performed as previously described (Chowdhury et al., 2006).

Array tomography was performed as described (Micheva and Smith, 2007) with modifications. In summary, acute hippocampal slices (300 um thick) were fixed in 4% paraformaldyhde for 1 hour at room temperature and embedded in LR White resin using the benchtop protocol. Ribbons of between 30-50 serial 100 nm sections of both WT and Ube3a KO were mounted side by side on subbed glass coverslips. Coverslips were immunostained with anti-SV2 (ms, DSHB, 1:100) and anti-GluR1 (Rb, Millipore, AB1504) or anti-NR1 (Rb, Millipore AB9864, 1:100) antibodies as described. Serial sections were imaged using a Zeiss Imager.Z1 microscope with a Photometrics CoolSNAP HQ2 camera on a PLAN APO 63x/1.4 objective. Tissue volumes were aligned using ImageJ (NIH) with the multistackreg plugin (Brad Busse). Reconstructed tissue volumes were cropped to include only stratum lucidum of CA3 and analyzed in Bitplane Imaris and custom software to count synapses. A synapse was counted if the distance between the center point of an SV2 puncta and a GluR1/NR1 puncta was equal to or less than the sum of the radii of the two puncta plus an empirically determined scaling factor of .15 µm. All experiments were carried out and analyzed blinded to genotype.

pSuper plasmids targeting MEF2A and MEF2D were previously described (Flavell et al., 2006). Bacterial and mammalian expression plasmids of wild type Ube3A were generously provided by P. M. Howley (Kumar et al., 1999). QuikChange mutagenesis was used to generate Ube3A C833A. Bacterial and mammalian expression plasmids for Arc were previously described (Chowdhury et al., 2006). Arc and Ube3A shRNAs were generated using the pSuper RNAi system (OliogoEngine, Seattle, WA) and the following sequences:
Ube3A RNAi #1: 5'- TC**T**CC**A**C**A**G**T**CCTGA**AT**AT-3' (SEQ ID NO: 7),
Ube3A RNAi #2 5'- CCC**AAT**GA**T**G**T**ATG**A**TCT**A**-3' (SEQ ID NO: 8),
Arc RNAi #1 5'AC**C**C**A**A**T**GTGAT**C**CTGCAG-3' (SEQ ID NO: 9),
Arc RNAi #2 5'- GC**TG**A**T**GGC**TA**CG**A**C**TA**C**A**-3' (SEQ ID NO: 10) (mismatches listed in bold for scrambled constructs). The following sequences were used to generate RNAi-resistant forms:
   Ube3Ares #1: TCTGCATAGCCCGGAGTACCTG (SEQ ID NO: 11),
   Ube3ares#2: TCCGATGATGTACGACCTGAAG (SEQ ID NO: 12),
   Arcres#1: ACCGAACGTCATACTCCAA (SEQ ID NO: 13),
   Arc Res#2: GCGGACGGGTATGATTATA (SEQ ID NO: 14).

### Example 2. Ubiquitination Assay and in vitro binding

Two (2) µg of Arc C-terminal protein (132-396 a.a.) was incubated with 2 µg of GST-WT or mutant Ube3A (C833A) in binding buffer (20 mM Tris-HCL, pH 7.4, 50 mM NaCl, 4 mM ATP, 10 mM MgC12, 0.2 mM dithiothreitol and 1% Triton X-100). After 2 hr mixing at 4°C, glutathione-Sepharose beads (GE Healthcare) were added and incubated for another 2 hr. The beads were washed twice with PBS + 1% Triton X-100 and twice with PBS. Proteins were eluted with SDS sample buffer and analyzed by Western blotting. For *in vitro* ubiquitination assays, 1 µg of Arc C-terminal protein was incubated with 50 ng of E1, 100 ng of UbcH7, 200 ng each of WT or mutant (C833A) Ube3A, and 4 µg of ubiquitin (BostonBiochem) in 20 mM Tris-HCL, pH 7.4, 50 mM NaCl, 4 mM ATP, 10 mM MgCl₂, and 0.2 mM dithiothreitol. Reactions were terminated after 2 hr at 30°C by the addition of SDS sample buffer and were analyzed by Western blotting.

### Example 3. Ube3A knockout cultures

Hippocampal cultures were prepared from Ube3A knockout and wild type littermate mice at P2 using a protocol adopted from K. Condon and M. Ehlers. Briefly, hippocampi were dissected in Dissociation Media (DM) (0.3% BSA, 12 mM MgSO4, 10 mM HEPES, 0.6% glucose in Hanks Balanced Salt Solution). Hippocampi were then placed in a papain solution 30 Units/mL in DM for fifteen minutes before resuspending in Neurobasal Medium. The cells were then plated on glass coverslips which had been coated overnight with PDL.

### Example 4. Animal experiments

Animals were handled in accordance with Federal guidelines and protocols approved by Children's Hospital, Boston. Hippocampal slices were prepared from wild type or Ube3A knockout mice between postnatal days 15 and18 (P15-P18). Animals were deeply anesthetized by inhalation of isoflurane. The cerebral hemispheres were quickly removed and placed into ice cold choline-based artificial cerebrospinal fluid (choline ACSF) containing (in mM): 110 choline chloride, 25 NaHCO₃, 1.25 NaH₂PO₄ 2.5 KCl, 7 MgCl₂, 25 glucose, 1 CaCl₂, 11.6 ascorbic acid, and 3.1 pyruvic acid, and equilibrated with 95% O₂/5% CO₂ Tissue was blocked and transferred into a slicing chamber containing choline-ACSF. Transverse hippocampal slices (300 µm) were cut with a Leica VT1000s (Leica Instruments, Nussloch, Germany) and transferred into a holding chamber containing ACSF consisting of 127 mM NaCl, 2.5 mM KCl, 25 mM NaHCO3, 1.25 mM NaH₂PO₄, 2.0 mM CaCl₂, 1.0 mM MgCl₂, and 25 mM glucose and were equilibrated with 95% O₂/5% CO₂. Slices were incubated at 31°C for 30-45 min and then left at room temperature until recordings were performed.

For seizures and enriched environment, *Ube3A* knockout mice were obtained from The Jackson Laboratory, strain 129- Ube3atm1Alb/J from stock number 004477. HA-ubiquitin mice were previously described (Ryu et al., 2007). Seizures were induced for three hours in adult CD1 mice by intraperitoneal injection of kainic acid (Ocean Produce International) at a dose of 25 mg/Kg. For enriched environment experiments, 6 week old CD1 male mice were either placed in standard laboratory cages or in cages containing a variety of rodent toys of various shapes and colors (PETCO) for three hours.

### Example 5. Quantitative Real-Time PCR

Quantitative Real-Time PCR was carried out following standard procedures. Total RNA was harvested from hippocampal neurons at 10 DIV following stimulation with the indicated agent using the RNeasy mini kit (Qiagen). Stimulants included Bicuculline (Sigma, 20 µm), Glutamate (Sigma, 10 µm), NMDA (Sigma, 20 µm), recombinant human BDNF (Peprotech, 50 ng/mL), NT3 (Peprotech, 50 ng/mL), NT4 (Peprotech, 50 ng/mL), and 55 mM KCl as previously described (Chen et al., 2003). Reverse transcription was performed using SuperScript III (Qiagen), and quantitative RT-PCR using SYBR Green Master Mix was performed on an ABI Prism 7700 according to the manufacturer's instructions. The primers used for this study are listed below:
ArcF: 5'-ACCGTCCCCTCCTCTCTTGA-3' (SEQ ID NO: 15);
ArcR: 5'-TCTTTGTAATCCTATTTTCTCTGCCTT-3' (SEQ ID NO: 16)
Beta3-tubulinF: 5'-CCCGAGGGCTCAAGATGTC-3' (SEQ ID NO: 17)
Beta3-tubulinR: 5'-TCTTTGTAATCCTATTTTCTCTGCCTT-3' (SEQ ID NO: 18)
CremF: 5'-AAAGCGGGAGCTGAGGCT-3' (SEQ ID NO: 19)
CremR: 5'-TTCTTTCTTCTTCCTGCGACACT-3' (SEQ ID NO: 20)
GapdhF: 5'-TCCATGACAACTTTGGCATCGTGG-3' (SEQ ID NO: 21)
GaphdhR: 5'-GTTTCTGTTGAAGTCACAGGAGAC-3' (SEQ ID NO: 22)
Ube3aF: 5'-TCCTCTTTGGGTGACTCCAG-3' (SEQ ID NO: 23)
Ube3aR: 5'-CGGAAGAGAAGCGTAACGAG-3' (SEQ ID NO: 24)

### Example 6. Chromatin immunoprecipitation

Chromatin immunoprecipitation was performed using the ChIP assay kit (Upstate) as previously described (Flavell et al., 2006). The consensus binding site for MEF2 is C/TTAWWWWTAA/G. Primers used for these assays are listed below:
Ube3A promoter 1 F: 5'-GCTCTGGTGGGGAAGACATA -3' (SEQ ID NO: 25)
R: 5'-CCAGAAGCAGCACACGAATA-3' (SEQ ID NO: 26)
Ube3A promoter 2 F: 5'-AGAAACCTCATAGTGCTTGCAG-3' (SEQ ID NO: 27)
R: 5'-TTCTCAACTCTGGCCATCAA-3' (SEQ ID NO: 28)
Ube3A promoter 3 F: 5'-TCTGCCCTCTCTACGTCAGG-3' (SEQ ID NO: 29)
R: 5'-ATGAAACGAAACCCCACAAG-3' (SEQ ID NO: 30)

### Example 7. Quantification of Synapse Density

At 14-18 DIV, cultured hippocampal neurons were fixed in 2% formaldehyde/4% sucrose for 2 minutes at room temperature and then transferred to 100% methanol for 10 minutes at -20°C. Coverslips were washed three times with PBS and incubated 1 hr in GDB (0.1% gelatin, 0.3% TritonX-100, 4.2% 0.4 M phosphate buffer, 9% 5M NaCl). Primary antibodies were incubated for 1 hr in GDB at room temperature at the indicated concentrations: PSD-95 (mouse, 1:200; Affinity BioReagents), Synapsin I (rabbit, 1:200; Chemicon), Gad67 (mouse, 1:100; Chemicon), GABAA 2 (rabbit, 1:100, Chemicon). Coverslips were then washed three times with PBS for ten minutes each and then incubated with Cy3- and Cy5-conjugated secondary antibodies (1:300 each; Jackson ImmunoResearch Laboratories) in GDB for one hour at room temperature. Coverslips were then washed three times with PBS for ten minutes each, dipped briefly in water, and mounted on glass slides using Aquamount (Lerner Laboratories). Synapse density was quantified as the overlap of GFP, pre-synaptic marker and post-synaptic marker using Metamorph software and custom macros as previously described (Paradis et al., 2007).

### Example 8. Mass Spectrometry

The sample was separated by SDS-PAGE on a 4-12% NuPAGE gel (Novex/Invitrogen). The gel band was excised and in-gel digested using trypsin prior to mass spectrometric analysis. All LC/MS experiments were performed by using a LTQ-FT ICR mass spectrometer (Thermo Finnegan, San Jose, CA) coupled to a microscale capillary HPLC (Famos micro-autosampler (LC Packings, Sunnyvale, CA) driven by an Eksigent). Columns were packed in-house by using Magic C18 beads (5 µm particle size, 200 Å pore size; Michrom BioResources, Auburn, CA. Buffer A was 97.3% H2O/ 2.5% acetonitrile/0.2% formic acid; buffer B was 97.3% acetonitrile /2.5% water/0.2% formic acid; and the loading buffer was buffer A plus 5% formic acid). Data were searched against the mouse IPI database v3.09.fasta using the Paragon and Mascot Algorithms. Mass additions for modifications such as carbamidomethylated cysteine and ubiquitinated lysine were permitted to allow for the detection of these modifications. A confidence score of 99 was required for a peptide for the Paragon algorithm and for Mascot our cutoff score was 40. All modification sites were manually confirmed by interrogating the data.

### Example 9. Electrophysiology

Electrophysiology was performed using standard methods. Whole-cell recordings were obtained from CA1 pyramidal cells visualized under IR-DIC. mEPSC and mIPSC recordings were performed and analyzed as described previously (Lin et al., 2008). Recording pipettes were pulled from borosilicate glass capillary tubing with filaments to yield tips of 2.5-4.5 MΩ resistance. Spontaneous miniature inhibitory postsynaptic potentials (mIPSC) were recorded with pipettes filled with (in mM): 147 CsCl, 5 Na₂- phosphocreatine, 10 HEPES, 2 MgATP, 0.3 Na₂ GTP, and 1 EGTA. Spontaneous miniature excitatory synaptic potentials (mEPSC) and AMPA/NMDA current ratios were recorded with pipettes filled with (in mM): 120 Cesium Methanesulfonate, 10 HEPES, 4 MgCl₂, 4 Na₂ ATP, 0.4 Na₂ GTP, 10 Na₂-phosphocreatine, and 1 EGTA. Intracellular solutions were adjusted to pH 7.3 with CsOH and were 290-300 mOSM. Inhibitory events were pharmacologically isolated by bath application of tetrototoxin (0.5 µM, Tocris Bioscience, Ellisville, MO), (R)-CPP (10 µM, Tocris Bioscience, Ellisville, Missouri), and NBQX disodium salt (10 µM, Tocris Bioscience, Ellisville, Missouri), to antagonize voltage-gate sodium channels (VGSC), NMDA receptors, and AMPA receptors, respectively. Excitatory events were isolated with tetrodotoxin, and picrotoxin (50 µM, Tocris Bioscience, Ellisville, Missouri) to antagonize VGSC and GABAA receptors, respectively. Additionally, cyclothiazide (10 µM, Tocris Bioscience, Ellisville, Missouri) was added to the bath to reduce AMPAR desensitization and facilitate measurement and quantification of mEPSCs. AMPA/NMDA ratios were measured in the presence of picrotoxin. For mIPSC and mEPSC recordings, cells were held at -70 mV; AMPA/NMDA current ratios were measured holding the cell at -70 and +40 mV to assess AMPAR and NMDAR mediated currents, respectively. Data were acquired using Clampex10 software and an Axopatch 200B amplifier. Current traces were filtered at 5 kHz, digitized at 10 kHz, and acquired in 10 sec intervals. The cell capacitance, input resistance and series resistance were monitored with a 5mV hyperpolarizing step delivered at the beginning of each sweep. Cells were discarded if the series resistance was greater than 25 MΩ. Data were analyzed in IgorPro 5.05 using custom software modified from Shankar et al., 2007. For mIPSC and mEPSC analyses, the root mean square (RMS) was calculated for the first 150 ms of each trace and the event threshold set to be 1.5 times the RMS. Currents were counted as events if they had a rapid rise time (1.5 pA/ms), an exponential decay (2 < τ < 200 ms, 1 < τ □< 50 ms for mIPSC and mEPSC, respectively), and crossed the event threshold. Data are displayed as the cumulative distribution of all events recorded from a given genotype. Statistical significance was determined by randomly selecting 50 events from each cell, pooling events from cells of the same genotype and running a Kolmogorov-Smirnov test on the pooled data. p < 0.05 was considered statistically significant. Furthermore, data were randomly resampled and the analysis was repeated > 10 times. For each resampling, p > 0.05 for all parameters. For AMPA/NMDA current ratios, an extracellular stimulating electrode was placed in stratum radiatum, approximately 200-300 µm from the patched cell in the direction of CA3. Brief current pulses were delivered (0.2 ms) and the evoked response was measured while holding the cell at -70 and +40 mV. The peak current measured at -70 mV was used in the numerator to represent the AMPAR-mediated response. The current amplitude 50-70 ms after the current peak measured at +40 was used in the denominator to represent the NMDAR-mediated response. Data are displayed as the geometric mean ± SEM. Significance was determined by students t-test of the log ratio measured from each cell.; *p*<0.05 was considered significant.

### Example 10. Acid Strip Immunocytochemical Protocol

Briefly, the hippocampus can be removed form rats, trypsinized (0.25%), dissociated by trituration, and plated onto poly-L-lysine (1 mg/ml) coated glass coverslips (80,000 cells/ml) for 4 h. The coverslips are then transferred to dishes containing a monolayer of glial cells in growth medium and the neurons were allowed to mature for 14-22 days. Surface AMPARs are labeled on live cells with an antibody directed against the extracellular N-terminus of the GluR1 subunit (amino acids 271-285; 5 µg per ml; Oncogene Research, San Diego, Calif., and a gift of R. Huganir). The neurons can be treated with a specific agonist or antagonist, or control medium for 5 min, Ten or fifty-five minutes following treatment, the cells are chilled in 4° C. Tris-buffered saline (TBS) is used to stop endocytosis, and then exposed to 0.5 M NaCl/0.2 M acetic acid (pH 3.5) for 4 min on ice to remove antibody bound to extracellular GluR1. Cultures are rinsed and fixed in 4% paraformaldehyde with 4% sucrose. Nonspecific staining is blocked and cells permeabilized in TBS containing 0.1% Triton-X, 4% goat serum and 2% BSA. Internalized primary antibody is made visible by incubation with a Cy3-labeled secondary antibody for 1 h (1:300). Synapses can be detected using antibodies directed against presynaptic proteins (synapsin 1, 1:1000, Chemicon; synaptophysin, 1:100, Boehringer Manheim, Irvine, Calif.) for 1 h at room temperature. Cultures were then rinsed and exposed to the appropriate fluorescent secondary antibodies (Jackson Immunoresearch, West Grove, Pa.).

## Claims

1. An agent that increases the expression of, or increases activity of, α-amino-3-hydroxy-5-methyl-4-isoxazolepropionic acid receptor (AMPAR) at neuronal synapses for use in the treatment of Angelman Syndrome, wherein the agent is an antagonist of metabotropic glutamate receptor subtype 5 (mGluR5) that comprises 2-methyl-6-(phenylethynyl)-pyridine (MPEP) or 3-[(2-methyl-1,3-thiazol-4-yl)ethynyl]pyridine (MTEP).

2. An agent that increases the expression of, or increases activity of, α-amino-3-hydroxy-5-methyl-4-isoxazolepropionic acid receptor (AMPAR) at neuronal synapses for use in the treatment of Angelman Syndrome, wherein the agent is an RNA interfering agent (RNAi) directed against the synaptic protein activity-regulated cytoskeleton-associated protein (Arc) and inhibits the expression of Arc.

3. The agent for use in treatment of claim 2, wherein the RNAi comprises SEQ ID NO: 9 or SEQ ID NO: 10.

4. The agent for use in treatment of any of claims 1-3, wherein the agent is administered to a human subject.

5. The agent for use in treatment of claim 4, wherein the human subject has a loss of function mutation in the E3 ubiquitin ligase gene Ube3a or a maternal deletion of chromosome 15q11-q13.

## Patentansprüche

1. Mittel, das die Expression oder Aktivität eines α-Amino-3-hydroxy-5-methyl-4-isoxazolpropionsäurerezeptors (AMPAR) an neuronalen Synapsen steigert, zum Gebrauch beim Behandeln von Angelman-Syndrom, wobei das Mittel ein Antagonist von metabotropem Glutamat-Rezeptor-Subtyp 5 (mGluR5) ist, der 2-Methyl-6-(phenylethinyl)-pyridin (MPEP) oder 3-[(2-Methyl-1,3-thiazol-4-yl)ethinyl]pyridin (MTEP) umfasst.

2. Mittel, das die Expression oder Aktivität eines α-Amino-3-hydroxy-5-methyl-4-isoxazolpropionsäurerezeptors (AMPAR) an neuronalen Synapsen steigert, zum Gebrauch beim Behandeln von Angelman-Syndrom, wobei das Mittel ein RNA-Interferenzmittel (RNAi) ist, welches gegen das synaptische Protein aktivitätsreguliertes cytoskelettassoziiertes Protein (Arc) gerichtet ist und die Expression von Arc hemmt.

3. Mittel für den Gebrauch beim Behandeln nach Anspruch 2, wobei das RNAi SEQ ID NO: 9 oder SEQ ID NO: 10 umfasst.

4. Mittel für den Gebrauch beim Behandeln nach einem der Ansprüche 1-3, wobei das Mittel einem menschlichen Subjekt verabreicht wird.

5. Mittel für den Gebrauch beim Behandeln nach Anspruch 4, wobei das menschliche Subjekt eine Funktionsverlustmutation in dem E3-Ubiquitinligasegen Ube3a oder eine mütterliche Deletion von Chromosom 15q11-q13 aufweist.

## Revendications

1. Agent qui augmente l'expression ou qui augmente l'activité du récepteur à l'acide α-amino-3-hydroxy-5-méthyl-4-isoxazolpropionique (AMPA) au niveau des synapses neuronales pour une utilisation dans le traitement du syndrome d'Angelman, l'agent étant un antagoniste du récepteur-5 métabotropique du glutamate (mGluR5) qui comprend la 2-méthyl-6-(phényléthynyl)-pyridine (MPEP) ou la 3-[(2-méthyl-1,3-thiazol-4-yl)éthynyl]pyridine (MTEP).

2. Agent qui augmente l'expression ou qui augmente l'activité du récepteur à l'acide α-amino-3-hydroxy-5-méthyl-4-isoxazolpropionique (AMPA) au niveau des synapses neuronales pour une utilisation dans le traitement du syndrome d'Angelman, l'agent étant un ARN interférent (ARNi) dirigé contre la protéine Arc (*activity-regulated cytoskeleton-associated protein)* synaptique et inhibant l'expression de la protéine Arc.

3. Agent pour l'utilisation thérapeutique selon la revendication 2, où l'ARNi comprend la SEQ ID N° : 9 ou la SEQ ID N° : 10.

4. Agent pour l'utilisation thérapeutique selon l'une quelconque des revendications 1-3, l'agent étant administré à un sujet humain.

5. Agent pour l'utilisation thérapeutique selon la revendication 4, où le sujet humain a une mutation conduisant à une perte de fonction du gène Ube3a (gène de l'ubiquitine ligase E3) ou une délétion de la région 15q11-q13 du chromosome 15 d'origine maternelle.


### SEQUENCE LISTING

<110> PRESIDENT AND FELLOWS OF HARVARD COLLEGE
<120> METHODS AND COMPOSITIONS FOR TREATMENT OF ANGELMAN SYNDROME AND AUTISM SPECTRUM DISORDERS
<130> 002806-067391-PCT
<140> PCT/US11/26687
   <141> 2011-03-01
<150> 61/309, 557
   <151> 2010-03-02
<160> 48
<170> PatentIn version 3.5
<210> 1
   <211> 55
   <212> PRT
   <213> Homo sapiens
<400> 1
   Pro Ala Glu Phe Ile Arg Phe His Pro Gln Tyr Gln Glu Val Asn Gly
   1               5                   10                  15
   Thr Leu Gln Val Asn Pro Lys Phe Lys Gln Asp Val Leu Gln Leu Leu
               20                  25                  30
   Trp Thr Ser Cys Pro Ile Leu Pro Glu Lys Ala Thr Pro Pro Gln Glu
           35                  40                  45
   Gln Leu Glu Gln Val Leu Asn
       50                  55
<210> 2
   <211> 55
   <212> PRT
   <213> Homo sapiens
<400> 2
   Pro Leu Glu Phe Leu Arg Asp Gln Pro Gln Phe Gln Asn Met Arg Gln
   1               5                   10                  15
   Val Ile Gln Gln Asn Pro Ala Leu Leu Pro Ala Leu Leu Gln Gln Leu
               20                  25                  30
   Gly Gln Glu Asn Pro Gln Leu Leu Gln Gln Ile Ser Arg His Gln Glu
           35                  40                  45
   Gln Phe Ile Gln Met Leu Asn
       50                  55
<210> 3
   <211> 64
   <212> PRT
   <213> Homo sapiens
<400> 3
   Glu Phe Lys Gln Gly Ser Val Lys Asn Trp Val Glu Phe Lys Lys Glu
   1               5                   10                  15
   Phe Leu Gln Tyr Ser Glu Gly Thr Leu Ser Arg Glu Ala Ile Gln Arg
               20                  25                  30
   Glu Leu Glu Leu Pro Gln Lys Gln Gly Glu Pro Leu Asp Gln Phe Leu
           35                  40                  45
   Trp Arg Lys Arg Asp Leu Tyr Gln Thr Leu Tyr Val Asp Ala Asp Glu
       50                  55                  60
<210> 4
   <211> 58
   <212> PRT
   <213> Homo sapiens
<400> 4
   Glu Phe Leu Arg Asp Gln Pro Gln Phe Gln Asn Met Arg Gln Val Ile
   1               5                   10                  15
   Gln Gln Asn Pro Ala Leu Leu Pro Ala Leu Leu Gln Gln Leu Gly Gln
               20                  25                  30
   Glu Asn Pro Gln Leu Leu Gln Gln Ile Ser Arg His Gln Glu Gln Phe
           35                  40                  45
   Ile Gln Met Leu Asn Glu Pro Pro Gly Glu
       50                  55
<210> 5
   <211> 15
   <212> PRT
   <213> Artificial Sequence
<220>
   <223> Description of Artificial Sequence: Synthetic peptide
<400> 5
   Lys Gly Gly Glu Phe Leu Gln Tyr Ser Glu Gly Thr Leu Ser Arg
   1               5                   10                  15
<210> 6
   <211> 14
   <212> PRT
   <213> Artificial Sequence
<220>
   <223> Description of Artificial Sequence: Synthetic peptide
<400> 6
   Leu Ile Phe Ala Gly Lys Gly Gly Gln Leu Glu Asp Gly Arg
   1               5                   10
<210> 7
   <211> 19
   <212> DNA
   <213> Artificial Sequence
<220>
   <223> Description of Artificial Sequence: Synthetic oligonucleotide
<400> 7
   tctccacagt cctgaatat
     19
<210> 8
   <211> 19
   <212> DNA
   <213> Artificial Sequence
<220>
   <223> Description of Artificial Sequence: Synthetic oligonucleotide
<400> 8
   cccaatgatg tatgatcta
     19
<210> 9
   <211> 19
   <212> DNA
   <213> Artificial Sequence
<220>
   <223> Description of Artificial Sequence: Synthetic oligonucleotide
<400> 9
   acccaatgtg atcctgcag
   19
<210> 10
   <211> 19
   <212> DNA
   <213> Artificial Sequence
<220>
   <223> Description of Artificial Sequence: Synthetic oligonucleotide
<400> 10
   gctgatggct acgactaca
   19
<210> 11
   <211> 22
   <212> DNA
   <213> Artificial Sequence
<220>
   <223> Description of Artificial Sequence: Synthetic oligonucleotide
<400> 11
   tctgcatagc ccggagtacc tg
   22
<210> 12
   <211> 22
   <212> DNA
   <213> Artificial Sequence
<220>
   <223> Description of Artificial Sequence: Synthetic oligonucleotide
<400> 12
   tccgatgatg tacgacctga ag
   22
<210> 13
   <211> 19
   <212> DNA
   <213> Artificial Sequence
<220>
   <223> Description of Artificial Sequence: Synthetic oligonucleotide
<400> 13
   accgaacgtc atactccaa
   19
<210> 14
   <211> 19
   <212> DNA
   <213> Artificial Sequence
<220>
   <223> Description of Artificial Sequence: Synthetic oligonucleotide
<400> 14
   gcggacgggt atgattata
   19
<210> 15
   <211> 20
   <212> DNA
   <213> Artificial Sequence
<220>
   <223> Description of Artificial Sequence: Synthetic primer
<400> 15
   accgtcccct cctctcttga
   20
<210> 16
   <211> 27
   <212> DNA
   <213> Artificial Sequence
<220>
   <223> Description of Artificial Sequence: Synthetic primer
<400> 16
   tctttgtaat cctattttct ctgcctt
   27
<210> 17
   <211> 19
   <212> DNA
   <213> Artificial Sequence
<220>
   <223> Description of Artificial Sequence: Synthetic primer
<400> 17
   cccgagggct caagatgtc
   19
<210> 18
   <211> 27
   <212> DNA
   <213> Artificial Sequence
<220>
   <223> Description of Artificial Sequence: Synthetic primer
<400> 18
   tctttgtaat cctattttct ctgcctt
   27
<210> 19
   <211> 18
   <212> DNA
   <213> Artificial Sequence
<220>
   <223> Description of Artificial Sequence: Synthetic primer
<400> 19
   aaagcgggag ctgaggct
   18
<210> 20
   <211> 23
   <212> DNA
   <213> Artificial Sequence
<220>
   <223> Description of Artificial Sequence: Synthetic primer
<400> 20
   ttctttcttc ttcctgcgac act
   23
<210> 21
   <211> 24
   <212> DNA
   <213> Artificial Sequence
<220>
   <223> Description of Artificial Sequence: Synthetic primer
<400> 21
   tccatgacaa ctttggcatc gtgg
   24
<210> 22
   <211> 24
   <212> DNA
   <213> Artificial Sequence
<220>
   <223> Description of Artificial Sequence: Synthetic primer
<400> 22
   gtttctgttg aagtcacagg agac
   24
<210> 23
   <211> 20
   <212> DNA
   <213> Artificial Sequence
<220>
   <223> Description of Artificial Sequence: Synthetic primer
<400> 23
   tcctctttgg gtgactccag
   20
<210> 24
   <211> 20
   <212> DNA
   <213> Artificial Sequence
<220>
   <223> Description of Artificial Sequence: Synthetic primer
<400> 24
   cggaagagaa gcgtaacgag
   20
<210> 25
   <211> 20
   <212> DNA
   <213> Artificial Sequence
<220>
   <223> Description of Artificial Sequence: Synthetic primer
<400> 25
   gctctggtgg ggaagacata
   20
<210> 26
   <211> 20
   <212> DNA
   <213> Artificial Sequence
<220>
   <223> Description of Artificial Sequence: Synthetic primer
<400> 26
   ccagaagcag cacacgaata
   20
<210> 27
   <211> 22
   <212> DNA
   <213> Artificial Sequence
<220>
   <223> Description of Artificial Sequence: Synthetic primer
<400> 27
   agaaacctca tagtgcttgc ag
   22
<210> 28
   <211> 20
   <212> DNA
   <213> Artificial Sequence
<220>
   <223> Description of Artificial Sequence: Synthetic primer
<400> 28
   ttctcaactc tggccatcaa
   20
<210> 29
   <211> 20
   <212> DNA
   <213> Artificial Sequence
<220>
   <223> Description of Artificial Sequence: Synthetic primer
<400> 29
   tctgccctct ctacgtcagg
   20
<210> 30
   <211> 20
   <212> DNA
   <213> Artificial Sequence
<220>
   <223> Description of Artificial Sequence: Synthetic primer
<400> 30
   atgaaacgaa accccacaag
   20
<210> 31
   <211> 5747
   <212> DNA
   <213> Homo sapiens
<400> 31
   atagagcttg ctgcctgtgt gagtgtgagg gggagagcga gagagagcaa gggagggaga 60
   gagaggcagg ctgcgagggg agaggagagg gagtggggga gccagcgctc cagctagcat 120
   gaggacgggc ttcttttccc gtgctcagtt aatctggctg tcagttggtg ttaacgctgc 180
   agtttaagtg ttcggattcc aagggaaaca gacaaacctc acgaaaggaa ggaagcaagc 240
   aagcaaggaa ggaactgcag gaggaaaaga acaggcagaa cagcgagaag aataaaggga 300
   aaggggggga aacaccaaat ctatgattgg acctgggctt ctttttcgcc aatgcaaaaa 360
   ggaatatgca gcacattttt gccttcttct gcaccggttt cctaggcgcg gtagtaggtg 420
   ccaatttccc caacaatatc cagatcgggg gattatttcc aaaccagcag tcacaggaac 480
   atgctgcttt tagatttgct ttgtcgcaac tcacagagcc cccgaagctg ctcccccaga 540
   ttgatattgt gaacatcagc gacagctttg agatgaccta tagattctgt tcccagttct 600
   ccaaaggagt ctatgccatc tttgggtttt atgaacgtag gactgtcaac atgctgacct 660
   ccttttgtgg ggccctccac gtctgcttca ttacgccgag ctttcccgtt gatacatcca 720
   atcagtttgt ccttcagctg cgccctgaac tgcaggatgc cctcatcagc atcattgacc 780
   attacaagtg gcagaaattt gtctacattt atgatgccga ccggggctta tccgtcctgc 840
   agaaagtcct ggatacagct gctgagaaga actggcaggt gacagcagtc aacattttga 900
   caaccacaga ggagggatac cggatgctct ttcaggacct ggagaagaaa aaggagcggc 960
   tggtggtggt ggactgtgaa tcagaacgcc tcaatgctat cttgggccag attataaagc 1020
   tagagaagaa tggcatcggc taccactaca ttcttgcaaa tctgggcttc atggacattg 1080
   acttaaacaa attcaaggag agtggcgcca atgtgacagg tttccagctg gtgaactaca 1140
   cagacactat tccggccaag atcatgcagc agtggaagaa tagtgatgct cgagaccaca 1200
   cacgggtgga ctggaagaga cccaagtaca cctctgcgct cacctacgat ggggtgaagg 1260
   tgatggctga ggctttccag agcctgcgga ggcagagaat tgatatatct cgccggggga 1320
   atgctgggga ttgtctggct aacccagctg ttccctgggg ccaagggatc gacatccaga 1380
   gagctctgca gcaggtgcga tttgaaggtt taacaggaaa cgtgcagttt aatgagaaag 1440
   gacgccggac caactacacg ctccacgtga ttgaaatgaa acatgacggc atccgaaaga 1500
   ttggttactg gaatgaagat gataagtttg tccctgcagc caccgatgcc caagctgggg 1560
   gcgataattc aagtgttcag aacagaacat acatcgtcac aacaatccta gaagatcctt 1620
   atgtgatgct caagaagaac gccaatcagt ttgagggcaa tgaccgttac gagggctact 1680
   gtgtagagct ggcggcagag attgccaagc acgtgggcta ctcctaccgt ctggagattg 1740
   tcagtgatgg aaaatacgga gcccgagacc ctgacacgaa ggcctggaat ggcatggtgg 1800
   gagagctggt ctatggaaga gcagatgtgg ctgtggctcc cttaactatc actttggtcc 1860
   gggaagaagt tatagatttc tccaaaccat ttatgagttt ggggatctcc atcatgatta 1920
   aaaaaccaca gaaatccaag ccgggtgtct tctccttcct tgatcctttg gcttatgaga 1980
   tttggatgtg cattgttttt gcctacattg gagtgagtgt tgtcctcttc ctggtcagcc 2040
   gcttcagtcc ctatgaatgg cacagtgaag agtttgagga aggacgggac cagacaacca
   2100
   gtgaccagtc caatgagttt gggatattca acagtttgtg gttctccctg ggagccttca 2160
   tgcagcaagg atgtgacatt tctcccaggt ccctgtctgg tcgcatcgtt ggtggcgtct 2220
   ggtggttctt caccttaatc atcatctcct catatacagc caatctggcc gccttcctga 2280
   ccgtggagag gatggtgtct cccattgaga gtgcagagga cctagcgaag cagacagaaa 2340
   ttgcctacgg gacgctggaa gcaggatcta ctaaggagtt cttcaggagg tctaaaattg 2400
   ctgtgtttga gaagatgtgg acatacatga agtcagcaga gccatcagtt tttgtgcgga 2460
   ccacagagga ggggatgatt cgagtgagga aatccaaagg caaatatgcc tacctcctgg 2520
   agtccaccat gaatgagtac attgagcagc ggaaaccctg tgacaccatg aaggtgggag 2580
   gtaacttgga ttccaaaggc tatggcattg caacacccaa ggggtctgcc ctgagaaatc 2640
   cagtaaacct ggcagtgtta aaactgaacg agcaggggct tttggacaaa ttgaaaaaca 2700
   aatggtggta cgacaagggc gagtgcggca gcgggggagg tgattccaag gacaagacaa 2760
   gcgctctgag cctcagcaat gtggcaggcg tgttctacat cctgatcgga ggacttggac 2820
   tagccatgct ggttgcctta atcgagttct gctacaaatc ccgtagtgaa tccaagcgga 2880
   tgaagggttt ttgtttgatc ccacagcaat ccatcaacga agccatacgg acatcgaccc 2940
   tcccccgcaa cagcggggca ggagccagca gcggcggcag tggagagaat ggtcgggtgg 3000
   tcagccatga cttccccaag tccatgcaat cgattccttg catgagccac agttcaggga 3060
   tgcccttggg agccacggga ttgtaactgg agcagatgga gaccccttgg ggagcaggct 3120
   cgggctcccc agccccatcc caaacccttc agtgccaaaa acaacaacaa aatgaaacgc 3180
   aaccaccacc aaccactgcg accacaagaa ggatgattca acaggttttc ctgaagaatt 3240
   gaaaaaccat tttgctgtcc cttttccttt tttgatgttc tttcaccctt ttctgtttgc 3300
   taagtgagga tgaaaaaata acactgtact gcaataaggg gagagtaacc ctgtctaatg 3360
   aaacctgtgt ctctgagagt agagtcactg gaacactaat gaggaaactg cactgtttta 3420
   ttttaattca gttgttagtg tgtcttagtg tgtgcaattt tttttcttac taatatccat 3480
   ggtttgcagg ttctgttagg ccctttcctt ctccttactt cttatcccca actccctacc 3540
   cacccctctt cagttttcag attggagatt caagatttgt tccactttac aagcaagagg 3600
   aaaaaaaagc aaccttcaaa ctaattctcc atgggggctc tccatgttac cctccactcc 3660
   ttggcccaaa cctctgatgg agatagacat tgttggagaa gtgggctgcc ttccccaagt 3720
   ggggcactgc ttaagcactt attcagtgga gaacacaggt gaaaagcaac tcaggatgag 3780
   ggtggtggag agggcagggg cagatgtgca gtcagagaag gactcctgaa gttactgctg 3840
   ctcagaaaaa cagttccttt aatgtggaag agccatttca taggtcatag gtggtatggt 3900
   atatttcttc agagtcaacc ttggccctga gaagtatgtc ctcctggtgt gctcaggctc 3960
   aacggcagtc tggtggctga aggcacttgg cctcctaaac caagcagaat tttgggaaga 4020
   gataacagcc agggagatat tgcccatgat tctcactttt tctttgcctg gcatctaagc 4080
   aggaacccat tgtggagtag actctcttct tctatggagc ctctgacatg gggagcaatg 4140
   ctaagcaagc taagtgtaaa agaaaagtga cagaataatt ttggaagagg aagcctcatc 4200
   aaaagctcac acaaaataga gcttcccatg gtgtgcccta tcctaggttt aagaaaacac 4260
   gtatgaagtt tatgctgatg caaagaactt gggtttttat gttaatataa agtgttgttt 4320
   tagcatgtgg ccagatgatg ctctgtcatc tttagaaagt gagataacca aggaaataat 4380
   tgaaggagta tagggagatg gattaagttg ataatgacat ttagggcaac ttaagacctt
   4440
   tgatcccagg ttctaactca aagaggctga ccttccccca gctaagatag catgaggacg
   4500
   ttgtattcca atatacgtat gattggggct acaaagctga actaaagcaa gattggtgaa
   4560
   gtggcagggt ttatagagag aagcccaggc tgagttcagc ttttgttgga agtgagaatc
   4620
   cctgacatat agctttcttg gagatcccaa ctctcattct tggtgcaact ggcttccagc
   4680
   tctccagcag tcactctcct aggtgcatga ttcagtgcgt gccatgtgtc attagctttt
   4740
   actgataacc atattctggc ttgttccctt accccctact tctatccaat tttctctgct
   4800
   aggggttatc attagcaatt gacatgctaa aggttttgga gcccacctag gggtaggtgc
   4860
   agctttattg gcttttctgt ggattctctc agtggaccca caccatctct atgtctctcc
   4920
   actctcctgc cttcagccat agcaaagaat ccttccaaaa tcaaactctt cacttttttg
   4980
   actcaagtgt tgttgttcag tctctcgcgt gtcaatgtgg tcatggttca tgaaaccgga
   5040
   ccctcaagat ggatgattgc ttttaactac tgccagctga tgtctctcag cccctgccct
   5100
   catacaagat ttttctcagc cttcagccta ccactgcaga atccgatgtg acccaccatt
   5160
   agggagtctg catcttggaa gagttggaaa taacccttta acatcaacat gcttcaaaga
   5220
   ctttttgcct ttggcctagt aagatgcctc tccagctact gagcccacaa gtaacatgag
   5280
   cggataaaaa gagacttgtt tgtgctagaa atgagggtct atgctatgag ggggtccaag
   5340
   actctggcga aatgtgcttt ttcatcaatg gagaaatgaa aggaaaacac aagcaagaaa
   5400
   aaagttaact tgtattatgt atttttacta cacttttctt aaaaatagag cattgggaaa
   5460
   actctgaaag agactgacat ttttctcaac aggaatccat acttaacagt tctggctttc
   5520
   attaaatttt gctctttggt acctgggcct tttatttaac atctatattt gttttaactc
   5580
   tcttggcaga tgtgtgaaag gattcttgct tgatcaaaca ctaagtattt ttttggttct 5640
   tgtttttctt tcaaatagcc aggttttttt cttttggtat ttgcataaaa tgaaaatatc 5700
   accgaatatt aaatcactgt ggatccatta aaaaaaaaaa aaaaaaa 5747
<210> 32
   <211> 5747
   <212> DNA
   <213> Homo sapiens
<400> 32
   atagagcttg ctgcctgtgt gagtgtgagg gggagagcga gagagagcaa gggagggaga 60
   gagaggcagg ctgcgagggg agaggagagg gagtggggga gccagcgctc cagctagcat 120
   gaggacgggc ttcttttccc gtgctcagtt aatctggctg tcagttggtg ttaacgctgc 180
   agtttaagtg ttcggattcc aagggaaaca gacaaacctc acgaaaggaa ggaagcaagc 240
   aagcaaggaa ggaactgcag gaggaaaaga acaggcagaa cagcgagaag aataaaggga 300
   aaggggggga aacaccaaat ctatgattgg acctgggctt ctttttcgcc aatgcaaaaa 360
   ggaatatgca gcacattttt gccttcttct gcaccggttt cctaggcgcg gtagtaggtg 420
   ccaatttccc caacaatatc cagatcgggg gattatttcc aaaccagcag tcacaggaac 480
   atgctgcttt tagatttgct ttgtcgcaac tcacagagcc cccgaagctg ctcccccaga 540
   ttgatattgt gaacatcagc gacagctttg agatgaccta tagattctgt tcccagttct 600
   ccaaaggagt ctatgccatc tttgggtttt atgaacgtag gactgtcaac atgctgacct 660
   ccttttgtgg ggccctccac gtctgcttca ttacgccgag ctttcccgtt gatacatcca 720
   atcagtttgt ccttcagctg cgccctgaac tgcaggatgc cctcatcagc atcattgacc 780
   attacaagtg gcagaaattt gtctacattt atgatgccga ccggggctta tccgtcctgc
   840
   agaaagtcct ggatacagct gctgagaaga actggcaggt gacagcagtc aacattttga
   900
   caaccacaga ggagggatac cggatgctct ttcaggacct ggagaagaaa aaggagcggc
   960
   tggtggtggt ggactgtgaa tcagaacgcc tcaatgctat cttgggccag attataaagc
   1020
   tagagaagaa tggcatcggc taccactaca ttcttgcaaa tctgggcttc atggacattg
   1080
   acttaaacaa attcaaggag agtggcgcca atgtgacagg tttccagctg gtgaactaca
   1140
   cagacactat tccggccaag atcatgcagc agtggaagaa tagtgatgct cgagaccaca
   1200
   cacgggtgga ctggaagaga cccaagtaca cctctgcgct cacctacgat ggggtgaagg
   1260
   tgatggctga ggctttccag agcctgcgga ggcagagaat tgatatatct cgccggggga
   1320
   atgctgggga ttgtctggct aacccagctg ttccctgggg ccaagggatc gacatccaga
   1380
   gagctctgca gcaggtgcga tttgaaggtt taacaggaaa cgtgcagttt aatgagaaag
   1440
   gacgccggac caactacacg ctccacgtga ttgaaatgaa acatgacggc atccgaaaga
   1500
   ttggttactg gaatgaagat gataagtttg tccctgcagc caccgatgcc caagctgggg
   1560
   gcgataattc aagtgttcag aacagaacat acatcgtcac aacaatccta gaagatcctt
   1620
   atgtgatgct caagaagaac gccaatcagt ttgagggcaa tgaccgttac gagggctact
   1680
   gtgtagagct ggcggcagag attgccaagc acgtgggcta ctcctaccgt ctggagattg
   1740
   tcagtgatgg aaaatacgga gcccgagacc ctgacacgaa ggcctggaat ggcatggtgg
   1800
   gagagctggt ctatggaaga gcagatgtgg ctgtggctcc cttaactatc actttggtcc
   1860
   gggaagaagt tatagatttc tccaaaccat ttatgagttt ggggatctcc atcatgatta
   1920
   aaaaaccaca gaaatccaag ccgggtgtct tctccttcct tgatcctttg gcttatgaga
   1980
   tttggatgtg cattgttttt gcctacattg gagtgagtgt tgtcctcttc ctggtcagcc 2040
   gcttcagtcc ctatgaatgg cacagtgaag agtttgagga aggacgggac cagacaacca 2100
   gtgaccagtc caatgagttt gggatattca acagtttgtg gttctccctg ggagccttca 2160
   tgcagcaagg atgtgacatt tctcccaggt ccctgtctgg tcgcatcgtt ggtggcgtct 2220
   ggtggttctt caccttaatc atcatctcct catatacagc caatctggcc gccttcctga 2280
   ccgtggagag gatggtgtct cccattgaga gtgcagagga cctagcgaag cagacagaaa 2340
   ttgcctacgg gacgctggaa gcaggatcta ctaaggagtt cttcaggagg tctaaaattg 2400
   ctgtgtttga gaagatgtgg acatacatga agtcagcaga gccatcagtt tttgtgcgga 2460
   ccacagagga ggggatgatt cgagtgagga aatccaaagg caaatatgcc tacctcctgg 2520
   agtccaccat gaatgagtac attgagcagc ggaaaccctg tgacaccatg aaggtgggag 2580
   gtaacttgga ttccaaaggc tatggcattg caacacccaa ggggtctgcc ctgagaggtc 2640
   ccgtaaacct agcggttttg aaactcagtg agcaaggcgt cttagacaag ctgaaaagca 2700
   aatggtggta cgataaaggg gaatgtggaa gcaaggactc cggaagtaag gacaagacaa 2760
   gcgctctgag cctcagcaat gtggcaggcg tgttctacat cctgatcgga ggacttggac 2820
   tagccatgct ggttgcctta atcgagttct gctacaaatc ccgtagtgaa tccaagcgga 2880
   tgaagggttt ttgtttgatc ccacagcaat ccatcaacga agccatacgg acatcgaccc 2940
   tcccccgcaa cagcggggca ggagccagca gcggcggcag tggagagaat ggtcgggtgg 3000
   tcagccatga cttccccaag tccatgcaat cgattccttg catgagccac agttcaggga
   3060
   tgcccttggg agccacggga ttgtaactgg agcagatgga gaccccttgg ggagcaggct
   3120
   cgggctcccc agccccatcc caaacccttc agtgccaaaa acaacaacaa aatgaaacgc 3180
   aaccaccacc aaccactgcg accacaagaa ggatgattca acaggttttc ctgaagaatt 3240
   gaaaaaccat tttgctgtcc cttttccttt tttgatgttc tttcaccctt ttctgtttgc 3300
   taagtgagga tgaaaaaata acactgtact gcaataaggg gagagtaacc ctgtctaatg 3360
   aaacctgtgt ctctgagagt agagtcactg gaacactaat gaggaaactg cactgtttta 3420
   ttttaattca gttgttagtg tgtcttagtg tgtgcaattt tttttcttac taatatccat 3480
   ggtttgcagg ttctgttagg ccctttcctt ctccttactt cttatcccca actccctacc 3540
   cacccctctt cagttttcag attggagatt caagatttgt tccactttac aagcaagagg 3600
   aaaaaaaagc aaccttcaaa ctaattctcc atgggggctc tccatgttac cctccactcc 3660
   ttggcccaaa cctctgatgg agatagacat tgttggagaa gtgggctgcc ttccccaagt 3720
   ggggcactgc ttaagcactt attcagtgga gaacacaggt gaaaagcaac tcaggatgag 3780
   ggtggtggag agggcagggg cagatgtgca gtcagagaag gactcctgaa gttactgctg 3840
   ctcagaaaaa cagttccttt aatgtggaag agccatttca taggtcatag gtggtatggt 3900
   atatttcttc agagtcaacc ttggccctga gaagtatgtc ctcctggtgt gctcaggctc 3960
   aacggcagtc tggtggctga aggcacttgg cctcctaaac caagcagaat tttgggaaga 4020
   gataacagcc agggagatat tgcccatgat tctcactttt tctttgcctg gcatctaagc 4080
   aggaacccat tgtggagtag actctcttct tctatggagc ctctgacatg gggagcaatg 4140
   ctaagcaagc taagtgtaaa agaaaagtga cagaataatt ttggaagagg aagcctcatc 4200
   aaaagctcac acaaaataga gcttcccatg gtgtgcccta tcctaggttt aagaaaacac 4260
   gtatgaagtt tatgctgatg caaagaactt gggtttttat gttaatataa agtgttgttt
   4320
   tagcatgtgg ccagatgatg ctctgtcatc tttagaaagt gagataacca aggaaataat 4380
   tgaaggagta tagggagatg gattaagttg ataatgacat ttagggcaac ttaagacctt 4440
   tgatcccagg ttctaactca aagaggctga ccttccccca gctaagatag catgaggacg 4500
   ttgtattcca atatacgtat gattggggct acaaagctga actaaagcaa gattggtgaa 4560
   gtggcagggt ttatagagag aagcccaggc tgagttcagc ttttgttgga agtgagaatc 4620
   cctgacatat agctttcttg gagatcccaa ctctcattct tggtgcaact ggcttccagc 4680
   tctccagcag tcactctcct aggtgcatga ttcagtgcgt gccatgtgtc attagctttt 4740
   actgataacc atattctggc ttgttccctt accccctact tctatccaat tttctctgct 4800
   aggggttatc attagcaatt gacatgctaa aggttttgga gcccacctag gggtaggtgc 4860
   agctttattg gcttttctgt ggattctctc agtggaccca caccatctct atgtctctcc 4920
   actctcctgc cttcagccat agcaaagaat ccttccaaaa tcaaactctt cacttttttg 4980
   actcaagtgt tgttgttcag tctctcgcgt gtcaatgtgg tcatggttca tgaaaccgga 5040
   ccctcaagat ggatgattgc ttttaactac tgccagctga tgtctctcag cccctgccct 5100
   catacaagat ttttctcagc cttcagccta ccactgcaga atccgatgtg acccaccatt 5160
   agggagtctg catcttggaa gagttggaaa taacccttta acatcaacat gcttcaaaga 5220
   ctttttgcct ttggcctagt aagatgcctc tccagctact gagcccacaa gtaacatgag 5280
   cggataaaaa gagacttgtt tgtgctagaa atgagggtct atgctatgag ggggtccaag 5340
   actctggcga aatgtgcttt ttcatcaatg gagaaatgaa aggaaaacac aagcaagaaa 5400
   aaagttaact tgtattatgt atttttacta cacttttctt aaaaatagag cattgggaaa 5460
   actctgaaag agactgacat ttttctcaac aggaatccat acttaacagt tctggctttc 5520
   attaaatttt gctctttggt acctgggcct tttatttaac atctatattt gttttaactc 5580
   tcttggcaga tgtgtgaaag gattcttgct tgatcaaaca ctaagtattt ttttggttct 5640
   tgtttttctt tcaaatagcc aggttttttt cttttggtat ttgcataaaa tgaaaatatc 5700
   accgaatatt aaatcactgt ggatccatta aaaaaaaaaa aaaaaaa 5747
<210> 33
   <211> 5755
   <212> DNA
   <213> Homo sapiens
<400> 33
   gagtcgcgca cgcgcgcccg ggactgcctg cccctctctg tgacttgcct gtgtgtgtgc 60
   gtgtgtgtat gtgtgtgtgt gtgtgtgtgt gcgcgcgcgc gtgagtgaga gaggagagag 120
   ggagaagaga gcgcgagaga gggtgagtgt gtgtgagtgc atgggagggt gctgaatatt 180
   ccgagacact gggaccacag cggcagctcc gctgaaaact gcattcagcc agtcctccgg 240
   acttctggag cggggacagg gcgcagggca tcagcagcca ccagcaggac ctgggaaata 300
   gggattcttc tgcctccact tcaggtttta gcagcttggt gctaaattgc tgtctcaaaa 360
   tgcagaggat ctaatttgca gaggaaaaca gccaaagaag gaagaggagg aaaaggaaaa 420
   aaaaaggggt atattgtgga tgctctactt ttcttggaaa tgcaaaagat tatgcatatt 480
   tctgtcctcc tttctcctgt tttatgggga ctgatttttg gtgtctcttc taacagcata 540
   cagatagggg ggctatttcc taggggcgcc gatcaagaat acagtgcatt tcgagtaggg 600
   atggttcagt tttccacttc ggagttcaga ctgacacccc acatcgacaa tttggaggtg 660
   gcaaacagct tcgcagtcac taatgctttc tgctcccagt tttcgagagg agtctatgct 720
   atttttggat tttatgacaa gaagtctgta aataccatca catcattttg cggaacactc 780
   cacgtctcct tcatcactcc cagcttccca acagatggca cacatccatt tgtcattcag 840
   atgagacccg acctcaaagg agctctcctt agcttgattg aatactatca atgggacaag 900
   tttgcatacc tctatgacag tgacagaggc ttatcaacac tgcaagctgt gctggattct 960
   gctgctgaaa agaaatggca agtgactgct atcaatgtgg gaaacattaa caatgacaag 1020
   aaagatgaga tgtaccgatc actttttcaa gatctggagt taaaaaagga acggcgtgta 1080
   attctggact gtgaaaggga taaagtaaac gacattgtag accaggttat taccattgga 1140
   aaacatgtta aagggtacca ctacatcatt gcaaatctgg gatttactga tggagaccta 1200
   ttaaaaatcc agtttggagg tgcaaatgtc tctggatttc agatagtgga ctatgatgat 1260
   tcgttggtat ctaaatttat agaaagatgg tcaacactgg aagaaaaaga ataccctgga 1320
   gctcacacaa caacaattaa gtatacttct gctctgacct atgatgccgt tcaagtgatg 1380
   actgaagcct tccgcaacct aaggaagcaa agaattgaaa tctcccgaag ggggaatgca 1440
   ggagactgtc tggcaaaccc agcagtgccc tggggacaag gtgtagaaat agaaagggcc 1500
   ctcaaacagg ttcaggttga aggtctctca ggaaatataa agtttgacca gaatggaaaa 1560
   agaataaact atacaattaa catcatggag ctcaaaacta atgggccccg gaagattggc 1620
   tactggagtg aagtggacaa aatggttgtt acccttactg agctcccttc tggaaatgac 1680
   acctctgggc ttgagaataa gactgttgtt gtcaccacaa ttttggaatc tccgtatgtt 1740
   atgatgaaga aaaatcatga aatgcttgaa ggcaatgagc gctatgaggg ctactgtgtt 1800
   gacctggctg cagaaatcgc caaacattgt gggttcaagt acaagttgac aattgttggt 1860
   gatggcaagt atggggccag ggatgcagac acgaaaattt ggaatgggat ggttggagaa 1920
   cttgtatatg ggaaagctga tattgcaatt gctccattaa ctattaccct tgtgagagaa 1980
   gaggtgattg acttctcaaa gcccttcatg agcctcggga tatctatcat gatcaagaag 2040
   cctcagaagt ccaaaccagg agtgttttcc tttcttgatc ctttagccta tgagatctgg 2100
   atgtgcattg tttttgccta cattggggtc agtgtagttt tattcctggt cagcagattt 2160
   agcccctacg agtggcacac tgaggagttt gaagatggaa gagaaacaca aagtagtgaa 2220
   tcaactaatg aatttgggat ttttaatagt ctctggtttt ccttgggtgc ctttatgcgg 2280
   caaggatgcg atatttcgcc aagatccctc tctgggcgca ttgttggagg tgtgtggtgg 2340
   ttctttaccc tgatcataat ctcctcctac acggctaact tagctgcctt cctgactgta 2400
   gagaggatgg tgtctcccat cgaaagtgct gaggatcttt ctaagcaaac agaaattgct 2460
   tatggaacat tagactctgg ctccactaaa gagtttttca ggagatctaa aattgcagtg 2520
   tttgataaaa tgtggaccta catgcggagt gcggagccct ctgtgtttgt gaggactacg 2580
   gccgaagggg tggctagagt gcggaagtcc aaagggaaat atgcctactt gttggagtcc 2640
   acgatgaacg agtacattga gcaaaggaag ccttgcgaca ccatgaaagt tggtggaaac 2700
   ctggattcca aaggctatgg catcgcaaca cctaaaggat cctcattaag aaccccagta 2760
   aatcttgcag tattgaaact cagtgagcaa ggcgtcttag acaagctgaa aaacaaatgg 2820
   tggtacgata aaggtgaatg tggagccaag gactctggaa gtaaggaaaa gaccagtgcc 2880
   ctcagtctga gcaacgttgc tggagtattc tacatccttg tcgggggcct tggtttggca 2940
   atgctggtgg ctttgattga gttctgttac aagtcaaggg ccgaggcgaa acgaatgaag 3000
   gtggcaaaga atgcacagaa tattaaccca tcttcctcgc agaattcaca gaattttgca
   3060
   acttataagg aaggttacaa cgtatatggc atcgaaagtg ttaaaattta ggggatgacc 3120
   ttgaatgatg ccatgaggaa caaggcaagg ctgtcaatta caggaagtac tggagaaaat 3180
   ggacgtgtta tgactccaga atttcccaaa gcagtgcatg ctgtccctta cgtgagtcct 3240
   ggcatgggaa tgaatgtcag tgtgactgat ctctcgtgat tgataagaac cttttgagtg 3300
   ccttacacaa tggttttctt gtgtgtttat tgtcaaagtg gtgagaggca tccagtatct 3360
   tgaagacttt tctttcagcc aagaattctt aaatatgtgg agttcatctt gaattgtaag 3420
   gaatgattaa ttaaaacaca acatcttttt ctactcgagt tacagacaaa gcgtggtgga 3480
   catgcacagc taacatggaa gtactataat ttacctgaag tctttgtaca gacaacaaac 3540
   ctgtttctgc agccactatt gttagtctct tgattcataa tgacttaagc acacttgaca 3600
   tcaactgcat caagatgtga catgttttat aaaaaaagga aaaaaaacat ttaaaactaa 3660
   aaaatatttt taggtatttt cacaaacaaa ctggctttta aataaatttg cttccatatt 3720
   ggttgaataa gacaaaaaca attaaactga gtgggaagtg aataaaaaaa ggctttaggt 3780
   atcgattcca tatttttcaa agccaaatat gtaaatgcta aggaaagtaa acaaagagga 3840
   gattccaatc ttgtaattta atattgttat taaaacttta atgtatccta ttctttaaca 3900
   tttggtgtta atataaaatt acttggcaat gcttgacatt tgaaataaac atttttctat 3960
   tgttttattg caagtggtcc aattaatttt gcttagctac agtttggtca taaatcaagt 4020
   gagtttaaag acactaccaa gttgttaggt gcccagagaa aatttctccc ttttaaaaag 4080
   gccaggtgat ttttcaaatg taatcttgcc cccaaagtaa tatctgaata tctttttgac 4140
   atgtctaaat atatatatat ataaagaaat atttgttaac acaaaagcat ttgatctatg 4200
   tagataaatg ctaatagatt taaaaagcta atattaacaa ataccagaat acgtgaagtt 4260
   ccatttttaa agtgtttgag cttacagaag agaaacattc attttaaatg aagtaaaaaa 4320
   tgccttgaaa gtaattcttt agatagttgc ccattgatta aattccaaaa actaaatatg 4380
   tttttagctt taaaattata aaagctgtca taaactttat atattatgaa ttttaaaata 4440
   tgtttgagtc tcctgcaata tagtttcatc ccattgacat caattaaaaa taaccctaat 4500
   atattatttt tatatttatt cctcaggtgg aatggctatt ttaatatgcc cagtgtggat 4560
   aaaatgtcac atttctgtaa cttttgacta aagagcctat atttatctag ttaatgaatt 4620
   taaaggatct atctttccct tcataaaata cctcttattt ccattaaagc cccccaagtt 4680
   taattaattt aggattttga atgattattg acatccaata gttattttta atatttgtat 4740
   tcttgttatt tctggaagaa agcctttgtg tagcacttgg tattttgcaa agtgctttta 4800
   aaacattctt acttaccgta tttcatagaa gggaaggaaa aatgtaaggt ttaacagtaa 4860
   gcacttgcat tgaacatgga ggcatgtggt atcatgatat tcttcactaa atttagctgt 4920
   ccctaatcac agatcctaag gtaatataat ataattttag tgcatttctc ctcatcagga 4980
   atgctggagg tgcattttaa gttttaataa taagtgctag aatgaccaaa ttgcagacta 5040
   attgtttcca tattgtactt aaaatgagtt tttaaaagtg aaaaagaaat gactatatac 5100
   aatcaatgct atttattgta cctctgggcc tactcttcta aaaattgtag cttatcgatt 5160
   tttctctgtc aagcttgaac taatgtaaat aattgaaata atgtaaagtt atattttcat 5220
   gtttttatag atacaacatg acaagaatac ataatgtaag agtatttcaa ctatggataa 5280
   tgttgattgg ataatgcaca tctcagttac aagcagtact catagtttaa tatccatgta 5340
   acggtgcatc aatatattgc tatataaata tgtctgtgtg catataagtg aaaagtggtc 5400
   aaacaagagt gatgacagct gtctaaaggt ttttttattc attttatata aaaactgtta 5460
   tggaaagacc aaaatgttta tgaactattc ttatgtaaat ttacaattgt cctttactgt 5520
   acttttttgt ttacagtata gtaccttatt ttctgctgtg ttaagtgggt gtcaaactcc 5580
   aagaagacat acactttcta taacttctat tgaagatatt ggaatttcca atttttcatg 5640
   tgtactatgt cagaaaatgc tttcgatttt atttttaaat ctaacatcgg atggcttttc 5700
   cggagtgttg taaaaacttc aatcatacat aaaacatgtt cttacaaaag gcaaa 5755
<210> 34
   <211> 5755
   <212> DNA
   <213> Homo sapiens
<400> 34
   gagtcgcgca cgcgcgcccg ggactgcctg cccctctctg tgacttgcct gtgtgtgtgc 60
   gtgtgtgtat gtgtgtgtgt gtgtgtgtgt gcgcgcgcgc gtgagtgaga gaggagagag 120
   ggagaagaga gcgcgagaga gggtgagtgt gtgtgagtgc atgggagggt gctgaatatt 180
   ccgagacact gggaccacag cggcagctcc gctgaaaact gcattcagcc agtcctccgg 240
   acttctggag cggggacagg gcgcagggca tcagcagcca ccagcaggac ctgggaaata 300
   gggattcttc tgcctccact tcaggtttta gcagcttggt gctaaattgc tgtctcaaaa 360
   tgcagaggat ctaatttgca gaggaaaaca gccaaagaag gaagaggagg aaaaggaaaa 420
   aaaaaggggt atattgtgga tgctctactt ttcttggaaa tgcaaaagat tatgcatatt 480
   tctgtcctcc tttctcctgt tttatgggga ctgatttttg gtgtctcttc taacagcata 540
   cagatagggg ggctatttcc taggggcgcc gatcaagaat acagtgcatt tcgagtaggg 600
   atggttcagt tttccacttc ggagttcaga ctgacacccc acatcgacaa tttggaggtg
    660
   gcaaacagct tcgcagtcac taatgctttc tgctcccagt tttcgagagg agtctatgct
    720
   atttttggat tttatgacaa gaagtctgta aataccatca catcattttg cggaacactc 780
   cacgtctcct tcatcactcc cagcttccca acagatggca cacatccatt tgtcattcag 840
   atgagacccg acctcaaagg agctctcctt agcttgattg aatactatca atgggacaag 900
   tttgcatacc tctatgacag tgacagaggc ttatcaacac tgcaagctgt gctggattct 960
   gctgctgaaa agaaatggca agtgactgct atcaatgtgg gaaacattaa caatgacaag 1020
   aaagatgaga tgtaccgatc actttttcaa gatctggagt taaaaaagga acggcgtgta 1080
   attctggact gtgaaaggga taaagtaaac gacattgtag accaggttat taccattgga 1140
   aaacatgtta aagggtacca ctacatcatt gcaaatctgg gatttactga tggagaccta 1200
   ttaaaaatcc agtttggagg tgcaaatgtc tctggatttc agatagtgga ctatgatgat 1260
   tcgttggtat ctaaatttat agaaagatgg tcaacactgg aagaaaaaga ataccctgga 1320
   gctcacacaa caacaattaa gtatacttct gctctgacct atgatgccgt tcaagtgatg 1380
   actgaagcct tccgcaacct aaggaagcaa agaattgaaa tctcccgaag ggggaatgca 1440
   ggagactgtc tggcaaaccc agcagtgccc tggggacaag gtgtagaaat agaaagggcc 1500
   ctcaaacagg ttcaggttga aggtctctca ggaaatataa agtttgacca gaatggaaaa 1560
   agaataaact atacaattaa catcatggag ctcaaaacta atgggccccg gaagattggc 1620
   tactggagtg aagtggacaa aatggttgtt acccttactg agctcccttc tggaaatgac 1680
   acctctgggc ttgagaataa gactgttgtt gtcaccacaa ttttggaatc tccgtatgtt 1740
   atgatgaaga aaaatcatga aatgcttgaa ggcaatgagc gctatgaggg ctactgtgtt
   1800
   gacctggctg cagaaatcgc caaacattgt gggttcaagt acaagttgac aattgttggt 1860
   gatggcaagt atggggccag ggatgcagac acgaaaattt ggaatgggat ggttggagaa 1920
   cttgtatatg ggaaagctga tattgcaatt gctccattaa ctattaccct tgtgagagaa 1980
   gaggtgattg acttctcaaa gcccttcatg agcctcggga tatctatcat gatcaagaag 2040
   cctcagaagt ccaaaccagg agtgttttcc tttcttgatc ctttagccta tgagatctgg 2100
   atgtgcattg tttttgccta cattggggtc agtgtagttt tattcctggt cagcagattt 2160
   agcccctacg agtggcacac tgaggagttt gaagatggaa gagaaacaca aagtagtgaa 2220
   tcaactaatg aatttgggat ttttaatagt ctctggtttt ccttgggtgc ctttatgcgg 2280
   caaggatgcg atatttcgcc aagatccctc tctgggcgca ttgttggagg tgtgtggtgg 2340
   ttctttaccc tgatcataat ctcctcctac acggctaact tagctgcctt cctgactgta 2400
   gagaggatgg tgtctcccat cgaaagtgct gaggatcttt ctaagcaaac agaaattgct 2460
   tatggaacat tagactctgg ctccactaaa gagtttttca ggagatctaa aattgcagtg 2520
   tttgataaaa tgtggaccta catgcggagt gcggagccct ctgtgtttgt gaggactacg 2580
   gccgaagggg tggctagagt gcggaagtcc aaagggaaat atgcctactt gttggagtcc 2640
   acgatgaacg agtacattga gcaaaggaag ccttgcgaca ccatgaaagt tggtggaaac 2700
   ctggattcca aaggctatgg catcgcaaca cctaaaggat cctcattaag aaatgcggtt 2760
   aacctcgcag tactaaaact gaatgaacaa ggcctgttgg acaaattgaa aaacaaatgg 2820
   tggtacgaca aaggagagtg cggcagcggg ggaggtgatt ccaaggaaaa gaccagtgcc 2880
   ctcagtctga gcaacgttgc tggagtattc tacatccttg tcgggggcct tggtttggca 2940
   atgctggtgg ctttgattga gttctgttac aagtcaaggg ccgaggcgaa acgaatgaag 3000
   gtggcaaaga atgcacagaa tattaaccca tcttcctcgc agaattcaca gaattttgca 3060
   acttataagg aaggttacaa cgtatatggc atcgaaagtg ttaaaattta ggggatgacc 3120
   ttgaatgatg ccatgaggaa caaggcaagg ctgtcaatta caggaagtac tggagaaaat 3180
   ggacgtgtta tgactccaga atttcccaaa gcagtgcatg ctgtccctta cgtgagtcct 3240
   ggcatgggaa tgaatgtcag tgtgactgat ctctcgtgat tgataagaac cttttgagtg 3300
   ccttacacaa tggttttctt gtgtgtttat tgtcaaagtg gtgagaggca tccagtatct 3360
   tgaagacttt tctttcagcc aagaattctt aaatatgtgg agttcatctt gaattgtaag 3420
   gaatgattaa ttaaaacaca acatcttttt ctactcgagt tacagacaaa gcgtggtgga 3480
   catgcacagc taacatggaa gtactataat ttacctgaag tctttgtaca gacaacaaac 3540
   ctgtttctgc agccactatt gttagtctct tgattcataa tgacttaagc acacttgaca 3600
   tcaactgcat caagatgtga catgttttat aaaaaaagga aaaaaaacat ttaaaactaa 3660
   aaaatatttt taggtatttt cacaaacaaa ctggctttta aataaatttg cttccatatt 3720
   ggttgaataa gacaaaaaca attaaactga gtgggaagtg aataaaaaaa ggctttaggt 3780
   atcgattcca tatttttcaa agccaaatat gtaaatgcta aggaaagtaa acaaagagga 3840
   gattccaatc ttgtaattta atattgttat taaaacttta atgtatccta ttctttaaca 3900
   tttggtgtta atataaaatt acttggcaat gcttgacatt tgaaataaac atttttctat 3960
   tgttttattg caagtggtcc aattaatttt gcttagctac agtttggtca taaatcaagt 4020
   gagtttaaag acactaccaa gttgttaggt gcccagagaa aatttctccc ttttaaaaag 4080
   gccaggtgat ttttcaaatg taatcttgcc cccaaagtaa tatctgaata tctttttgac 4140
   atgtctaaat atatatatat ataaagaaat atttgttaac acaaaagcat ttgatctatg 4200
   tagataaatg ctaatagatt taaaaagcta atattaacaa ataccagaat acgtgaagtt 4260
   ccatttttaa agtgtttgag cttacagaag agaaacattc attttaaatg aagtaaaaaa 4320
   tgccttgaaa gtaattcttt agatagttgc ccattgatta aattccaaaa actaaatatg 4380
   tttttagctt taaaattata aaagctgtca taaactttat atattatgaa ttttaaaata 4440
   tgtttgagtc tcctgcaata tagtttcatc ccattgacat caattaaaaa taaccctaat 4500
   atattatttt tatatttatt cctcaggtgg aatggctatt ttaatatgcc cagtgtggat 4560
   aaaatgtcac atttctgtaa cttttgacta aagagcctat atttatctag ttaatgaatt 4620
   taaaggatct atctttccct tcataaaata cctcttattt ccattaaagc cccccaagtt 4680
   taattaattt aggattttga atgattattg acatccaata gttattttta atatttgtat 4740
   tcttgttatt tctggaagaa agcctttgtg tagcacttgg tattttgcaa agtgctttta 4800
   aaacattctt acttaccgta tttcatagaa gggaaggaaa aatgtaaggt ttaacagtaa 4860
   gcacttgcat tgaacatgga ggcatgtggt atcatgatat tcttcactaa atttagctgt 4920
   ccctaatcac agatcctaag gtaatataat ataattttag tgcatttctc ctcatcagga 4980
   atgctggagg tgcattttaa gttttaataa taagtgctag aatgaccaaa ttgcagacta 5040
   attgtttcca tattgtactt aaaatgagtt tttaaaagtg aaaaagaaat gactatatac 5100
   aatcaatgct atttattgta cctctgggcc tactcttcta aaaattgtag cttatcgatt 5160
   tttctctgtc aagcttgaac taatgtaaat aattgaaata atgtaaagtt atattttcat 5220
   gtttttatag atacaacatg acaagaatac ataatgtaag agtatttcaa ctatggataa
   5280
   tgttgattgg ataatgcaca tctcagttac aagcagtact catagtttaa tatccatgta
   5340
   acggtgcatc aatatattgc tatataaata tgtctgtgtg catataagtg aaaagtggtc
   5400
   aaacaagagt gatgacagct gtctaaaggt ttttttattc attttatata aaaactgtta
   5460
   tggaaagacc aaaatgttta tgaactattc ttatgtaaat ttacaattgt cctttactgt
   5520
   acttttttgt ttacagtata gtaccttatt ttctgctgtg ttaagtgggt gtcaaactcc
   5580
   aagaagacat acactttcta taacttctat tgaagatatt ggaatttcca atttttcatg
   5640
   tgtactatgt cagaaaatgc tttcgatttt atttttaaat ctaacatcgg atggcttttc
   5700
   cggagtgttg taaaaacttc aatcatacat aaaacatgtt cttacaaaag gcaaa
   5755
<210> 35
   <211> 5266
   <212> DNA
   <213> Homo sapiens
<400> 35
   gtgtgtgcgc gcgcgcgtga gtgagagagg agagagggag aagagagcgc gagagagggg
   60
   gggctatttc ctaggggcgc cgatcaagaa tacagtgcat ttcgagtagg gatggttcag
   120
   ttttccactt cggagttcag actgacaccc cacatcgaca atttggaggt ggcaaacagc
   180
   ttcgcagtca ctaatgcttt ctgctcccag ttttcgagag gagtctatgc tatttttgga
   240
   ttttatgaca agaagtctgt aaataccatc acatcatttt gcggaacact ccacgtctcc
   300
   ttcatcactc ccagcttccc aacagatggc acacatccat ttgtcattca gatgagaccc
   360
   gacctcaaag gagctctcct tagcttgatt gaatactatc aatgggacaa gtttgcatac
   420
   ctctatgaca gtgacagagg cttatcaaca ctgcaagctg tgctggattc tgctgctgaa
   480
   aagaaatggc aagtgactgc tatcaatgtg ggaaacatta acaatgacaa gaaagatgag
   540
   atgtaccgat cactttttca agatctggag ttaaaaaagg aacggcgtgt aattctggac 600
   tgtgaaaggg ataaagtaaa cgacattgta gaccaggtta ttaccattgg aaaacatgtt 660
   aaagggtacc actacatcat tgcaaatctg ggatttactg atggagacct attaaaaatc 720
   cagtttggag gtgcaaatgt ctctggattt cagatagtgg actatgatga ttcgttggta 780
   tctaaattta tagaaagatg gtcaacactg gaagaaaaag aataccctgg agctcacaca 840
   acaacaatta agtatacttc tgctctgacc tatgatgccg ttcaagtgat gactgaagcc 900
   ttccgcaacc taaggaagca aagaattgaa atctcccgaa gggggaatgc aggagactgt 960
   ctggcaaacc cagcagtgcc ctggggacaa ggtgtagaaa tagaaagggc cctcaaacag 1020
   gttcaggttg aaggtctctc aggaaatata aagtttgacc agaatggaaa aagaataaac 1080
   tatacaatta acatcatgga gctcaaaact aatgggcccc ggaagattgg ctactggagt 1140
   gaagtggaca aaatggttgt tacccttact gagctccctt ctggaaatga cacctctggg 1200
   cttgagaata agactgttgt tgtcaccaca attttggaat ctccgtatgt tatgatgaag 1260
   aaaaatcatg aaatgcttga aggcaatgag cgctatgagg gctactgtgt tgacctggct 1320
   gcagaaatcg ccaaacattg tgggttcaag tacaagttga caattgttgg tgatggcaag 1380
   tatggggcca gggatgcaga cacgaaaatt tggaatggga tggttggaga acttgtatat 1440
   gggaaagctg atattgcaat tgctccatta actattaccc ttgtgagaga agaggtgatt 1500
   gacttctcaa agcccttcat gagcctcggg atatctatca tgatcaagaa gcctcagaag 1560
   tccaaaccag gagtgttttc ctttcttgat cctttagcct atgagatctg gatgtgcatt 1620
   gtttttgcct acattggggt cagtgtagtt ttattcctgg tcagcagatt tagcccctac 1680
   gagtggcaca ctgaggagtt tgaagatgga agagaaacac aaagtagtga atcaactaat 1740
   gaatttggga tttttaatag tctctggttt tccttgggtg cctttatgcg gcaaggatgc 1800
   gatatttcgc caagatccct ctctgggcgc attgttggag gtgtgtggtg gttctttacc 1860
   ctgatcataa tctcctccta cacggctaac ttagctgcct tcctgactgt agagaggatg 1920
   gtgtctccca tcgaaagtgc tgaggatctt tctaagcaaa cagaaattgc ttatggaaca 1980
   ttagactctg gctccactaa agagtttttc aggagatcta aaattgcagt gtttgataaa 2040
   atgtggacct acatgcggag tgcggagccc tctgtgtttg tgaggactac ggccgaaggg 2100
   gtggctagag tgcggaagtc caaagggaaa tatgcctact tgttggagtc cacgatgaac 2160
   gagtacattg agcaaaggaa gccttgcgac accatgaaag ttggtggaaa cctggattcc 2220
   aaaggctatg gcatcgcaac acctaaagga tcctcattaa gaaccccagt aaatcttgca 2280
   gtattgaaac tcagtgagca aggcgtctta gacaagctga aaaacaaatg gtggtacgat 2340
   aaaggtgaat gtggagccaa ggactctgga agtaaggaaa agaccagtgc cctcagtctg 2400
   agcaacgttg ctggagtatt ctacatcctt gtcgggggcc ttggtttggc aatgctggtg 2460
   gctttgattg agttctgtta caagtcaagg gccgaggcga aacgaatgaa ggtggcaaag 2520
   aatgcacaga atattaaccc atcttcctcg cagaattcac agaattttgc aacttataag 2580
   gaaggttaca acgtatatgg catcgaaagt gttaaaattt aggggatgac cttgaatgat 2640
   gccatgagga acaaggcaag gctgtcaatt acaggaagta ctggagaaaa tggacgtgtt 2700
   atgactccag aatttcccaa agcagtgcat gctgtccctt acgtgagtcc tggcatggga 2760
   atgaatgtca gtgtgactga tctctcgtga ttgataagaa ccttttgagt gccttacaca 2820
   atggttttct tgtgtgttta ttgtcaaagt ggtgagaggc atccagtatc ttgaagactt 2880
   ttctttcagc caagaattct taaatatgtg gagttcatct tgaattgtaa ggaatgatta 2940
   attaaaacac aacatctttt tctactcgag ttacagacaa agcgtggtgg acatgcacag 3000
   ctaacatgga agtactataa tttacctgaa gtctttgtac agacaacaaa cctgtttctg 3060
   cagccactat tgttagtctc ttgattcata atgacttaag cacacttgac atcaactgca 3120
   tcaagatgtg acatgtttta taaaaaaagg aaaaaaaaca tttaaaacta aaaaatattt 3180
   ttaggtattt tcacaaacaa actggctttt aaataaattt gcttccatat tggttgaata 3240
   agacaaaaac aattaaactg agtgggaagt gaataaaaaa aggctttagg tatcgattcc 3300
   atatttttca aagccaaata tgtaaatgct aaggaaagta aacaaagagg agattccaat 3360
   cttgtaattt aatattgtta ttaaaacttt aatgtatcct attctttaac atttggtgtt 3420
   aatataaaat tacttggcaa tgcttgacat ttgaaataaa catttttcta ttgttttatt 3480
   gcaagtggtc caattaattt tgcttagcta cagtttggtc ataaatcaag tgagtttaaa 3540
   gacactacca agttgttagg tgcccagaga aaatttctcc cttttaaaaa ggccaggtga 3600
   tttttcaaat gtaatcttgc ccccaaagta atatctgaat atctttttga catgtctaaa 3660
   tatatatata tataaagaaa tatttgttaa cacaaaagca tttgatctat gtagataaat 3720
   gctaatagat ttaaaaagct aatattaaca aataccagaa tacgtgaagt tccattttta 3780
   aagtgtttga gcttacagaa gagaaacatt cattttaaat gaagtaaaaa atgccttgaa 3840
   agtaattctt tagatagttg cccattgatt aaattccaaa aactaaatat gtttttagct 3900
   ttaaaattat aaaagctgtc ataaacttta tatattatga attttaaaat atgtttgagt 3960
   ctcctgcaat atagtttcat cccattgaca tcaattaaaa ataaccctaa tatattattt
   4020
   ttatatttat tcctcaggtg gaatggctat tttaatatgc ccagtgtgga taaaatgtca
   4080
   catttctgta acttttgact aaagagccta tatttatcta gttaatgaat ttaaaggatc
   4140
   tatctttccc ttcataaaat acctcttatt tccattaaag ccccccaagt ttaattaatt
   4200
   taggattttg aatgattatt gacatccaat agttattttt aatatttgta ttcttgttat
   4260
   ttctggaaga aagcctttgt gtagcacttg gtattttgca aagtgctttt aaaacattct
   4320
   tacttaccgt atttcataga agggaaggaa aaatgtaagg tttaacagta agcacttgca
   4380
   ttgaacatgg aggcatgtgg tatcatgata ttcttcacta aatttagctg tccctaatca
   4440
   cagatcctaa ggtaatataa tataatttta gtgcatttct cctcatcagg aatgctggag
   4500
   gtgcatttta agttttaata ataagtgcta gaatgaccaa attgcagact aattgtttcc
   4560
   atattgtact taaaatgagt ttttaaaagt gaaaaagaaa tgactatata caatcaatgc
   4620
   tatttattgt acctctgggc ctactcttct aaaaattgta gcttatcgat ttttctctgt
   4680
   caagcttgaa ctaatgtaaa taattgaaat aatgtaaagt tatattttca tgtttttata
   4740
   gatacaacat gacaagaata cataatgtaa gagtatttca actatggata atgttgattg
   4800
   gataatgcac atctcagtta caagcagtac tcatagttta atatccatgt aacggtgcat
   4860
   caatatattg ctatataaat atgtctgtgt gcatataagt gaaaagtggt caaacaagag
   4920
   tgatgacagc tgtctaaagg tttttttatt cattttatat aaaaactgtt atggaaagac
   4980
   caaaatgttt atgaactatt cttatgtaaa tttacaattg tcctttactg tacttttttg
   5040
   tttacagtat agtaccttat tttctgctgt gttaagtggg tgtcaaactc caagaagaca
   5100
   tacactttct ataacttcta ttgaagatat tggaatttcc aatttttcat gtgtactatg
   5160
   tcagaaaatg ctttcgattt tatttttaaa tctaacatcg gatggctttt ccggagtgtt 5220
   gtaaaaactt caatcataca taaaacatgt tcttacaaaa ggcaaa 5266
<210> 36
   <211> 5195
   <212> DNA
   <213> Homo sapiens
<400> 36
   agagatcctg ggagcgagag ggagagagag ggagcaagaa aggaagagag agcgagcgag 60
   agagagcgag cgaataagag agagagtaag agggagagag aagaagagga agaagaggag 120
   gcggcggcag cggaggagga ggaggactag tgtggggtgg aaaggaagag tgagcgagag 180
   caagttaagg ggagggggtg taagagccag cgaattcttt ttctttttct attattattt 240
   tgacgactcc tgagttgcgc ccatgctctt gtcagcttcg ttttaggcgt agcatggcca 300
   ggcagaagaa aatggggcaa agcgtgctcc gggcggtctt ctttttagtc ctggggcttt 360
   tgggtcattc tcacggagga ttccccaaca ccatcagcat aggtggactt ttcatgagaa 420
   acacagtgca ggagcacagc gctttccgct ttgccgtgca gttatacaac accaaccaga 480
   acaccaccga gaagcccttc catttgaatt accacgtaga tcacttggat tcctccaata 540
   gtttttccgt gacaaatgct ttctgctccc agttctcgag aggggtgtat gccatctttg 600
   gattctatga ccagatgtca atgaacaccc tgacctcctt ctgtggggcc ctgcacacat 660
   cctttgttac gcctagcttc cccactgacg cagatgtgca gtttgtcatc cagatgcgcc 720
   cagccttgaa gggcgctatt ctgagtcttc tgggtcatta caagtgggag aagtttgtgt 780
   acctctatga cacagaacga ggattttcca tcctccaagc gattatggaa gcagcagtgc 840
   aaaacaactg gcaagtaaca gcaaggtctg tgggaaacat aaaggacgtc caagaattca 900
   ggcgcatcat tgaagaaatg gacaggaggc aggaaaagcg atacttgatt gactgcgaag 960
   tcgaaaggat taacacaatt ttggaacagg ttgtgatcct agggaaacac tcaagaggtt 1020
   atcactacat gctcgctaac ctgggtttta ctgatatttt actggaaaga gtcatgcatg 1080
   ggggagccaa cattacaggt ttccagattg tcaacaatga aaaccctatg gttcagcagt 1140
   tcatacagcg ctgggtgagg ctggatgaaa gggaattccc tgaagccaag aatgcaccac 1200
   taaagtatac atctgcattg acacacgacg caatactggt catagcagaa gctttccgct 1260
   acctgaggag gcagcgagta gatgtgtccc ggagaggaag tgctggagac tgcttagcaa 1320
   atcctgctgt gccctggagt caaggaattg atattgagag agctctgaaa atggtgcaag 1380
   tacaaggaat gactggaaat attcaatttg acacttatgg acgtaggaca aattatacca 1440
   tcgatgtgta tgaaatgaaa gtcagtggct ctcgaaaagc tggctactgg aatgagtatg 1500
   aaaggtttgt gcctttctca gatcagcaaa tcagcaatga cagtgcatcc tcagagaatc 1560
   ggaccatagt agtgactacc attctggaat caccatatgt aatgtacaag aagaaccatg 1620
   agcaactgga aggaaatgaa cgatatgaag gctattgtgt agacctagcc tatgaaatag 1680
   ccaaacatgt aaggatcaaa tacaaattgt ccatcgttgg tgacgggaaa tatggtgcaa 1740
   gggatccaga gactaaaata tggaacggca tggttgggga acttgtctat gggagagctg 1800
   atatagctgt tgctccactc actataacat tggtccgtga agaagtcata gatttttcaa 1860
   agccattcat gagcctgggc atctccatca tgataaagaa gcctcagaaa tcaaaaccag 1920
   gcgtattctc atttctggat cccctggctt atgaaatctg gatgtgcatt gtctttgctt 1980
   acattggagt cagcgtagtt cttttcctag tcagcaggtt cagtccttat gaatggcact 2040
   tggaagacaa caatgaagaa cctcgtgacc cacaaagtcc tcctgatcct ccaaatgaat 2100
   ttggaatatt taacagtctt tggttttcct tgggtgcctt tatgcagcaa ggatgtgata 2160
   tttctccaag atcactctcc gggcgcattg ttggaggggt ttggtggttc ttcaccctga 2220
   tcataatttc ttcctatact gccaatctcg ctgctttcct gactgtggag aggatggttt 2280
   ctcccataga gagtgctgaa gacttagcta aacagactga aattgcatat gggaccctgg 2340
   actccggttc aacaaaagaa tttttcagaa gatccaaaat tgctgtgtac gagaaaatgt 2400
   ggtcttacat gaaatcagcg gagccatctg tgtttaccaa aacaacagca gacggagtgg 2460
   cccgagtgcg aaagtccaag ggaaagttcg ccttcctgct ggagtcaacc atgaatgagt 2520
   acattgagca gagaaaacca tgtgatacga tgaaagttgg tggaaatctg gattccaaag 2580
   gctatggtgt ggcaacccct aaaggctcag cattaggaac gcctgtaaac cttgcagtat 2640
   tgaaactcag tgaacaaggc atcttagaca agctgaaaaa caaatggtgg tacgataagg 2700
   gggaatgtgg agccaaggac tccgggagta aggacaagac cagcgctctg agcctgagca 2760
   atgtggcagg cgttttctat atacttgtcg gaggtctggg gctggccatg atggtggctt 2820
   tgatagaatt ctgttacaaa tcacgggcag agtccaaacg catgaaactc acaaagaaca 2880
   cccaaaactt taagcctgct cctgccacca acactcagaa ttatgctaca tacagagaag 2940
   gctacaacgt gtatggaaca gagagtgtta agatctaggg atcccttccc actggaggca 3000
   tgtgatgaga ggaaatcacc gaaaacgtgg ctgcttcaag gatcctgagc cagatttcac 3060
   tctccttggt gtcgggcatg acacgaatat tgctgatggt gcaatgacct ttcaatagga 3120
   aaaactgatt tttttttcct tcagtgcctt atggaacact ctgagactcg cgacaatgca 3180
   aaccatcatt gaaatctttt tgctttgctt gaaaaaaaat aattaaaata aaaaccaaca
   3240
   aaaatggaca tgcaagattc cagtatgcga aaaaaaatct tattaagtca attcaacaaa 3300
   agccattctt tgataccact gcagagtata taaacaccat gttctttaat acacacacac 3360
   acacacacac acacacacac acacatttaa attccaattc agcaaagagg cccatctaag 3420
   ctaaaaaaat taattcttcc tgattaaaaa gaaaaaatct gtctcccagt gtttgggaag 3480
   acggactggc atttcttcta ggatctgctg accagatgtt tttggtattt cctgttggtg 3540
   gtgatgttct gtgcactcta tttcctttca atgttgctga aatgtgtata tctttagaat 3600
   gtaaatgcaa cacttaagaa aattcaaaca ctttggaaaa gggactaaac agtgatttct 3660
   ctgtgttctt gaaatggttt tgtgaaaatg ctttgataac ttcccactca aagaagagat 3720
   ttacagagct ttcgaaattg actttgtgtg tagcaaggga cggggcacta tcaggatacc 3780
   tcttggtgct ttcctaaaat ggatcccggg gctttccaag gagcctggaa tttcagctca 3840
   cagatctgtt tttcttgctt cagtgtgcat tttaagtcaa tagagctgag tatctagcat 3900
   tgaggtgagg gaaatgctgc ctatactccc agatgtgttt agaatatctc agaaacaaca 3960
   ctgtgtttag ctcggctttc tctgctaagt atgcctttca agtgtacacc acggagacag 4020
   gaccgcgttg caaggcggga cagcaggttc agaccacagt tctcagtctg actttactct 4080
   tgctaggtct gtcctactag ctgttgcctg ctaccgccca tggctctcca tcggactgca 4140
   tgtgtccttt tctagtttgc aaagactaaa atgcattccc aaacctactg ctaatctgag 4200
   ggcctcagca tcacttccag atccttgctt ggagcagtct ctctattgac tctctcagat 4260
   cgctccactg ctccatgggc tatcaagtaa ctaactgcat acctgccgtt ggcatcatca 4320
   gaacagtccg aagaaatagt ctccactcac taattacctc ctatataacg acgtatgctt 4380
   cctgtagttc agtagtttgc tctcatcgat aacgtgcatt gggaagtttc cagactgcaa 4440
   aaactaggag ctcgcattca tttcccaagt gtgaccctta gatgcttagt tgactcgctg 4500
   catatttgct cttgtcttca gaaaagaaag gaagaagtat cgttccaacg aaatgtttcc 4560
   agaaaagtgt actataaact ttcattccaa aaatggtgtc ataagcaaac aactcacttg 4620
   tcaaatttca aatggtattg aacaaaaaaa gaaagctgtt gtgtttttgt tttgttttgt 4680
   tttcatgaaa ctgtgatttt caacttatga atgctataat gtcccagcgc gggaagctca 4740
   cgctgtgtga acatgaagtt gtataaaaca aaccaaccaa cctacacaca aatgttttca 4800
   taggcactgt ataaagaaaa atgtatgttt attaactcaa atcagttttt cagagaggaa 4860
   acgtcactga gatgaagagg cgggtaaatt ggtttgttat tttttaaaaa aaacttgcat 4920
   gtttaaaaaa aagttgattg cttcaaattt ctgctactaa cttcaagcta tgggagtttg 4980
   gcagtagtca cttgaggatt ttttttccaa ttcttttctt tttgttgtta aagctgtact 5040
   tcagtgaaca gaaaaattgc caagcaaact aatggctata aaagcgtaat ttgcatgtgt 5100
   gggcataaac tacagagcct cattgccatg aggtattgta caaagtttta atacattttg 5160
   taaataaaat tgtaaagaaa gaaaaaaaaa aaaaa 5195
<210> 37
   <211> 5195
   <212> DNA
   <213> Homo sapiens
<400> 37
   agagatcctg ggagcgagag ggagagagag ggagcaagaa aggaagagag agcgagcgag 60
   agagagcgag cgaataagag agagagtaag agggagagag aagaagagga agaagaggag 120
   gcggcggcag cggaggagga ggaggactag tgtggggtgg aaaggaagag tgagcgagag 180
   caagttaagg ggagggggtg taagagccag cgaattcttt ttctttttct attattattt 240
   tgacgactcc tgagttgcgc ccatgctctt gtcagcttcg ttttaggcgt agcatggcca 300
   ggcagaagaa aatggggcaa agcgtgctcc gggcggtctt ctttttagtc ctggggcttt 360
   tgggtcattc tcacggagga ttccccaaca ccatcagcat aggtggactt ttcatgagaa 420
   acacagtgca ggagcacagc gctttccgct ttgccgtgca gttatacaac accaaccaga 480
   acaccaccga gaagcccttc catttgaatt accacgtaga tcacttggat tcctccaata 540
   gtttttccgt gacaaatgct ttctgctccc agttctcgag aggggtgtat gccatctttg 600
   gattctatga ccagatgtca atgaacaccc tgacctcctt ctgtggggcc ctgcacacat 660
   cctttgttac gcctagcttc cccactgacg cagatgtgca gtttgtcatc cagatgcgcc 720
   cagccttgaa gggcgctatt ctgagtcttc tgggtcatta caagtgggag aagtttgtgt 780
   acctctatga cacagaacga ggattttcca tcctccaagc gattatggaa gcagcagtgc 840
   aaaacaactg gcaagtaaca gcaaggtctg tgggaaacat aaaggacgtc caagaattca 900
   ggcgcatcat tgaagaaatg gacaggaggc aggaaaagcg atacttgatt gactgcgaag 960
   tcgaaaggat taacacaatt ttggaacagg ttgtgatcct agggaaacac tcaagaggtt 1020
   atcactacat gctcgctaac ctgggtttta ctgatatttt actggaaaga gtcatgcatg 1080
   ggggagccaa cattacaggt ttccagattg tcaacaatga aaaccctatg gttcagcagt 1140
   tcatacagcg ctgggtgagg ctggatgaaa gggaattccc tgaagccaag aatgcaccac 1200
   taaagtatac atctgcattg acacacgacg caatactggt catagcagaa gctttccgct 1260
   acctgaggag gcagcgagta gatgtgtccc ggagaggaag tgctggagac tgcttagcaa 1320
   atcctgctgt gccctggagt caaggaattg atattgagag agctctgaaa atggtgcaag 1380
   tacaaggaat gactggaaat attcaatttg acacttatgg acgtaggaca aattatacca 1440
   tcgatgtgta tgaaatgaaa gtcagtggct ctcgaaaagc tggctactgg aatgagtatg 1500
   aaaggtttgt gcctttctca gatcagcaaa tcagcaatga cagtgcatcc tcagagaatc 1560
   ggaccatagt agtgactacc attctggaat caccatatgt aatgtacaag aagaaccatg 1620
   agcaactgga aggaaatgaa cgatatgaag gctattgtgt agacctagcc tatgaaatag 1680
   ccaaacatgt aaggatcaaa tacaaattgt ccatcgttgg tgacgggaaa tatggtgcaa 1740
   gggatccaga gactaaaata tggaacggca tggttgggga acttgtctat gggagagctg 1800
   atatagctgt tgctccactc actataacat tggtccgtga agaagtcata gatttttcaa 1860
   agccattcat gagcctgggc atctccatca tgataaagaa gcctcagaaa tcaaaaccag 1920
   gcgtattctc atttctggat cccctggctt atgaaatctg gatgtgcatt gtctttgctt 1980
   acattggagt cagcgtagtt cttttcctag tcagcaggtt cagtccttat gaatggcact 2040
   tggaagacaa caatgaagaa cctcgtgacc cacaaagtcc tcctgatcct ccaaatgaat 2100
   ttggaatatt taacagtctt tggttttcct tgggtgcctt tatgcagcaa ggatgtgata 2160
   tttctccaag atcactctcc gggcgcattg ttggaggggt ttggtggttc ttcaccctga 2220
   tcataatttc ttcctatact gccaatctcg ctgctttcct gactgtggag aggatggttt 2280
   ctcccataga gagtgctgaa gacttagcta aacagactga aattgcatat gggaccctgg 2340
   actccggttc aacaaaagaa tttttcagaa gatccaaaat tgctgtgtac gagaaaatgt 2400
   ggtcttacat gaaatcagcg gagccatctg tgtttaccaa aacaacagca gacggagtgg 2460
   cccgagtgcg aaagtccaag ggaaagttcg ccttcctgct ggagtcaacc atgaatgagt
   2520
   acattgagca gagaaaacca tgtgatacga tgaaagttgg tggaaatctg gattccaaag
   2580
   gctatggtgt ggcaacccct aaaggctcag cattaggaaa tgctgttaac ctggcagtat
   2640
   taaaactgaa tgagcaaggc ctcttggaca aattgaaaaa caaatggtgg tacgacaaag
   2700
   gagagtgcgg cagcgggggc ggtgactcca aggacaagac cagcgctctg agcctgagca
   2760
   atgtggcagg cgttttctat atacttgtcg gaggtctggg gctggccatg atggtggctt
   2820
   tgatagaatt ctgttacaaa tcacgggcag agtccaaacg catgaaactc acaaagaaca
   2880
   cccaaaactt taagcctgct cctgccacca acactcagaa ttatgctaca tacagagaag
   2940
   gctacaacgt gtatggaaca gagagtgtta agatctaggg atcccttccc actggaggca
   3000
   tgtgatgaga ggaaatcacc gaaaacgtgg ctgcttcaag gatcctgagc cagatttcac
   3060
   tctccttggt gtcgggcatg acacgaatat tgctgatggt gcaatgacct ttcaatagga
   3120
   aaaactgatt tttttttcct tcagtgcctt atggaacact ctgagactcg cgacaatgca
   3180
   aaccatcatt gaaatctttt tgctttgctt gaaaaaaaat aattaaaata aaaaccaaca
   3240
   aaaatggaca tgcaagattc cagtatgcga aaaaaaatct tattaagtca attcaacaaa
   3300
   agccattctt tgataccact gcagagtata taaacaccat gttctttaat acacacacac
   3360
   acacacacac acacacacac acacatttaa attccaattc agcaaagagg cccatctaag
   3420
   ctaaaaaaat taattcttcc tgattaaaaa gaaaaaatct gtctcccagt gtttgggaag
   3480
   acggactggc atttcttcta ggatctgctg accagatgtt tttggtattt cctgttggtg
   3540
   gtgatgttct gtgcactcta tttcctttca atgttgctga aatgtgtata tctttagaat
   3600
   gtaaatgcaa cacttaagaa aattcaaaca ctttggaaaa gggactaaac agtgatttct
   3660
   ctgtgttctt gaaatggttt tgtgaaaatg ctttgataac ttcccactca aagaagagat
   3720
   ttacagagct ttcgaaattg actttgtgtg tagcaaggga cggggcacta tcaggatacc
   3780
   tcttggtgct ttcctaaaat ggatcccggg gctttccaag gagcctggaa tttcagctca
   3840
   cagatctgtt tttcttgctt cagtgtgcat tttaagtcaa tagagctgag tatctagcat
   3900
   tgaggtgagg gaaatgctgc ctatactccc agatgtgttt agaatatctc agaaacaaca
   3960
   ctgtgtttag ctcggctttc tctgctaagt atgcctttca agtgtacacc acggagacag
   4020
   gaccgcgttg caaggcggga cagcaggttc agaccacagt tctcagtctg actttactct
   4080
   tgctaggtct gtcctactag ctgttgcctg ctaccgccca tggctctcca tcggactgca
   4140
   tgtgtccttt tctagtttgc aaagactaaa atgcattccc aaacctactg ctaatctgag
   4200
   ggcctcagca tcacttccag atccttgctt ggagcagtct ctctattgac tctctcagat
   4260
   cgctccactg ctccatgggc tatcaagtaa ctaactgcat acctgccgtt ggcatcatca
   4320
   gaacagtccg aagaaatagt ctccactcac taattacctc ctatataacg acgtatgctt
   4380
   cctgtagttc agtagtttgc tctcatcgat aacgtgcatt gggaagtttc cagactgcaa
   4440
   aaactaggag ctcgcattca tttcccaagt gtgaccctta gatgcttagt tgactcgctg
   4500
   catatttgct cttgtcttca gaaaagaaag gaagaagtat cgttccaacg aaatgtttcc
   4560
   agaaaagtgt actataaact ttcattccaa aaatggtgtc ataagcaaac aactcacttg
   4620
   tcaaatttca aatggtattg aacaaaaaaa gaaagctgtt gtgtttttgt tttgttttgt
   4680
   tttcatgaaa ctgtgatttt caacttatga atgctataat gtcccagcgc gggaagctca
   4740
   cgctgtgtga acatgaagtt gtataaaaca aaccaaccaa cctacacaca aatgttttca
   4800
   taggcactgt ataaagaaaa atgtatgttt attaactcaa atcagttttt cagagaggaa
   4860
   acgtcactga gatgaagagg cgggtaaatt ggtttgttat tttttaaaaa aaacttgcat
   4920
   gtttaaaaaa aagttgattg cttcaaattt ctgctactaa cttcaagcta tgggagtttg
   4980
   gcagtagtca cttgaggatt ttttttccaa ttcttttctt tttgttgtta aagctgtact
   5040
   tcagtgaaca gaaaaattgc caagcaaact aatggctata aaagcgtaat ttgcatgtgt
   5100
   gggcataaac tacagagcct cattgccatg aggtattgta caaagtttta atacattttg
   5160
   taaataaaat tgtaaagaaa gaaaaaaaaa aaaaa
   5195
<210> 38
   <211> 5508
   <212> DNA
   <213> Homo sapiens
<400> 38
   agtggcagaa gagggctagg ctgagaggga agccaggact gtaggagagg gaggcagccc
   60
   gtcctcctca cgaacctgca aggatgcggc aggggcctgg gggcatgggg aggtactaac
   120
   cccccggagc ccccgattgg ggcttgcaga cctggcccgt gggcggattt tctgcctagc
   180
   gcagccgaga agcagaggtg ccaggaaaac caagagaggg gcgctggggg tgcccatccc
   240
   cagagtcggt ccctctgcga accgaggaag aaaagaggag ggagtcagcg agtggtcaga
   300
   agggaaaacc tgacaccaga ctggctccgg agcgtccggg agactggggc gctccgcgcc
   360
   atcgtcttca atgcttctct gaacagcctt taggaagagt gcgagagaaa gagagagagc
   420
   gcgcgccagg gagaggagaa aagaagatga ggattatttc cagacagatt gtcttgttat
   480
   tttctggatt ttggggactc gccatgggag cctttccgag cagcgtgcaa ataggtggtc
   540
   tcttcatccg aaacacagat caggaataca ctgcttttcg attagcaatt tttcttcata
   600
   acaccagccc caatgcgtcg gaagctcctt ttaatttggt acctcatgtg gacaacattg 660
   agacagccaa cagttttgct gtaacaaacg ccttctgttc ccagtattct agaggagtat 720
   ttgccatttt tggactctat gataagaggt cggtacatac cttgacctca ttctgcagcg 780
   ccttacatat ctccctcatc acaccaagtt tccctactga gggggagagc cagtttgtgc 840
   tgcaactaag accttcgtta cgaggagcac tcttgagttt gctggatcac tacgaatgga 900
   actgttttgt cttcctgtat gacacagaca ggggatactc gatactccaa gctattatgg 960
   aaaaagcagg acaaaatggt tggcatgtca gcgctatatg tgtggaaaat tttaatgatg 1020
   tcagctatag gcaacttcta gaagaacttg acagaagaca agagaagaag tttgtaatag 1080
   actgtgagat agagagactt caaaacatat tagaacagat tgtaagtgtt ggaaagcatg 1140
   ttaaaggcta ccattatatc attgcaaact tgggattcaa ggatatttct cttgagaggt 1200
   ttatacatgg tggagccaat gttactggat tccagttggt ggattttaat acacctatgg 1260
   taatcaaact aatggatcgc tggaagaaac tagatcagag agagtatcca ggatctgaga 1320
   ctcctccaaa gtacacctct gctctgactt atgatggagt ccttgtgatg gctgaaactt 1380
   tccgaagtct taggaggcag aaaattgata tctcaaggag aggaaatgct ggggattgtc 1440
   tggcaaatcc tgctgctcca tggggccagg gaattgacat ggagaggaca ctcaaacagg 1500
   ttcgaattca agggctgaca gggaatgttc agtttgacca ctatggacgt agagtcaatt 1560
   acacaatgga tgtgtttgag ctgaaaagca caggacctag aaaggttggt tactggaatg 1620
   atatggataa gttagtcttg attcaagatg taccaactct tggcaatgac acagctgcta 1680
   ttgagaacag aacagtggtt gtaaccacaa ttatggaatc cccatatgtt atgtacaaga 1740
   aaaatcatga aatgtttgaa ggaaatgaca agtatgaagg atactgtgta gatttggcat
   1800
   ctgaaattgc aaaacatatt ggtatcaagt ataaaattgc cattgtccct gatggaaaat
   1860
   atggagcaag ggatgcagac acaaaaatct ggaatgggat ggtaggagaa cttgtttatg
   1920
   ggaaagcaga gattgctatt gcccctctga caatcacttt ggtacgagag gaggtcattg
   1980
   acttttctaa gcccttcatg agtttgggca tatctatcat gatcaaaaag cctcagaaat
   2040
   ccaaaccagg agtgttttcc ttcttggatc ctctggccta tgagatttgg atgtgcatag
   2100
   tctttgccta cattggtgtc agcgtggtct tattcctagt tagtagattt agtccatatg
   2160
   agtggcacac agaagagcca gaggacggaa aggaaggacc cagcgaccag cctcccaatg
   2220
   agtttggcat ctttaacagc ctctggtttt ccctgggtgc ttttatgcag caaggatgtg
   2280
   acatttcacc cagatccctc tcaggtcgaa ttgttggagg tgtttggtgg ttctttacac
   2340
   tcatcattat atcatcttat actgctaacc tcgctgcttt cctgacggtt gagcgaatgg
   2400
   tctctcccat agaaagtgca gaagacctgg ccaaacaaac agaaattgcc tatggaacac
   2460
   tggattcagg atcaacaaaa gaattcttca gaagatcaaa aatagcagtg tatgaaaaga
   2520
   tgtggaccta catgcgatca gcagagccat cagtattcac taggactaca gctgagggag
   2580
   tagctcgtgt ccgcaaatcc aagggcaaat ttgcctttct cctggagtcc actatgaatg
   2640
   aatacattga gcagcgaaag ccatgtgaca cgatgaaagt gggaggaaat ctggattcca
   2700
   aaggctatgg agtagcaacg cccaagggtt cctcattagg aactcctgta aaccttgccg
   2760
   ttttgaaact cagtgaggca ggcgtcttag acaagctgaa aaacaaatgg tggtacgata
   2820
   aaggtgaatg tggacccaag gactctggaa gcaaggacaa gacgagtgcc ttgagcctga
   2880
   gcaatgtagc aggcgtcttc tacattctgg ttggcggctt gggcttggca atgctggtgg
   2940
   ctttgataga gttctgttac aagtccaggg cagaagcgaa gagaatgaag ctgacctttt 3000
   ctgaagccat aagaaacaaa gccagattat ccatcactgg gagtgtggga gagaatggcc 3060
   gcgtcttgac gcctgactgc ccaaaggctg tacacactgg aactgcaatc agacaaagtt 3120
   caggattggc tgtcattgca tcggacctac cataaaaacc aaaaaaataa ttgagtgcct 3180
   taattaaact gttggtgact ggtggaaacg cagccctgag ggacacgcca cgcgcgggtc 3240
   tttgctaaac caatcctttg gctgagagcg ggaagtccgt cctaacgcgc tggccggaca 3300
   tcagcagcag caacgtgtgc atgagctcag ctcggaaacc caaactcaga ttttatatca 3360
   ggaaaactca caattgaggt ttttttcggg gagtgggtgg gggagggatc tgggatgggt 3420
   gtattaacag caacaaattt cattcgagtg gactcaaaaa ctaatcagac ttatgagtta 3480
   gcgcattaaa ctgtgaagtt cttgctcaga aaggcctttg tcttcaccgg aaaggataaa 3540
   atagttgtag aagtccgtga acatgctaac ctgtgtctcc agaacatcca tatagtccat 3600
   ggaagaaaat ccagctgaga aaacaaatca ctaaactgtg ataagaaaat aatgaacaaa 3660
   catgtaaaac ctgtgggaaa aaaaaataaa ggaagtatgt acacttactt tggagaaaac 3720
   aaatactgaa acatgcttgc tttttaactg acgtaaattc agtagaggac aacacaattc 3780
   ttttttctaa ccatcttagg gaacaataca ttgcaataat tgatataaat gccatcactg 3840
   taataaactt tagagacttt tttttataaa agttgttggt catcttcttg tttgctgtaa 3900
   ccttcactat gtcacatgag tcgattcacc gattgcattt gtctcacaac caggaagaaa 3960
   agcaaaagga agaaaacgtt taggttcaat catcagtctg cggtgtagac tcgaaagaga 4020
   tgacaggtca ctcatgttaa tggtattatt tataatctca ttctgtgtac aacattgtgg 4080
   tttttgtacc caccaaaaag aataaaacag cagatgttct tacaatatct acagagctta 4140
   aaagtttttt cttatcgtta taaaagttat ttgagaaatt ataagactat aagagagatt 4200
   gtattagtgg tgggccatag tggaaaatgt agctagccct cattattttt tgcatactaa 4260
   gctacccctc cttttcagat ctttgactca ttaacagatt aaactgtcaa agatggagtc 4320
   tttgagttgg ggaatgaatc actgtcctaa caacaacata ccttgtaatt gtgtgttgaa 4380
   attttacttg actgtatttt gctgcataaa attatgtgtc tcttgggctt cttcccttat 4440
   tcctattgtt ccctttaaat catatgaagg cattcataat agcttggggt agataacaaa 4500
   tgaagaatta gtctttgttt tcaactggaa attgtaaaga aaattatact catgtttatt 4560
   tataaaaatc accttatgta tgaattaaac taacatggtt caaaagaagg tttggttcat 4620
   ttgaaataat aaataagtac tctaatacag ataaaaatca tgtacttagg gtattggcag 4680
   aaagcacaag ttaggatgat ttcagaagtc tggccttgaa ggatgagttg agttttaaca 4740
   ggaggagaag gtgttaagag ccatatgagt gagcagtggc ccaaagccat gcacatcagt 4800
   ggctcattta aggaatgaat gccattagat gggctactga gagtacaggg atattatgga 4860
   agataaagtt ggaaaagctg aaggattgat tttcttccat caactctcaa gatcccattc 4920
   gccattcaat ctctgtgctg cagtaagagc aatcttaaac agtataaatc acacacacac 4980
   acacacacac acacacacac acacacaagt ccctcaggaa aaattccaag ctcttgagaa 5040
   gatcacatga gccccttcat gacctggcgc ttgcttattt cttccaggac ttctctcact 5100
   tctatccagc tattcccgtc agcaaatgaa cctccaaagc agcacatgga gcactgcata 5160
   gactatttcc tcagtgcgta actcctccct gtctcctctt tacctgagta acttgtactc 5220
   atccttcaat actccaactg aattttactt accctgaaaa gatttccatg gctatccacc
   5280
   acccccctgc ctgtgagact gagttaggtg ccctttttca tgtctttccc ccatcacggc
   5340
   acttaccata ctgcgttgta attgcctgtg tactcgtctg tataactact agactgtaag
   5400
   ctccttgagg gcagggactg tgtctatctt gttcacagtt gtatccccag cacccagcac
   5460
   agtgcctggc atattgtagg tgcttaataa atatttgttg aatgaatg
   5508
<210> 39
   <211> 5621
   <212> DNA
   <213> Homo sapiens
<400> 39
   agtggcagaa gagggctagg ctgagaggga agccaggact gtaggagagg gaggcagccc
   60
   gtcctcctca cgaacctgca aggatgcggc aggggcctgg gggcatgggg aggtactaac
   120
   cccccggagc ccccgattgg ggcttgcaga cctggcccgt gggcggattt tctgcctagc
   180
   gcagccgaga agcagaggtg ccaggaaaac caagagaggg gcgctggggg tgcccatccc
   240
   cagagtcggt ccctctgcga accgaggaag aaaagaggag ggagtcagcg agtggtcaga
   300
   agggaaaacc tgacaccaga ctggctccgg agcgtccggg agactggggc gctccgcgcc
   360
   atcgtcttca atgcttctct gaacagcctt taggaagagt gcgagagaaa gagagagagc
   420
   gcgcgccagg gagaggagaa aagaagatga ggattatttc cagacagatt gtcttgttat
   480
   tttctggatt ttggggactc gccatgggag cctttccgag cagcgtgcaa ataggtggtc
   540
   tcttcatccg aaacacagat caggaataca ctgcttttcg attagcaatt tttcttcata
   600
   acaccagccc caatgcgtcg gaagctcctt ttaatttggt acctcatgtg gacaacattg
   660
   agacagccaa cagttttgct gtaacaaacg ccttctgttc ccagtattct agaggagtat
   720
   ttgccatttt tggactctat gataagaggt cggtacatac cttgacctca ttctgcagcg
   780
   ccttacatat ctccctcatc acaccaagtt tccctactga gggggagagc cagtttgtgc 840
   tgcaactaag accttcgtta cgaggagcac tcttgagttt gctggatcac tacgaatgga 900
   actgttttgt cttcctgtat gacacagaca ggggatactc gatactccaa gctattatgg 960
   aaaaagcagg acaaaatggt tggcatgtca gcgctatatg tgtggaaaat tttaatgatg 1020
   tcagctatag gcaacttcta gaagaacttg acagaagaca agagaagaag tttgtaatag 1080
   actgtgagat agagagactt caaaacatat tagaacagat tgtaagtgtt ggaaagcatg 1140
   ttaaaggcta ccattatatc attgcaaact tgggattcaa ggatatttct cttgagaggt 1200
   ttatacatgg tggagccaat gttactggat tccagttggt ggattttaat acacctatgg 1260
   taatcaaact aatggatcgc tggaagaaac tagatcagag agagtatcca ggatctgaga 1320
   ctcctccaaa gtacacctct gctctgactt atgatggagt ccttgtgatg gctgaaactt 1380
   tccgaagtct taggaggcag aaaattgata tctcaaggag aggaaatgct ggggattgtc 1440
   tggcaaatcc tgctgctcca tggggccagg gaattgacat ggagaggaca ctcaaacagg 1500
   ttcgaattca agggctgaca gggaatgttc agtttgacca ctatggacgt agagtcaatt 1560
   acacaatgga tgtgtttgag ctgaaaagca caggacctag aaaggttggt tactggaatg 1620
   atatggataa gttagtcttg attcaagatg taccaactct tggcaatgac acagctgcta 1680
   ttgagaacag aacagtggtt gtaaccacaa ttatggaatc cccatatgtt atgtacaaga 1740
   aaaatcatga aatgtttgaa ggaaatgaca agtatgaagg atactgtgta gatttggcat 1800
   ctgaaattgc aaaacatatt ggtatcaagt ataaaattgc cattgtccct gatggaaaat 1860
   atggagcaag ggatgcagac acaaaaatct ggaatgggat ggtaggagaa cttgtttatg 1920
   ggaaagcaga gattgctatt gcccctctga caatcacttt ggtacgagag gaggtcattg 1980
   acttttctaa gcccttcatg agtttgggca tatctatcat gatcaaaaag cctcagaaat 2040
   ccaaaccagg agtgttttcc ttcttggatc ctctggccta tgagatttgg atgtgcatag 2100
   tctttgccta cattggtgtc agcgtggtct tattcctagt tagtagattt agtccatatg 2160
   agtggcacac agaagagcca gaggacggaa aggaaggacc cagcgaccag cctcccaatg 2220
   agtttggcat ctttaacagc ctctggtttt ccctgggtgc ttttatgcag caaggatgtg 2280
   acatttcacc cagatccctc tcaggtcgaa ttgttggagg tgtttggtgg ttctttacac 2340
   tcatcattat atcatcttat actgctaacc tcgctgcttt cctgacggtt gagcgaatgg 2400
   tctctcccat agaaagtgca gaagacctgg ccaaacaaac agaaattgcc tatggaacac 2460
   tggattcagg atcaacaaaa gaattcttca gaagatcaaa aatagcagtg tatgaaaaga 2520
   tgtggaccta catgcgatca gcagagccat cagtattcac taggactaca gctgagggag 2580
   tagctcgtgt ccgcaaatcc aagggcaaat ttgcctttct cctggagtcc actatgaatg 2640
   aatacattga gcagcgaaag ccatgtgaca cgatgaaagt gggaggaaat ctggattcca 2700
   aaggctatgg agtagcaacg cccaagggtt cctcattagg aaatgctgtt aacctcgcag 2760
   ttttaaaact gaatgaacaa ggcctcttgg acaaattgaa aaacaaatgg tggtacgaca 2820
   aaggagaatg tggcagcggg ggaggtgact ccaaggacaa gacgagtgcc ttgagcctga 2880
   gcaatgtagc aggcgtcttc tacattctgg ttggcggctt gggcttggca atgctggtgg 2940
   ctttgataga gttctgttac aagtccaggg cagaagcgaa gagaatgaag gtggcaaaga 3000
   gtgcacagac ttttaaccca acttcctcgc agaataccca gaatttagca acctatagag 3060
   aaggttacaa cgtatatgga accgaaagta ttaaaattta gggctgacct tttctgaagc 3120
   cataagaaac aaagccagat tatccatcac tgggagtgtg ggagagaatg gccgcgtctt 3180
   gacgcctgac tgcccaaagg ctgtacacac tggaactgca atcagacaaa gttcaggatt 3240
   ggctgtcatt gcatcggacc taccataaaa accaaaaaaa taattgagtg ccttaattaa 3300
   actgttggtg actggtggaa acgcagccct gagggacacg ccacgcgcgg gtctttgcta 3360
   aaccaatcct ttggctgaga gcgggaagtc cgtcctaacg cgctggccgg acatcagcag 3420
   cagcaacgtg tgcatgagct cagctcggaa acccaaactc agattttata tcaggaaaac 3480
   tcacaattga ggtttttttc ggggagtggg tgggggaggg atctgggatg ggtgtattaa 3540
   cagcaacaaa tttcattcga gtggactcaa aaactaatca gacttatgag ttagcgcatt 3600
   aaactgtgaa gttcttgctc agaaaggcct ttgtcttcac cggaaaggat aaaatagttg 3660
   tagaagtccg tgaacatgct aacctgtgtc tccagaacat ccatatagtc catggaagaa 3720
   aatccagctg agaaaacaaa tcactaaact gtgataagaa aataatgaac aaacatgtaa 3780
   aacctgtggg aaaaaaaaat aaaggaagta tgtacactta ctttggagaa aacaaatact 3840
   gaaacatgct tgctttttaa ctgacgtaaa ttcagtagag gacaacacaa ttcttttttc 3900
   taaccatctt agggaacaat acattgcaat aattgatata aatgccatca ctgtaataaa 3960
   ctttagagac ttttttttat aaaagttgtt ggtcatcttc ttgtttgctg taaccttcac 4020
   tatgtcacat gagtcgattc accgattgca tttgtctcac aaccaggaag aaaagcaaaa 4080
   ggaagaaaac gtttaggttc aatcatcagt ctgcggtgta gactcgaaag agatgacagg 4140
   tcactcatgt taatggtatt atttataatc tcattctgtg tacaacattg tggtttttgt 4200
   acccaccaaa aagaataaaa cagcagatgt tcttacaata tctacagagc ttaaaagttt
   4260
   tttcttatcg ttataaaagt tatttgagaa attataagac tataagagag attgtattag
   4320
   tggtgggcca tagtggaaaa tgtagctagc cctcattatt ttttgcatac taagctaccc
   4380
   ctccttttca gatctttgac tcattaacag attaaactgt caaagatgga gtctttgagt
   4440
   tggggaatga atcactgtcc taacaacaac ataccttgta attgtgtgtt gaaattttac
   4500
   ttgactgtat tttgctgcat aaaattatgt gtctcttggg cttcttccct tattcctatt
   4560
   gttcccttta aatcatatga aggcattcat aatagcttgg ggtagataac aaatgaagaa
   4620
   ttagtctttg ttttcaactg gaaattgtaa agaaaattat actcatgttt atttataaaa
   4680
   atcaccttat gtatgaatta aactaacatg gttcaaaaga aggtttggtt catttgaaat
   4740
   aataaataag tactctaata cagataaaaa tcatgtactt agggtattgg cagaaagcac
   4800
   aagttaggat gatttcagaa gtctggcctt gaaggatgag ttgagtttta acaggaggag
   4860
   aaggtgttaa gagccatatg agtgagcagt ggcccaaagc catgcacatc agtggctcat
   4920
   ttaaggaatg aatgccatta gatgggctac tgagagtaca gggatattat ggaagataaa
   4980
   gttggaaaag ctgaaggatt gattttcttc catcaactct caagatccca ttcgccattc
   5040
   aatctctgtg ctgcagtaag agcaatctta aacagtataa atcacacaca cacacacaca
   5100
   cacacacaca cacacacaca agtccctcag gaaaaattcc aagctcttga gaagatcaca
   5160
   tgagcccctt catgacctgg cgcttgctta tttcttccag gacttctctc acttctatcc
   5220
   agctattccc gtcagcaaat gaacctccaa agcagcacat ggagcactgc atagactatt
   5280
   tcctcagtgc gtaactcctc cctgtctcct ctttacctga gtaacttgta ctcatccttc
   5340
   aatactccaa ctgaatttta cttaccctga aaagatttcc atggctatcc accacccccc
   5400
   tgcctgtgag actgagttag gtgccctttt tcatgtcttt cccccatcac ggcacttacc
   5460
   atactgcgtt gtaattgcct gtgtactcgt ctgtataact actagactgt aagctccttg
   5520
   agggcaggga ctgtgtctat cttgttcaca gttgtatccc cagcacccag cacagtgcct
   5580
   ggcatattgt aggtgcttaa taaatatttg ttgaatgaat g
   5621
<210> 40
   <211> 3345
   <212> DNA
   <213> Homo sapiens
<400> 40
   agtggcagaa gagggctagg ctgagaggga agccaggact gtaggagagg gaggcagccc
   60
   gtcctcctca cgaacctgca aggatgcggc aggggcctgg gggcatgggg aggtactaac
   120
   cccccggagc ccccgattgg ggcttgcaga cctggcccgt gggcggattt tctgcctagc
   180
   gcagccgaga agcagaggtg ccaggaaaac caagagaggg gcgctggggg tgcccatccc
   240
   cagagtcggt ccctctgcga accgaggaag aaaagaggag ggagtcagcg agtggtcaga
   300
   agggaaaacc tgacaccaga ctggctccgg agcgtccggg agactggggc gctccgcgcc
   360
   atcgtcttca atgcttctct gaacagcctt taggaagagt gcgagagaaa gagagagagc
   420
   gcgcgccagg gagaggagaa aagaagatga ggattatttc cagacagatt gtcttgttat
   480
   tttctggatt ttggggactc gccatgggag cctttccgag cagcgtgcaa ataggtggtc
   540
   tcttcatccg aaacacagat caggaataca ctgcttttcg attagcaatt tttcttcata
   600
   acaccagccc caatgcgtcg gaagctcctt ttaatttggt acctcatgtg gacaacattg
   660
   agacagccaa cagttttgct gtaacaaacg ccttctgttc ccagtattct agaggagtat
   720
   ttgccatttt tggactctat gataagaggt cggtacatac cttgacctca ttctgcagcg
   780
   ccttacatat ctccctcatc acaccaagtt tccctactga gggggagagc cagtttgtgc 840
   tgcaactaag accttcgtta cgaggagcac tcttgagttt gctggatcac tacgaatgga 900
   actgttttgt cttcctgtat gacacagaca ggggatactc gatactccaa gctattatgg 960
   aaaaagcagg acaaaatggt tggcatgtca gcgctatatg tgtggaaaat tttaatgatg 1020
   tcagctatag gcaacttcta gaagaacttg acagaagaca agagaagaag tttgtaatag 1080
   actgtgagat agagagactt caaaacatat tagaacagat tgtaagtgtt ggaaagcatg 1140
   ttaaaggcta ccattatatc attgcaaact tgggattcaa ggatatttct cttgagaggt 1200
   ttatacatgg tggagccaat gttactggat tccagttggt ggattttaat acacctatgg 1260
   taatcaaact aatggatcgc tggaagaaac tagatcagag agagtatcca ggatctgaga 1320
   ctcctccaaa gtacacctct gctctgactt atgatggagt ccttgtgatg gctgaaactt 1380
   tccgaagtct taggaggcag aaaattgata tctcaaggag aggaaatgct ggggattgtc 1440
   tggcaaatcc tgctgctcca tggggccagg gaattgacat ggagaggaca ctcaaacagg 1500
   ttcgaattca agggctgaca gggaatgttc agtttgacca ctatggacgt agagtcaatt 1560
   acacaatgga tgtgtttgag ctgaaaagca caggacctag aaaggttggt tactggaatg 1620
   atatggataa gttagtcttg attcaagatg taccaactct tggcaatgac acagctgcta 1680
   ttgagaacag aacagtggtt gtaaccacaa ttatgcctct gatgaagaat cctattttaa 1740
   gaaattgatc aagaaagaaa agagttccgc gctgttcgac cattcctaac taaggctcaa 1800
   gtcttgttct ccagtgtagt aaatttaagc ttatttttca tgtgggattc ttcttggatg 1860
   accaactctg gactaccaga aaaaaaaaat tttaagttct gtgacttttc tgagatacta 1920
   gaacaaaaga agaattaatc ttcatctttc tcaagaaata gatgttgaca aagaatcact 1980
   tagcgattct gacatatcaa ttcccctatc ttgaaatgag gtcactgtat gtaaatgatg 2040
   gaattatatc actccatttc caagggtaga ttttctataa gtaaatatct cggaatttgt 2100
   gtgcttgttt tctgaatata tacagttgtt ttctttaaag atctcttgga attttgcctg 2160
   ttctgtgtga aataaagtgt tttaatgtgc attataggta tgatatagag aatctccttt 2220
   ccatccttgt tactaaaggg actggacaaa taaatcttaa aaccaaaata ctgaattaat 2280
   tttgcaagca tggctagttt ttaggaagca tgctatcaaa aaaaaaaaga ctaaaaatga 2340
   ctgaaaaaat ccaactgttt tatatatata taaatatata tatatttata tatatatata 2400
   aaggatattc tgtaaagtta tatgttgttt gacagtaaag ccatcaatat ttttgctatc 2460
   aaaatagtat aatactagta tctttttgta tgaaaatgta atctttatat aaataatacc 2520
   tctgatattt gcaactgcat aatcgttcag taattcaaaa agacatacta gaatcctttt 2580
   tctgaaagtg ttccttcaat ttgcttttgt tgaaaacggt agtccaggac ctatgatatc 2640
   cctccacttc attcattatg aaagaaatcc cttgtagata aacaagatat tggcatctgc 2700
   atgtaattat ccccagattc agctgaaaac tcccaacaca gatggaattg gctagacatt 2760
   ttaatatatg tgatacctat atctagatat agaaggctga gagtgagcac tggatataat 2820
   tcattttgat tgaaattgat atggtgttat tgttcttcca gttgtctgtc ctttgtgtat 2880
   gttcttattt atatgttgat acactgtaac actatatgct attgctaaat aaaattgatt 2940
   gagaaattca gttattcata aatatttatt gagcgtctgc tatgtgctag gcacagttct 3000
   aggccctggg gatatgtcac agacaaaaat cctgcactca atgaaactta tagtatattg 3060
   agagaaagca gaccagaaac ataattaaga attatattag ctatctttat taaatataat
   3120
   gtagtgttag cttttatggc tgttgaaagt tattttttct tgtaacagtg ttgtatatct
   3180
   acaatgtgat tttcatttta ataatgaatt tattctacct gaatataatc atactgaata
   3240
   taccacagca aaatctaata gaaaataaaa ttaatatcat catttttatc tttaagtctt
   3300
   gttgactaaa aatgttataa aatcaataaa atttataaga ctgtg
   3345
<210> 41
   <211> 3213
   <212> DNA
   <213> Homo sapiens
<400> 41
   agccactaga cgctccacca ccatcttttg catgtgcaac atttgcagcc ggacagaaaa
   60
   cctctcccag ggctatggag actgcgggaa aaatctggcg gctcgcgatg gattgctaag
   120
   gagaactagt cataatctta aaccaccgaa acctctttcc ttttttttct ttcttttctt
   180
   tcttttcttt tttttttttt ttttttggtt gattttaatt ttagcgccat cgtcttcaat
   240
   gcttctctga acagccttta ggaagagtgc gagagaaaga gagagagcgc gcgccaggga
   300
   gaggagaaaa gaagatgagg attatttcca gacagattgt cttgttattt tctggatttt
   360
   ggggactcgc catgggagcc tttccgagca gcgtgcaaat aggtggtctc ttcatccgaa
   420
   acacagatca ggaatacact gcttttcgat tagcaatttt tcttcataac accagcccca
   480
   atgcgtcgga agctcctttt aatttggtac ctcatgtgga caacattgag acagccaaca
   540
   gttttgctgt aacaaacgcc ttctgttccc agtattctag aggagtattt gccatttttg
   600
   gactctatga taagaggtcg gtacatacct tgacctcatt ctgcagcgcc ttacatatct
   660
   ccctcatcac accaagtttc cctactgagg gggagagcca gtttgtgctg caactaagac
   720
   cttcgttacg aggagcactc ttgagtttgc tggatcacta cgaatggaac tgttttgtct
   780
   tcctgtatga cacagacagg ggatactcga tactccaagc tattatggaa aaagcaggac
   840
   aaaatggttg gcatgtcagc gctatatgtg tggaaaattt taatgatgtc agctataggc
   900
   aacttctaga agaacttgac agaagacaag agaagaagtt tgtaatagac tgtgagatag
   960
   agagacttca aaacatatta gaacagattg taagtgttgg aaagcatgtt aaaggctacc
   1020
   attatatcat tgcaaacttg ggattcaagg atatttctct tgagaggttt atacatggtg
   1080
   gagccaatgt tactggattc cagttggtgg attttaatac acctatggta atcaaactaa
   1140
   tggatcgctg gaagaaacta gatcagagag agtatccagg atctgagact cctccaaagt
   1200
   acacctctgc tctgacttat gatggagtcc ttgtgatggc tgaaactttc cgaagtctta
   1260
   ggaggcagaa aattgatatc tcaaggagag gaaatgctgg ggattgtctg gcaaatcctg
   1320
   ctgctccatg ggccagggga attgacatgg agaggacact caaacaggtt cgaattcaag
   1380
   ggctgacagg gaatgttcag tttgaccact atggacgtag agtcaattac acaatggatg
   1440
   tgtttgagct gaaaagcaca ggacctagaa aggttggtta ctggaatgat atggataagt
   1500
   tagtcttgat tcaagatgta ccaactcttg gcaatgacac agctgctatt gagaacagaa
   1560
   cagtggttgt aaccacaatt atgcctctga tgaagaatcc tattttaaga aattgatcaa
   1620
   gaaagaaaag agttccgcgc tgttcgacca ttcctaacta aggctcaagt cttgttctcc
   1680
   agtgtagtaa atttaagctt atttttcatg tgggattctt cttggatgac caactctgga
   1740
   ctaccagaaa aaaaaaattt taagttctgt gacttttctg agatactaga acaaaagaag
   1800
   aattaatctt catctttctc aagaaataga tgttgacaaa gaatcactta gcgattctga
   1860
   catatcaatt cccctatctt gaaatgaggt cactgtatgt aaatgatgga attatatcac
   1920
   tccatttcca agggtagatt ttctataagt aaatatctcg gaatttgtgt gcttgttttc
   1980
   tgaatatata cagttgtttt ctttaaagat ctcttggaat tttgcctgtt ctgtgtgaaa
   2040
   taaagtgttt taatgtgcat tataggtatg atatagagaa tctcctttcc atccttgtta
   2100
   ctaaagggac tggacaaata aatcttaaaa ccaaaatact gaattaattt tgcaagcatg
   2160
   gctagttttt aggaagcatg ctatcaaaaa aaaaaagact aaaaatgact gaaaaaatcc
   2220
   aactgtttta tatatatata aatatatata tatttatata tatatataaa ggatattctg
   2280
   taaagttata tgttgtttga cagtaaagcc atcaatattt ttgctatcaa aatagtataa
   2340
   tactagtatc tttttgtatg aaaatgtaat ctttatataa ataatacctc tgatatttgc
   2400
   aactgcataa tcgttcagta attcaaaaag acatactaga atcctttttc tgaaagtgtt
   2460
   ccttcaattt gcttttgttg aaaacggtag tccaggacct atgatatccc tccacttcat
   2520
   tcattatgaa agaaatccct tgtagataaa caagatattg gcatctgcat gtaattatcc
   2580
   ccagattcag ctgaaaactc ccaacacaga tggaattggc tagacatttt aatatatgtg
   2640
   atacctatat ctagatatag aaggctgaga gtgagcactg gatataattc attttgattg
   2700
   aaattgatat ggtgttattg ttcttccagt tgtctgtcct ttgtgtatgt tcttatttat
   2760
   atgttgatac actgtaacac tatatgctat tgctaaataa aattgattga gaaattcagt
   2820
   tattcataaa tatttattga gcgtctgcta tgtgctaggc acagttctag gccctgggga
   2880
   tatgtcacag acaaaaatcc tgcactcaat gaaacttata gtatattgag agaaagcaga
   2940
   ccagaaacat aattaagaat tatattagct atctttatta aatataatgt agtgttagct
   3000
   tttatggctg ttgaaagtta ttttttcttg taacagtgtt gtatatctac aatgtgattt
   3060
   tcattttaat aatgaattta ttctacctga atataatcat actgaatata ccacagcaaa 3120
   atctaataga aaataaaatt aatatcatca tttttatctt taagtcttgt tgactaaaaa 3180
   tgttataaaa tcaataaaat ttataagact gtg 3213
<210> 42
   <211> 7855
   <212> DNA
   <213> Homo sapiens
<400> 42
   gtttagaaga tcatgaccac atggatcatc taactaaatg gtacatgggg acaaaatggt 60
   cctttagaaa atacatctga attgctggct aatttcttga tttgcgactc aacgtaggac 120
   atcgcttgtt cgtagctatc agaaccctcc tgaattttcc ccaccatgct atctttattg 180
   gcttgaactc ctttcctaaa atggtccttc tgttgatcct gtcagtctta cttttgaaag 240
   aagatgtccg tgggagtgca cagtccagtg agaggagggt ggtggctcac atgccgggtg 300
   acatcattat tggagctctc ttttctgttc atcaccagcc tactgtggac aaagttcatg 360
   agaggaagtg tggggcggtc cgtgaacagt atggcattca gagagtggag gccatgctgc 420
   ataccctgga aaggatcaat tcagacccca cactcttgcc caacatcaca ctgggctgtg 480
   agataaggga ctcctgctgg cattcggctg tggccctaga gcagagcatt gagttcataa 540
   gagattccct catttcttca gaagaggaag aaggcttggt acgctgtgtg gatggctcct 600
   cctcttcctt ccgctccaag aagcccatag taggggtcat tgggcctggc tccagttctg 660
   tagccattca ggtccagaat ttgctccagc ttttcaacat acctcagatt gcttactcag 720
   caaccagcat ggatctgagt gacaagactc tgttcaaata tttcatgagg gttgtgcctt 780
   cagatgctca gcaggcaagg gccatggtgg acatagtgaa gaggtacaac tggacctatg 840
   tatcagccgt gcacacagaa ggcaactatg gagaaagtgg gatggaagcc ttcaaagata 900
   tgtcagcgaa ggaagggatt tgcatcgccc actcttacaa aatctacagt aatgcagggg 960
   agcagagctt tgataagctg ctgaagaagc tcacaagtca cttgcccaag gcccgggtgg 1020
   tggcctgctt ctgtgagggc atgacggtga gaggtctgct gatggccatg aggcgcctgg 1080
   gtctagcggg agaatttctg cttctgggca gtgatggctg ggctgacagg tatgatgtga 1140
   cagatggata tcagcgagaa gctgttggtg gcatcacaat caagctccaa tctcccgatg 1200
   tcaagtggtt tgatgattat tatctgaagc tccggccaga aacaaaccac cgaaaccctt 1260
   ggtttcaaga attttggcag catcgttttc agtgccgact ggaagggttt ccacaggaga 1320
   acagcaaata caacaagact tgcaatagtt ctctgactct gaaaacacat catgttcagg 1380
   attccaaaat gggatttgtg atcaacgcca tctattcgat ggcctatggg ctccacaaca 1440
   tgcagatgtc cctctgccca ggctatgcag gactctgtga tgccatgaag ccaattgatg 1500
   gacggaaact tttggagtcc ctgatgaaaa ccaattttac tggggtttct ggagatacga 1560
   tcctattcga tgagaatgga gactctccag gaaggtatga aataatgaat ttcaaggaaa 1620
   tgggaaaaga ttactttgat tatatcaacg ttggaagttg ggacaatgga gaattaaaaa 1680
   tggatgatga tgaagtatgg tccaagaaaa gcaacatcat cagatctgtg tgcagtgaac 1740
   catgtgagaa aggccagatc aaggtgatcc gaaagggaga agtcagctgt tgttggacct 1800
   gtacaccttg taaggagaat gagtatgtct ttgatgagta cacatgcaag gcatgccaac 1860
   tggggtcttg gcccactgat gatctcacag gttgtgactt gatcccagta cagtatcttc 1920
   gatggggtga ccctgaaccc attgcagctg tggtgtttgc ctgccttggc ctcctggcca 1980
   ccctgtttgt tactgtagtc ttcatcattt accgtgatac accagtagtc aagtcctcaa
   2040
   gcagggaact ctgctacatt atccttgctg gcatctgcct gggctactta tgtaccttct
   2100
   gcctcattgc gaagcccaaa cagatttact gctaccttca gagaattggc attggtctct
   2160
   ccccagccat gagctactca gcccttgtaa caaagaccaa ccgtattgca aggatcctgg
   2220
   ctggcagcaa gaagaagatc tgtaccaaaa agcccagatt catgagtgcc tgtgcccagc
   2280
   tagtgattgc tttcattctc atatgcatcc agttgggcat catcgttgcc ctctttataa
   2340
   tggagcctcc tgacataatg catgactacc caagcattcg agaagtctac ctgatctgta
   2400
   acaccaccaa cctaggagtt gtcactccac ttggatacaa tggattgttg attttgagct
   2460
   gcaccttcta tgcgttcaag accagaaatg ttccagctaa cttcaacgag gccaagtata
   2520
   tcgccttcac aatgtacacg acctgcatta tatggctagc ttttgtgcca atctactttg
   2580
   gcagcaacta caaaatcatc accatgtgtt tctcggtcag cctcagtgcc acagtggccc
   2640
   taggctgcat gtttgtgccg aaggtgtaca tcatcctggc caaaccagag agaaacgtgc
   2700
   gcagcgcctt caccacatct accgtggtgc gcatgcatgt aggggatggc aagtcatcct
   2760
   ccgcagccag cagatccagc agcctagtca acctgtggaa gagaaggggc tcctctgggg
   2820
   aaaccttaag gtacaaagac aggagactgg cccagcacaa gtcggaaata gagtgtttca
   2880
   cccccaaagg gagtatgggg aatggtggga gagcaacaat gagcagttcc aatggaaaat
   2940
   ccgtcacgtg ggcccagaat gagaagagca gccgggggca gcacctgtgg cagcgcctgt
   3000
   ccatccacat caacaagaaa gaaaacccca accaaacggc cgtcatcaag cccttcccca
   3060
   agagcacgga gagccgtggc ctgggcgctg gcgctggcgc aggcgggagc gctgggggcg
   3120
   tgggggccac gggcggtgcg ggctgcgcag gcgccggccc aggcgggccc gagtccccag
   3180
   acgccggccc caaggcgctg tatgatgtgg ccgaggctga ggagcacttc ccggcgcccg 3240
   cgcggccgcg ctcaccgtcg cccatcagca cgctgagcca ccgcgcgggc tcggccagcc 3300
   gcacggacga cgatgtgccg tcgctgcact cggagcctgt ggcgcgcagc agctcctcgc 3360
   agggctccct catggagcag atcagcagtg tggtcacccg cttcacggcc aacatcagcg 3420
   agctcaactc catgatgctg tccaccgcgg cccccagccc cggcgtcggc gccccgctct 3480
   gctcgtccta cctgatcccc aaagagatcc agttgcccac gaccatgacg acctttgccg 3540
   aaatccagcc tctgccggcc atcgaagtca cgggaggcgc gcagcccgcg gcaggggcgc 3600
   aggcggctgg ggacgcggcc cgggagagcc ccgcggccgg tcccgaggct gcggccgcca 3660
   agccagacct ggaggagctg gtggctctca ccccgccgtc ccccttcaga gactcggtgg 3720
   actcggggag cacaaccccc aactcgccag tgtccgagtc ggccctctgt atcccgtcgt 3780
   ctcccaaata tgacactctt atcataagag attacactca gagctcctcg tcgttgtgaa 3840
   tgtccctgga aagcacgccg gcctgcgcgt gcggagcgga gccccccgtg ttcacacaca 3900
   cacaatggca agcatagtcg cctggttacg gcccaggggg aagatgccaa gggcacccct 3960
   taatggaaac acgagatcag tagtgctatc tcatgacaac cgacgaagaa accgacgaca 4020
   aatcttttgg cagattttct tctagtggcc ttagaaaaca tgggctttta agaaacacgg 4080
   ctgatatctt tgagggctga caaggcgtct cttcaaacag ttccatacca agtgctttgc 4140
   tctagggaag cagtgcgtgt gaaacagcgt aacggagggt gaagagcata gttaataagc 4200
   aactgtaaaa agttttattt gtttacttta attcttttcc cagaagagtc tttgattcac 4260
   caaacatgaa tgtacatttt ctaacaaact caaaatctgg gaccaaaaca tcaacttttt 4320
   tctttctttt ttctttcttt ttgttttttc tttcctgtaa agaccttgaa aagcagtaac 4380
   ttgggtccag tatttacgga ggcgttgtga atgtgtccca tgcataacac actactggat 4440
   agtgagtgct gcgctaatgt actacgtagg gcttctacca gagattttcc tctccaattg 4500
   ggttgtgaaa tactcttcca aaagcctgca tcggggattc cacctactta tttcagattc 4560
   acctccatta accaagaaaa ccagtggaag atttcttgac tatttcacca tgttgccaat 4620
   caatactgga gtagcaaaaa aaatattttc tggaatactg ttttgtaatt ccctcactgg 4680
   ggtgcattgt agctggaaat tctctttata aaaatcattc ttgagctcca gcctggctat 4740
   ctctttcaag aaacatggcc actctttagg aatgctgttg cgtttgcatt gccaactaaa 4800
   atattaaaat atgcattggg gcttcttcat tcctttattt tgagaacctg atgcacaaag 4860
   agctcctttg ttcttttcga gtcccaccac tggaagagtg gtccatagac cccatgaaga 4920
   cattgtcatg atttgagaga ctgttgttga aaggattaac acaatcttaa tacactgaaa 4980
   attttaactg tgtcaagtca gcttagtgga gatttagcta tgccagtgag cagtgatttt 5040
   aactattctt ggctgcttaa acagggcagc tatgaactat gacaaatgta gatttttcaa 5100
   agcaatacaa aatactaaaa aagaggaacc ttaatgaata ttaaccacac agtctttctt 5160
   agccattcca aaaagaggca aagcaattct tattttcttt tttaaaataa tgattaatat 5220
   gattttgtgc acttcatact gtcacttttt aaaactacag aaaagagatt tagagtataa 5280
   cagaaacaag tgtgctttga tagtctcaaa taggtagaat tcatagttca agacctgaat 5340
   ccactgtcat ctctttcttc ctcccattgc agctatcctc aggtaccaaa tgttttgatt 5400
   tttaaataag gatagtaata aatggaggag gtgtcctata aatttaaagt tcagttgacc 5460
   cagccttata cttaagatag ccttatgaaa aatatgtgct gtgaggcaga agtatatttt
   5520
   ggcagagaga ataataaata aaactttttc ttttagctca atatccttac tttggtaagt
   5580
   attttttttt atttcacatc tacttaacag aaaataaact gagaaataga agtcagtcca
   5640
   ttggcataat ttatcattct tcactttaaa aaattctaat aaatattctg cttgagtttt
   5700
   cttttctgct atttgttctt acttgcaact ttaagtcaaa cctcccaata caaaacatta
   5760
   aaagctaaca ttaatgtact aaagtattaa tttaaaagaa atcgaacctc ccatgctaga
   5820
   tttgaaaata acatcatcac agcaccctga tcccaaatat tacaccgagg cttttaaaat
   5880
   gtaagtgaaa tctagctaag tttcatggtt tcattaaaag caaatgtctg cctctatctg
   5940
   aaaaacaaat ggaaatcttt tgaggtgtta ataccctttg gatcctcatc aaaaggatgg
   6000
   cattcacctg aggattccta tcttgacttc ttaggtatta aaaacctttc ttgatatgct
   6060
   ctacatttta aaatttgttt tataaaatcc ttatgttgat tttcatttta ttctcaagta
   6120
   caatacgttt cactctagac cagttgaaga acatgtttaa actttgttca tggtcaaatt
   6180
   cattttctat ttttttagta acatatctct taaaaagcac actaccttat aaaaaacttc
   6240
   atcagaaatt aaatttaatg caagtaaatt gccatctgat acttccacat gctatcataa
   6300
   tcaactgtaa taataaaaat gatttatcca attagaaaag gacaagatat atttttctct
   6360
   gtatttctat aacttttgcc actccattga atacattgta tgttggacat aagattatta
   6420
   gtaatgcatt cttgagatct tttattttgg aatgatgcta actctgtctc tttgccaatt
   6480
   ctaataccag gttccaagta ataactctac agtacaaaga gaactgaata ttcattctag
   6540
   ggctatagga tatgaacttc acaattcatt tgggtacatt ctcattgaat ttccttcaaa
   6600
   acaatctgtt cctggtgccc agtgataatt cagtcgggac cagcatgact aaaaggaagg
   6660
   ggatatgcta aggctcagca aagtgaccct aaatgagaga tatgtcccag gatggaaaga
   6720
   agaagacgtg gtttaaccaa gttatactga ctaatctaag cagtccactc atccttccat
   6780
   tttgggaaag gagtgggggc agcctaagaa gaacatatct ggattgggaa gaaccgtctt
   6840
   tctgggctag ggatggggaa cagaaaggga gtatggaaag aaaaattata agagatttga
   6900
   ctgaagcaag gaaaaaaagc aaatccccaa acgtgctaat ccttgaaagt aactatcttt
   6960
   cccaaactac tgctgttacc agcaagtgat caggaagact aggagctatt tctgactgta
   7020
   aatgaattgt ataatagctc tgctgcagtt ctgtgacttc caagccagga attaaatgct
   7080
   ctttttaaga ataacaaaaa acaaaagcat ttcctatgct agtctcccag taaaatgtac
   7140
   atgttttgga gacttcaaag gtattatgtg agttcacatt tagcaacagc ttattaataa
   7200
   ccctcaagct gtcagaatct ctatagttac catttacaat tttatactgt gaaaaaatac
   7260
   agatcagtga aagcataaag acaagtcaga attcactttg aagagggtct gaggcctggg
   7320
   agagtctcta ctgtctattg aagaatgagg catgtataaa atagttggtt gaatttcact
   7380
   gatcttccca atgtgaacaa atatactatg tatattgtgt gtatttctag aaatcaatgg
   7440
   cagctgctga tggtgttgta attagaaatc tatatagatt atagatgttt tagaaagatg
   7500
   gtgccaatcc taaaagattt gtgtgggcta aaagtgcttg tacttacttt tttctgcact
   7560
   tataactgat ttggttttaa aattgtgtgc gtgtatctgt tctttctctg ttgtggcagc
   7620
   ttgtactatt aaaataatag agaatgttaa attattttga tgtgaactgc aaatgatttt
   7680
   ttttcataaa gtttaacatt tttatcagca ttgttttgct ttgtacttgt ataaatatgt
   7740
   tttattttag cacttcaaaa tatacttgcc tgtttctcag ttgtctaaat catgttgtac
   7800
   ttggtgtttg tgaagccagt tacttttcaa aaaaattaaa aaacctataa tatga
   7855
<210> 43
   <211> 7927
   <212> DNA
   <213> Homo sapiens
<400> 43